(19) Europäisches Patentamt
European Patent Office
Office européen des brevets

(11) Veröffentlichungsnummer: **0 421 102 B1**

(12) # EUROPÄISCHE PATENTSCHRIFT

(45) Veröffentlichungstag der Patentschrift: **29.03.95**

(51) Int. Cl.[6]: **C07C 69/743**, C07C 69/76, C07D 213/55, C07D 237/24, C07D 241/12, C07D 239/26, C07D 213/61, C07D 261/08, C07D 263/34, C07C 69/734, C07D 277/30, C07D 317/68, A01N 37/36, A01N 37/38, A01N 37/44, A01N 37/48, A01N 43/30, A01N 43/40, A01N 43/74, A01N 43/80

(21) Anmeldenummer: **90115952.5**

(22) Anmeldetag: **21.08.90**

Die Akte enthält technische Angaben, die nach dem Eingang der Anmeldung eingereicht wurden und die nicht in dieser Patentschrift enthalten sind.

(54) **Substituierte Acrylsäureester.**

(30) Priorität: **01.09.89 DE 3928999**
**10.05.90 DE 4014940**

(43) Veröffentlichungstag der Anmeldung:
**10.04.91 Patentblatt 91/15**

(45) Bekanntmachung des Hinweises auf die Patenterteilung:
**29.03.95 Patentblatt 95/13**

(84) Benannte Vertragsstaaten:
**AT BE CH DE DK ES FR GB GR IT LI NL**

(56) Entgegenhaltungen:
**EP-A- 0 178 826**
**EP-A- 0 203 606**
**EP-A- 0 242 081**

(73) Patentinhaber: **BAYER AG**

**D-51368 Leverkusen (DE)**

(72) Erfinder: **Klausener, Alexander, Dr.**
**Weissdornweg 37**
**D-5190 Stolberg (DE)**
Erfinder: **Gayer, Herbert, Dr.**
**Alfred-Delp-Strasse 4**
**D-4019 Monheim-Baumberg (DE)**
Erfinder: **Krämer, Wolfgang, Dr.**
**Rosenkranz 25**
**D-5093 Burscheid 2 (DE)**
Erfinder: **Berg, Dieter, Dr.**
**Gellertweg 27**
**D-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 421 102 B1

Erfinder: **Brandes, Wilhelm, Dr.**
**Eichendorffstrasse 3**
**D-5653 Leichlingen 1 (DE)**
Erfinder: **Hänssler, Gerd, Dr.**
**Am Arenzberg 58a**
**D-5090 Leverkusen 3 (DE)**
Erfinder: **Wachendorff-Neumann, Ulrike, Dr.**
**Krischer Strasse 81**
**D-4019 Monheim (DE)**

## Beschreibung

Die Erfindung betrifft neue substituierte Acrylsäureester, mehrere Verfahren zu ihrer Herstellung, ihre Verwendung zur Bekämpfung von Schädlingen und neue Zwischenprodukte.

Es ist bekannt, daß bestimmte substituierte Acrylsäureester, wie beispielsweise die Verbindung 3-Methoxy-2-(2-methylphenyl)-acrylsäuremethylester fungizide Eigenschaften besitzen (vgl. z.B. EP 178826).

Es wurden neue substituierte Acrylsäureester der allgemeinen Formel (I) gefunden,

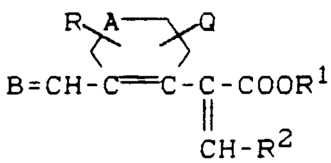

(I)

in welcher

- $R^1$ für Alkyl oder für unsubstituiertes oder substituiertes Aralkyl steht,
- $R^2$ für Dialkylamino oder für einen Rest $-Z-R^3$ steht,
- $R^3$ für Alkyl oder für unsubstituiertes oder substituiertes Aralkyl steht,
- Z für Sauerstoff oder Schwefel steht,
- A für Sauerstoff, Schwefel oder für eine der folgenden Gruppierungen steht $-(CH_2)-_n$,

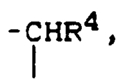 , 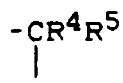 oder 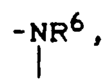 ,

  wobei
- n für eine Zahl 0 bis 6 steht,
- $R^4$ und $R^5$ jeweils unabhängig voneinander für Alkyl stehen oder gemeinsam für eine Alkylkette mit 2 bis 7 Kohlenstoffatomen stehen und
- $R^6$ für Alkyl oder für einen Rest

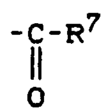

  steht,
  wobei
- $R^7$ für Alkyl, Alkoxy oder Dialkylamino steht,
- B für die Gruppe $CH-R^8$ oder $NO-R^9$ steht,
  wobei
- $R^8$ für unsubstituiertes oder substituiertes Aryl oder Hetaryl steht und
- $R^9$ für unsubstituiertes oder substituiertes Alkyl, Alkenyl, Alkinyl, Aralkyl, Aryl oder Hetaryl steht und
- R und Q unabhängig voneinander für Wasserstoff, Alkyl, Halogenalkyl oder Alkoxy stehen.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Acrylsäureester der allgemeinen Formel (I),

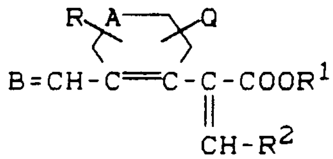

(I)

in welcher

| | |
|---|---|
| $R^1$ | für Alkyl oder für unsubstituiertes oder substituiertes Aralkyl steht, |
| $R^2$ | für Dialkylamino oder für einen Rest $-Z-R^3$ steht, |
| $R^3$ | für Alkyl oder für unsubstituiertes oder substituiertes Aralkyl steht, |
| Z | für Sauerstoff oder Schwefel steht, |
| A | für Sauerstoff, Schwefel oder für eine der folgenden Gruppierungen steht $-(CH_2)-_n$, |

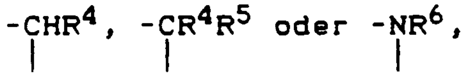

wobei

| | |
|---|---|
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und |
| $R^6$ | für Alkyl oder für einen Rest |

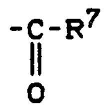

steht,
wobei

| | |
|---|---|
| $R^7$ | für Alkyl, Alkoxy oder Dialkylamino steht, |
| B | für die Gruppe $CH-R^8$ oder $NO-R^9$ steht, wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen, |

nach einem der im folgenden beschriebenen Verfahren erhält:

a) Man erhält substituierte Acrylsäureester der Formel (Ia),

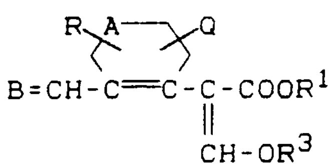

(Ia)

in welcher

$R^1$, $R^3$, A, B, R und Q die oben angegebene Bedeutung haben,

wenn man Hydroxyacrylsäureester oder deren Alkalimetallsalze der Formel (II),

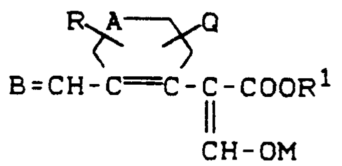 (II)

in welcher

M für Wasserstoff oder für ein Alkalimetallkation steht und

$R^1$, A, B, R und Q die oben angegebene Bedeutung haben,

mit Alkylierungsmitteln der Formel (III)

$R^3$-$E^1$ (III)

in welcher

$E^1$ für eine elektronenanziehende Abgangsgruppe steht und

$R^3$ die oben angegebene Bedeutung hat,

gegebenenfalls in Gegenwart eines Verdünnungsmittels und gegebenenfalls in Gegenwart eines Reaktionshilfsmittels umsetzt;

b) man erhält substituierte Acrylsäureester der Formel (Ib),

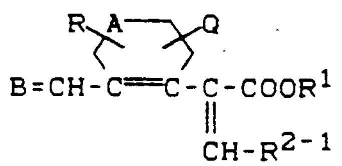 (Ib)

in welcher

$R^{2-1}$ für Dialkylamino steht und

$R^1$, A, B, R und Q die oben angegebene Bedeutung haben,

wenn man substituierte Essigsäureester der Formel (IV),

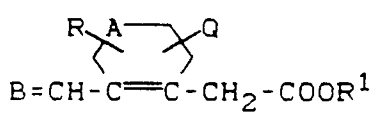 (IV)

in welcher

$R^1$, A, B, R und Q die oben angegebene Bedeutung haben,

mit Formamiden der Formel (Va)

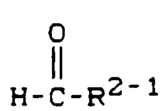 (Va)

in welcher

$R^{2-1}$ die oben angegebene Bedeutung hat,

oder mit Formamid-Derivaten der Formel (Vb)

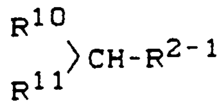

(Vb)

in welcher

$R^{10}$ und $R^{11}$ unabhängig voneinander für Alkoxy oder Dialkylamino stehen und

$R^{2-1}$ die oben angegebene Bedeutung hat, gegebenenfalls in Gegenwart eines Verdünnungsmittels umsetzt;

c) man erhält substituierte Acrylsäureester der Formel (Ic),

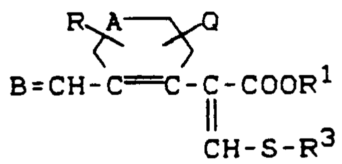

(Ic)

in welcher

$R^1$, $R^3$, A, B, R und Q die oben angegebene Bedeutung haben,

wenn man Ketocarbonsäurederivate der Formel (VI)

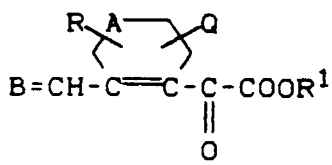

(VI)

in welcher

$R^1$, A, B, R und Q die oben angegebene Bedeutung haben,

mit metallorganischen Verbindungen der Formel (VII),

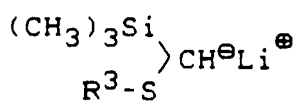

(VII)

in welcher

$R^3$ die oben angegebene Bedeutung hat,

gegebenenfalls in Gegenwart eines Verdünnungsmittels umsetzt;

d) man erhält substituierte Acrylsäureester der Formel (Ic) weiterhin,

wenn man substituierte Acrylsäureester der Formel (VIII),

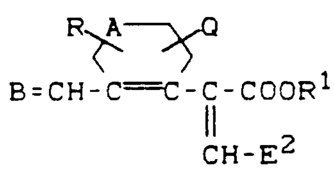

(VIII)

in welcher

$R^1$, A, B, R und Q die oben angegebene Bedeutung haben und

$E^2$ für eine elektronenanziehende Abgangsgruppe steht,
mit Thiolen der Formel (IX),

$R^3$-SH (IX)

in welcher
$R^3$ die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Acrylsäureester der allgemeinen Formel (I) eine gute Wirkung gegen Schädlinge besitzen.

Überraschenderweise zeigen die erfindungsgemäßen substituierten Acrylsäureester der allgemeinen Formel (I) eine insektizide Wirkung sowie eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche strukturell und wirkungsmäßig naheliegende Verbindungen sind.

Bevorzugte Substituenten bzw. Bereiche der in den oben und nachstehend erwähnten Formeln aufgeführten Reste werden im folgenden erläutert:
Für unsubstituiertes oder substituiertes Alkyl in den Definitionen von $R^1$, $R^3$, $R^4$, $R^5$, $R^6$, $R^7$, $R^9$, R und Q in den allgemeinen Formeln, steht geradkettiges oder verzweigtes Alkyl mit vorzugsweise 1 bis 8, besonders bevorzugt 1 bis 6 und insbesondere 1 bis 4, Kohlenstoffatomen. Beispielhaft seien unsubstituiertes oder substituiertes Methyl, Ethyl, n-Propyl, i-Propyl, n-Butyl, sec-Butyl, i-Butyl, t-Butyl, n-Pentyl, i-Pentyl und t-Pentyl genannt.

Für den Begriff unsubstituiertes oder substituiertes Alkenyl in den Definitionen von $R^9$ in den allgemeinen Formeln steht geradkettiges oder verzweigtes Alkenyl mit vorzugsweise 2 bis 8, besonders bevorzugt 2 bis 6 und insbesondere 2 bis 4, ganz besonders bevorzugt 3 Kohlenstoffatomen. Beispielhaft seien unsubstituiertes oder substituiertes Vinyl, Allyl, Propenyl-(2), Butenyl-(1), 2-Butenyl, 3-Butenyl und 1-Methallyl genannt.

Dialkylamino in der Definiton von $R^2$ und $R^7$ steht für eine Aminogruppe mit 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Unter dem Begriff unsubstituiertes oder substituiertes Aryl in der Definition von $R^8$ und $R^9$ in den allgemeinen Formeln ist Aryl mit vorzugsweise 6 bis 10 Kohlenstoffatomen im Arylteil zu verstehen. Beispielhaft und vorzugsweise seien unsubstituiertes oder substituiertes Phenyl oder Naphthyl, insbesondere Phenyl genannt.

Unsubstituiertes oder substituiertes Aralkyl in den Definitionen von $R^1$, $R^3$ und $R^9$ enthält vorzugsweise 1 bis 6, insbesondere 1 bis 4 Kohlenstoffatome im geradkettigen oder verzweigten Alkylteil und vorzugsweise Phenyl als Arylteil. Als Aralkylgruppen seien beispielhaft und vorzugsweise Benzyl und Phenethyl genannt.

Heteroaryl in der Definition von $R^8$ und $R^9$ steht im allgemeinen für einen 5- bis 9-gliedrigen Ring, der ein bis 4, bevorzugt 1 bis 3 gleiche oder verschiedene Heteroatome, enthält. Als Heteroatome seien vorzugsweise Sauerstoff, Schwefel und Stickstoff genannt; beispielhaft und vorzugsweise seien genannt: Pyrimidinyl, Pyrrolyl, Isothiazolyl, Oxazolyl, Pyridyl, Thienyl, Furyl, Pyridazinyl, Pyrazinyl, Isoxazolyl, Thiazolyl.

Unter dem Begriff unsubstituiertes oder substituiertes Alkinyl in der Definition von $R^9$ in den allgemeinen Formeln ist geradkettiges oder verzweigtes Alkinyl mit 2 bis 8, vorzugsweise 2 bis 6, insbesondere 2 bis 4, besonders bevorzugt 3 Kohlenstoffatomen zu verstehen. Beispielhaft seien unsubstituiertes oder substituiertes Ethinyl, 2-Propinyl, 2-Butinyl, 3-Butinyl und 1-Methyl-2-propinyl genannt.

Halogenalkyl steht in den Definitionen R und Q für geradkettige oder verzweigte Reste mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl.

Unter dem Begriff unsubstituiertes oder substituiertes Alkoxy in der Definition von $R^7$, R und Q in den allgemeinen Formeln ist geradkettiges oder verzweigtes Alkoxy mit vorzugsweise 1 bis 6, insbesondere 1 bis 4 Kohlenstoffatomen zu verstehen. Beispielhaft seien unsubstituiertes oder substituiertes Methoxy, Ethoxy, Propoxy, Butoxy sowie ihre Isomeren, i-Propoxy, i-, s- und t-Butoxy genannt.

Die Substituenten für die Arylreste als solche oder in Zusammensetzungen wie Arylalkyl, Aryloxy, Arylthio, Aralkyloxy und für die heterocyclischen Ringe wie Heteroarylalkyl und Heteroaryl haben die im folgenden angegebenen Bedeutungen.

Halogen steht im allgemeinen als Substituent für Fluor, Chlor, Brom und Iod, bevorzugt für Fluor, Chlor und Brom, besonders bevorzugt für Fluor und Chlor.

Alkyl steht im allgemeinen als Substituent oder in Zusammensetzungen wie Alkoximinoalkyl für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkoxy steht im allgemeinen als Substituent oder in Zusammensetzungen wie Alkoximinoalkyl für geradkettiges oder verzweigtes Alkoxy mit 1 bis 6, besonders bevorzugt 1 bis 3 Kohlenstoffatomen je Alkylrest; beispielhaft und vorzugsweise seien genannt: Methoxy, Ethoxy, n-und i-Propoxy, n-, i-, s- und t-Butoxy, n-Hexoxy und i-Hexoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio, Ethylthio, Propylthio, Butylthio, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen im allgemeinen als Substituenten in den Resten für geradkettiges oder verzweigtes Halogenalkyl oder Halogenalkoxy mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettige oder verzweigtes Halogenalkylthio mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Alkoxycarbonyl steht im allgemeinen als Substituent in den Resten für geradkettiges oder verzweigtes Alkoxycarbonyl mit 1 bis 4, vorzugsweise 1 oder 2 Kohlenstoffatomen im Alkoxyrest; beispielhaft seien genannt: Methoxycarbonyl, Ethoxycarbonyl, n-Propoxycarbonyl, i-Propoxycarbonyl, n-, i-, s- und t-Butoxycarbonyl.

Cycloalkyl steht im allgemeinen als Substituent in den Resten für Cycloalkyl mit vorzugsweise 3 bis 7, insbesondere 3, 5 oder 6 Kohlenstoffatomen. Beispielhaft seien unsubstituiertes oder substituiertes Cyclopropyl, Cyclobutyl, Cyclopentyl, Cyclohexyl und Cycloheptyl genannt.

Unsubstituiertes oder substituiertes Aryl, Aryloxy und Arylthio stehen im allgemeinen als Substituenten in der Definition von $R^8$ und $R^9$ in den allgemeinen Formeln für Aryl mit vorzugsweise 6 bis 10 Kohlenstoffatomen im Arylteil. Beispielhaft seien unsubstituiertes oder substituiertes Phenyl oder Naphthyl, Phenoxy oder Phenylthio, insbesondere Phenyl genannt.

Unsubstituiertes oder substituiertes Aralkyl, Aralkyloxy oder Aralkylthio enthalten im allgemeinen als Substituenten in den Definitionen von $R^8$ und $R^9$ vorzugsweise 1 bis 6 Kohlenstoffatome im geradkettigen oder verzweigten Alkylteil und vorzugsweise Phenyl als Arylteil. Als Aralkylgruppen seien beispielhaft Benzyl, Phenethyl, Benzyloxy und Benzylthio genannt.

Heteroaryl, Heteroaryloxy und Heteroarylthio in der Definition von $R^8$ und $R^9$ stehen im allgemeinen als Substituenten für einen 5- bis 9-gliedrigen Ring, der ein oder mehrere Heteroatome, bevorzugt 1 bis 3 gleiche oder verschiedene Heteroatome, enthält. Als Heteroatome seien vorzugsweise Sauerstoff, Schwefel und Stickstoff genannt; beispielhaft seien genannt: Pyridyl, Thienyl, Furyl, Pyridazinyl, Pyrazinyl, Isoxazolyl und Thiazolyl.

Die erfindungsgemäßen substituierten Acrylsäureester sind durch die Formel (I) allgemein definiert. Bevorzugt sind Verbindungen der Formel (I), bei welchen

$R^1$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

$R^2$ für Dialkylamino mit jeweils 1 bis 6 Kohlenstoffatomen in den einzelnen geradkettigen oder verzweigten Alkylteilen oder für einen Rest $-Z-R^3$ steht,

wobei

$R^3$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

Z für Sauerstoff oder Schwefel steht,

A für Sauerstoff, Schwefel oder für eine der folgenden Gruppierungen steht

$-(CH_2)-_n$,

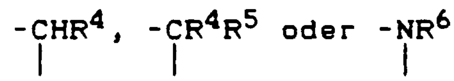

wobei

n für eine Zahl 0 bis 3 steht,

$R^4$ und $R^5$ jeweils unabhängig voneinander für geradkettiges oder verzweigtes Alkyl mit 1 bis 6 Kohlenstoffatomen stehen oder gemeinsam für eine Alkylkette mit 2 bis 7 Kohlenstoffatomen stehen und

$R^6$ für geradkettiges oder verzweigtes Alkyl mit 1 bis 6 Kohlenstoffatomen oder für einen Rest

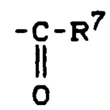

steht,

wobei

$R^7$ für geradkettiges oder verzweigtes Alkyl mit 1 bis 6 Kohlenstoffatomen, geradkettiges oder verzweigtes Alkoxy mit 1 bis 6 Kohlenstoffatomen oder geradkettiges oder verzweigtes Dialkylamino mit jeweils 1 bis 6 Kohlenstoffatomen in den einzelnen Alkylteilen steht,

B für die Gruppe $CH-R^8$ oder $NO-R^9$ steht,

wobei,

$R^8$ für unsubstituiertes oder substituiertes Aryl mit 6 bis 10 Kohlenstoffatomen im Arylteil oder für Heteroaryl mit 2 bis 9 Kohlenstoffatomen und 1 bis 4 gleichen oder verschiedenen Heteroatomen, - insbesondere Stickstoff, Sauerstoff und/oder Schwefel - im Heteroarylteil steht,

wobei als Aryl- und Heteroarylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen,

Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil,

$R^9$ für jeweils unsubstituiertes oder substituiertes Alkyl mit 1 bis 8 Kohlenstoffatomen, unsubstituiertes oder substituiertes Alkenyl mit 2 bis 8 Kohlenstoffatomen oder unsubstituiertes oder substituiertes Alkinyl mit 2 bis 8 Kohlenstoffatomen steht, wobei als Substituenten jeweils infrage kommen: Cyano, Alkoxy mit 1 bis 4 Kohlenstoffatomen oder Alkoxycarbonyl mit 1 bis 4 Kohlenstoffatomen im Alkoxyteil,
weiterhin
für unsubstituiertes oder einfach bis mehrfach, gleich oder verschieden substituiertes Aryl mit 6 bis 10 Kohlenstoffatomen, unsubstituiertes oder einfach bis mehrfach, gleich oder verschieden substituiertes Aralkyl mit 6 bis 10 Kohlenstoffatomen im Arylteil und 1 bis 2 Kohlenstoffatomen im Alkylteil steht, wobei als Substituenten genannt seien:
Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen oder Alkylidendioxy mit 1 bis 2 Kohlenstoffatomen,
ferner
für Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil steht und

R und Q unabhängig voneinander für Wasserstoff, Alkyl mit 1 bis 4 Kohlenstoffatomen, Alkoxy mit 1 bis 4 Kohlenstoffatomen oder Halogenalkyl mit 1 bis 4 Kohlenstoffatomen und 1 bis 9 gleichen oder verschiedenen Halogenatomen stehen.

Besonders bevorzugt sind Verbindungen der Formel (I), bei welchen

$R^1$ für Methyl, Ethyl, n- oder i-Propyl, n-, i-, s-oder t-Butyl oder für gegebenenfalls ein- bis dreifach, gleich oder verschieden substituiertes Benzyl steht, wobei als Substituenten die bei $R^8$ genannten infrage kommen,

$R^2$ für Dialkylamino mit jeweils 1 bis 4 Kohlenstoffatomen in den einzelnen geradkettigen oder verzweigten Alkylteilen steht oder für einen Rest $-Z-R^3$ steht,
wobei

$R^3$ für Methyl, Ethyl, n- oder i-Propyl, n-, i-, s- oder t-Butyl oder für gegebenenfalls ein-bis dreifach, gleich oder verschieden substituiertes Benzyl steht, wobei als Substituenten die bei $R^8$ genannten infrage kommen und

Z für Sauerstoff oder Schwefel steht;

A für Sauerstoff, Schwefel oder für eine der folgenden Gruppierungen steht
$-(CH_2)-n$,

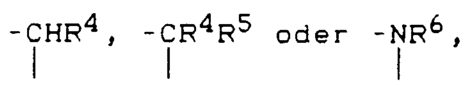

wobei

n für die Zahlen 0, 1, 2 und 3 steht,

$R^4$ und $R^5$ jeweils unabhängig voneinander für geradkettiges oder verzweigtes Alkyl mit 1 bis 4 Kohlenstoffatomen stehen oder gemeinsam für eine Alkylkette mit 2 bis 5 Kohlenstoffatomen stehen und

$R^6$ für geradkettiges oder verzweigtes Alkyl mit 1 bis 4 Kohlenstoffatomen oder für einen Rest

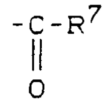

steht,
wobei
$R^7$ für geradkettiges oder verzweigtes Alkyl mit 1 bis 4 Kohlenstoffatomen, geradkettiges oder verzweigtes Alkoxy mit 1 bis 4 Kohlenstoffatomen oder geradkettiges oder verzweigtes Dialkylamino mit jeweils 1 bis 4 Kohlenstoffatomen in den einzelnen Alkylresten steht,

B für die Gruppe CH-$R^8$ oder NO-$R^9$ steht,

wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,
wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Benzyloxy, Phenylthio oder Benzylthio;

$R^9$ für jeweils unsubstituiertes oder substituiertes Alkyl mit 1 bis 6 Kohlenstoffatomen, unsubstituiertes oder substituiertes Alkenyl mit 2 bis 6 Kohlenstoffatomen, unsubstituiertes oder substituiertes Alkinyl mit 2 bis 6 Kohlenstoffatomen, wobei als Substituenten jeweils infrage kommen:
Cyano, Methoxy, Ethoxy, n- und i-Propoxy, n-, i-, s- und t-Butoxy, Methoxycarbonyl, Ethoxycarbonyl, n- und i-Propoxycarbonyl, n-, i-, s- und t-Butoxycarbonyl,
weiterhin
für unsubstituiertes oder einfach bis dreifach, gleich oder verschieden substituiertes Phenyl oder Benzyl steht, wobei als Substituenten genannt seien:
Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Halogenalkyl mit 1 oder 2 Kohlenstoffatomen und 1 bis 5 gleichen oder verschiedenen Fluor- oder Chloratomen, Methylidendioxy und Ethylidendioxy steht und

R und Q unabhängig voneinander für Wasserstoff, Methyl, Ethyl, n- oder i-Propyl, Methoxy, Ethoxy oder Halogenalkyl mit 1 oder 2 Kohlenstoffatomen und 1 bis 5 gleichen oder verschiedenen Fluor- oder Chloratomen steht.

Ganz besonders bevorzugt sind Verbindungen der Formel (I), bei welchen

$R^1$ für Methyl, Ethyl oder Benzyl steht, wobei als Benzyl-Substituenten die bei $R^8$ genannten infrage kommen,

$R^2$ für Dimethylamino, Diethylamino oder für einen Rest -Z-$R^3$ steht,
wobei

$R^3$ für Methyl, Ethyl, n- oder i-Propyl oder Benzyl steht,

Z für Sauerstoff oder Schwefel steht;

A für Sauerstoff, Schwefel oder für eine der folgenden Gruppierungen steht,
$-(CH_2)-_n$, $>CH-CH_3$, $>CH-C_2H_5$,
$>CH-CH(CH_3)_2$, $>CH-C(CH_3)_3$, $>C(CH_3)_2$,

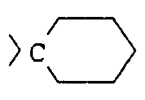 ,

$>N-COOCH_3$, $>N-COOC_2H_5$,

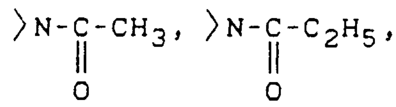

wobei

n für die Zahlen 0, 1, 2 und 3 steht,

B für die Gruppe CH-$R^8$ oder NO-$R^9$ steht,

wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,

wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl, Benzyloxy, Phenylthio oder Benzylthio;

$R^9$ für jeweils unsubstituiertes oder substituiertes Alkyl mit 1-6 Kohlenstoffatomen, unsubstituiertes oder substituiertes Alkenyl mit 2 bis 4 Kohlenstoffatomen und unsubstituiertes oder substituiertes Alkinyl mit 2 bis 4 Kohlenstoffatomen steht,

wobei als Substituenten infrage kommen, Cyano, Methoxycarbonyl, Ethoxycarbonyl, Methoxy oder Alkoxy;

weiterhin für unsubstituiertes oder einfach oder zweifach, gleich oder verschieden substituiertes Phenyl oder Benzyl steht,

wobei als Substituenten infrage kommen: Brom, Fluor, Chlor, Methoxy, Ethoxy, Methylendioxy, Methyl, Ethyl, Trifluormethyl und

R und Q unabhängig voneinander für Wasserstoff oder Methyl stehen.

Insbesondere bevorzugt sind Verbindungen der Formel (I), bei welchen

$R^1$ für Methyl oder Ethyl steht,

$R^2$ für Methoxy, Ethoxy, Methylthio oder Dimethylamino steht,

A für Sauerstoff, Schwefel oder für eine der folgenden Gruppierungen steht

$-(CH_2)-_n$,

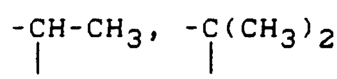

wobei

n für die Zahlen 0, 1 oder 2 steht,

B für die Gruppe CH-$R^8$ oder NO-$R^9$ steht,

wobei

$R^8$ für jeweils unsubstituiertes oder ein- bis dreifach, gleich oder verschieden substituiertes Phenyl, Naphthyl, Pyridinyl, Pyrimidinyl, Thienyl, Furyl, Thiazolyl, Oxazolyl, Isoxazolyl steht,

wobei als Substituenten infrage kommen: Fluor, Chlor, Brom, Cyano, Nitro, Methyl, Ethyl, Methoxy, Ethoxy, Methylendioxy, Methylthio, Trifluormethyl, Methoxycarbonyl, Ethoxycarbonyl, Cyclopentyl, Cyclohexyl, Phenoxy, Phenylthio, Benzyloxy, Benzylthio;

$R^9$ für Methyl, Ethyl, n- und i-Propyl, n-, i- und t-Butyl, n- und iso-Amyl, Allyl, Methallyl, Propargyl, Benzyl, o-, m- und p-Chlorbenzyl, o-, m- und p-Methoxy-benzyl, o-, m- und p-Methyl-benzyl oder o-, m- und p-Fluorbenzyl steht und

R und Q für Wasserstoff stehen.

Im einzelnen seien außer den bei den Herstellungsbeispielen genannten Verbindungen die folgenden substituierten Acrylsäureester der allgemeinen Formel (I) genannt:

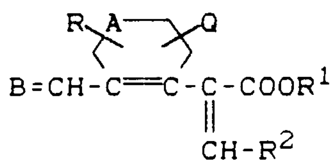

(I)

Tabelle 1:

| $R^1$ | $R^2$ | A | R | Q | B |
|---|---|---|---|---|---|
| $CH_3$ | $OCH_3$ | - | H | H | CH-(2-Cl-$C_6H_4$) |
| $CH_3$ | $OCH_3$ | - | H | H | CH-(3-Cl-$C_6H_4$) |
| $CH_3$ | $OCH_3$ | - | H | H | CH-(2,3-$Cl_2$-$C_6H_3$) |
| $CH_3$ | $OCH_3$ | - | H | H | CH-(2,6-$Cl_2$-$C_6H_3$) |
| $CH_3$ | $OCH_3$ | - | H | H | CH-(2,5-$Cl_2$-$C_6H_3$) |
| $CH_3$ | $OCH_3$ | - | H | H | CH-(3,5-$Cl_2$-$C_6H_3$) |
| $CH_3$ | $OCH_3$ | - | H | H | CH-(2,4,6-$Cl_3$-$C_6H_2$) |
| $CH_3$ | $OCH_3$ | - | H | H | CH-(4-F-$C_6H_4$) |
| $CH_3$ | $OCH_3$ | - | H | H | CH-(4-$C_2H_5$-$C_6H_4$) |
| $CH_3$ | $OCH_3$ | - | H | H | 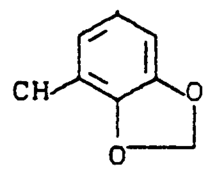 |
| $CH_3$ | $OCH_3$ | - | H | H | CH-(3,4-$F_2$-$C_6H_3$) |
| $CH_3$ | $OCH_3$ | - | H | H | 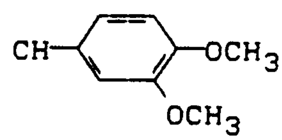 |
| $CH_3$ | $OCH_3$ | - | H | H | 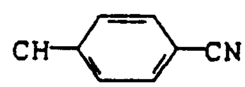 |
| $CH_3$ | $OCH_3$ | - | H | H | 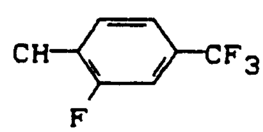 |
| $CH_3$ | $OCH_3$ | - | H | H | 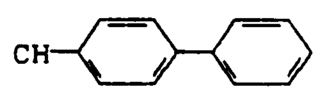 |

Tabelle 1 - Fortsetzung

| $R^1$ | $R^2$ | A | P | Q | B |
|---|---|---|---|---|---|
| $CH_3$ | $OCH_3$ | - | H | H | 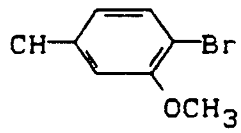 |
| $CH_3$ | $OCH_3$ | - | H | H | $CH\text{-}(3\text{-}Cl\text{-}4\text{-}CF_3\text{-}C_6H_3)$ |
| $CH_3$ | $OCH_3$ | - | H | H | $CH\text{-}(C_6Cl_5)$ |
| $CH_3$ | $OCH_3$ | - | H | H | 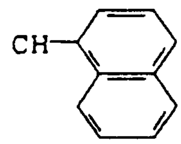 |
| $CH_3$ | $OCH_3$ | - | H | H | 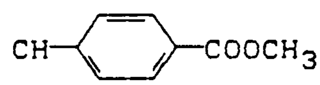 |
| $CH_3$ | $OCH_3$ | - | H | H | 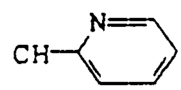 |
| $CH_3$ | $OCH_3$ | - | H | H | 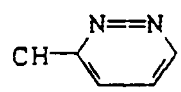 |
| $CH_3$ | $OCH_3$ | - | H | H | 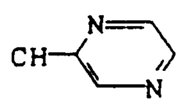 |
| $CH_3$ | $OCH_3$ | - | H | H | 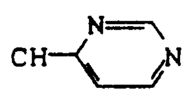 |
| $CH_3$ | $OCH_3$ | - | H | H | 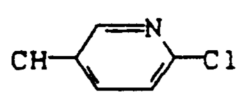 |
| $CH_3$ | $OCH_3$ | - | H | H | 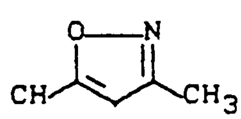 |

Tabelle 1 - Fortsetzung

| $R^1$ | $R^2$ | A | P | Q | B |
|---|---|---|---|---|---|
| $CH_3$ | $OCH_3$ | - | H | H | 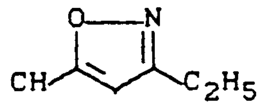 |
| $CH_3$ | $OCH_3$ | - | H | H | 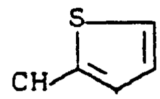 |
| $CH_3$ | $OCH_3$ | - | H | H | 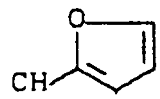 |
| $CH_3$ | $OCH_3$ | - | H | H | 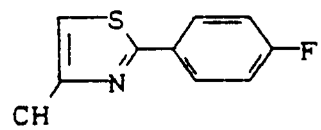 |
| $CH_3$ | $OCH_3$ | - | H | H | 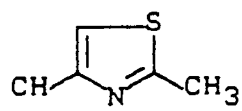 |
| $CH_2CH_3$ | $OCH_3$ | - | H | H | $CH-C_6H_5$ |
| $CH_2CH_3$ | $OCH_3$ | - | H | H | $CH-(4-Cl-C_6H_4)$ |
| $CH_2CH_3$ | $OCH_3$ | - | H | H | $CH-(3,4-Cl_2-C_6H_3)$ |
| $CH_3$ | $OCH_2CH_3$ | - | H | H | $CH-[3,4-(CH_3)_2-C_6H_3]$ |
| $CH_3$ | $OCH_2CH_3$ | - | H | H | $CH-(3,4-Cl_2-C_6H_3)$ |
| $CH_3$ | $OCH_2CH_3$ | - | H | H | $CH-(4-Cl-C_6H_4)$ |
| $CH_2CH_3$ | $OCH_2CH_3$ | - | H | H | $CH-(4-Br-C_6H_4)$ |
| $CH_2CH_3$ | $OCH_2CH_3$ | - | H | H | 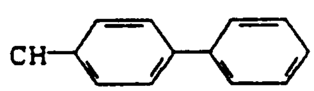 |
| $CH_2CH_3$ | $OCH_2CH_3$ | - | H | H | 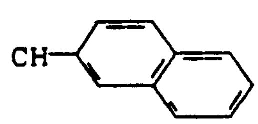 |

Tabelle 1 - Fortsetzung

| $R^1$ | $R^2$ | A | R | Q | B |
|---|---|---|---|---|---|
| $CH_3$ | $OCH_3$ | $CH_2$ | H | H | $CH(2\text{-}Cl\text{-}C_6H_4)$ |
| $CH_3$ | $OCH_3$ | $CH_2$ | H | H | $CH(3\text{-}Cl\text{-}C_6H_4)$ |
| $CH_3$ | $OCH_3$ | $CH_2$ | H | H | $CH(3,4\text{-}F_2\text{-}C_6H_3)$ |
| $CH_3$ | $OCH_3$ | $CH_2$ | H | H | $CH(2,3\text{-}Cl_2\text{-}C_6H_3)$ |
| $CH_3$ | $OCH_3$ | $CH_2$ | H | H | CH 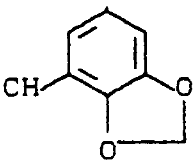 |
| $CH_3$ | $OCH_3$ | $CH_2$ | H | H | CH 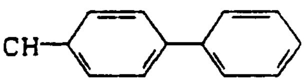 |
| $CH_3$ | $OCH_3$ | $CH_2$ | H | H | CH 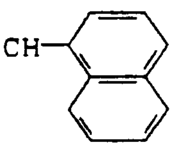 |
| $CH_3$ | $OCH_3$ | $CH_2$ | H | H | CH 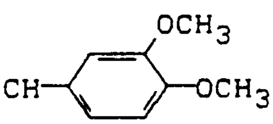 |
| $CH_3$ | $OCH_3$ | $CH_2$ | H | H | CH 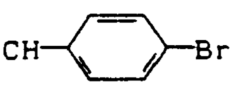 |
| $CH_3$ | $OCH_3$ | $CH_2$ | H | H | CH 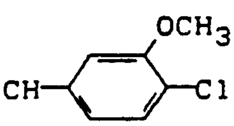 |
| $CH_3$ | $OCH_3$ | $CH_2$ | H | H | CH 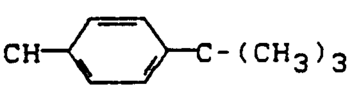 |
| $CH_3$ | $SCH_3$ | $CH_2$ | H | H | $CHC_6H_5$ |

Tabelle 1 - Fortsetzung

| $R^1$ | $R^2$ | A | R | Q | B |
|---|---|---|---|---|---|
| $CH_3$ | $SCH_3$ | $CH_2$ | H | H | $CH(3,4\text{-}Cl_2\text{-}C_6H_3)$ |
| $CH_3$ | $N(CH_3)_2$ | $CH_2$ | H | H | $CH(4\text{-}Br\text{-}C_6H_4)$ |
| $CH_3$ | $N(CH_3)_2$ | $CH_2$ | H | H | $CHC_6H_5$ |
| $CH_3$ | $N(CH_3)_2$ | $CH_2$ | H | H | $CH(3,4\text{-}Cl_2\text{-}C_6H_3)$ |
| $CH_3$ | $SCH_3$ | $CH_2$ | H | H | $CH(4\text{-}Br\text{-}C_6H_4)$ |
| $CH_2C_6H_5$ | $OCH_3$ | $CH_2$ | H | H | $CHC_6H_5$ |
| $CH_2C_6H_5$ | $OCH_3$ | $CH_2$ | H | H | $CH(3,4\text{-}Cl_2\text{-}C_6H_3)$ |
| $CH_2C_6H_5$ | $OCH_3$ | $CH_2$ | H | H | $CH(4\text{-}Br\text{-}C_6H_4)$ |
| $CH_3$ | $OCH_3$ | $CH_2$ | H | H | 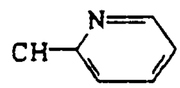 |
| $CH_3$ | $OCH_3$ | $CH_2$ | H | H | 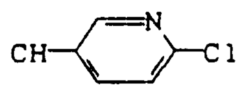 |
| $CH_3$ | $OCH_3$ | $CH_2$ | H | H | 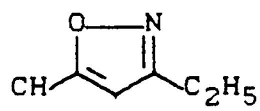 |
| $CH_3$ | $OCH_3$ | $CH_2$ | | | 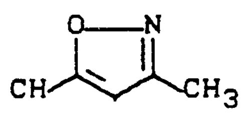 |
| $CH_3$ | $OCH_3$ | $CH_2$ | H | H | 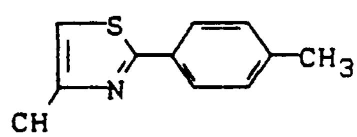 |

Tabelle 1 - Fortsetzung

| $R^1$ | $R^2$ | A | R | Q | B |
|---|---|---|---|---|---|
| $CH_3$ | $OCH_3$ | $CH_2$ | H | H | 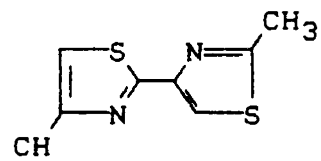 |
| $CH_3$ | $OCH_3$ | $CH_2$ | H | H | 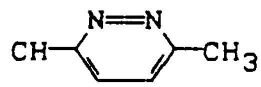 |
| $CH_3$ | $OCH_3$ | $CH_2$ | H | H | 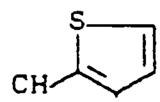 |
| $CH_3$ | $OCH_3$ | $CH_2$ | H | H | 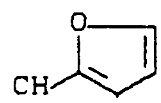 |
| $CH_3$ | $OCH_2H_3$ | $CH_2$ | H | H | 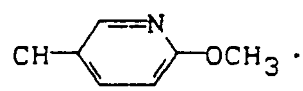 |
| $CH_3$ | $OCH_2CH_3$ | $CH_2$ | H | H | 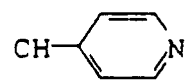 |
| $CH_2CH_3$ | $OCH_3$ | $CH_2$ | H | H | 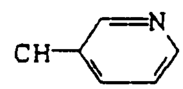 |
| $CHCH_3$ | $OCH_3$ | $CH_2$ | H | H | $CH-C_6H_5$ |
| $CH_3$ | $OCH_3$ | $CH_2$ | H | H | $CH-(2,6-F_2-C_6H_3)$ |
| $CH_3$ | $OCH_3$ | $CH_2$ | H | H | $CH-(2-Cl-6-F-C_6H_3)$ |
| $CH_3$ | $OCH_3$ | $CH_2$ | H | H | $CH-(3-COOCH_3-C_6H_4)$ |
| $CH_2CH_3$ | $OCH_3$ | $CH_2$ | H | H | $CH-(3-CF_3-C_6H_4)$ |
| $CH_3$ | $OCH_3$ | $CH_2$ | H | H | 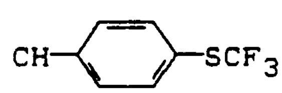 |

Tabelle 1 - Fortsetzung

| $R^1$ | $R^2$ | A | R | Q | B |
|---|---|---|---|---|---|
| $CH_3$ | $OCH_3$ | $CH_2$ | H | H | 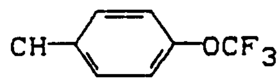 |
| $CH_3$ | $OCH_3$ | $CH_2$ | H | H | 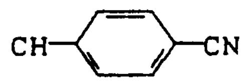 |
| $CH_3$ | $OCH_3$ | $CH_2CH_2$ | H | H | $CH(2\text{-}Cl\text{-}C_6H_4)$ |
| $CH_3$ | $OCH_3$ | $CH_2CH_2$ | H | H | $CH(3\text{-}Cl\text{-}C_6H_4)$ |
| $CH_3$ | $OCH_3$ | $CH_2CH_2$ | H | H | $CH(2,3\text{-}Cl_2\text{-}C_6H_3)$ |
| $CH_3$ | $OCH_3$ | $CH_2CH_2$ | H | H | $CH(4\text{-}Br\text{-}C_6H_4)$ |
| $CH_3$ | $OCH_3$ | $CH_2CH_2$ | H | H | $CH(3\text{-}CF_3\text{-}C_6H_4)$ |
| $CH_3$ | $OCH_3$ | $CH_2CH_2$ | H | H | $CH(3\text{-}OCH_3\text{-}4\text{-}Cl\text{-}C_6H_3)$ |
| $CH_3$ | $OCH_3$ | $CH_2CH_2$ | H | H | $CH(3\text{-}OCH_3\text{-}C_6H_4)$ |
| $CH_3$ | $OCH_3$ | $CH_2CH_2$ | H | H | $CH(3\text{-}CH_3\text{-}C_6H_4)$ |
| $CH_3$ | $OCH_3$ | $CH_2CH_2$ | H | H | $CH(4\text{-}F\text{-}C_6H_3)$ |
| $CH_3$ | $OCH_3$ | $CH_2CH_2$ | H | H | 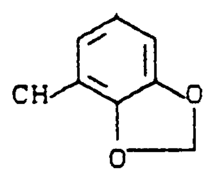 |
| $CH_3$ | $OCH_3$ | $CH_2CH_2$ | H | H | 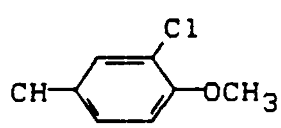 |
| $CH_3$ | $OCH_3$ | $CH_2CH_2$ | H | H | 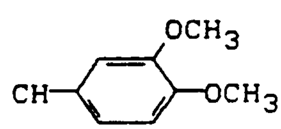 |

Tabelle 1 - Fortsetzung

| $R^1$ | $R^2$ | A | R | Q | B |
|---|---|---|---|---|---|
| $CH_3$ | $OCH_3$ | $CH_2CH_2$ | H | H | CH 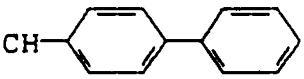 |
| $CH_3$ | $OCH_3$ | $CH_2CH_2$ | H | H | CH 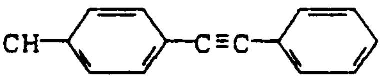 |
| $CH_3$ | $OCH_3$ | $CH_2CH_2$ | H | H | CH 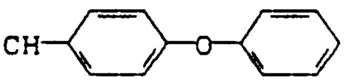 |
| $CH_3$ | $OCH_3$ | $CH_2CH_2$ | H | H | CH 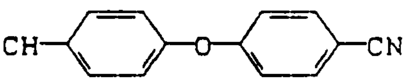 |
| $CH_3$ | $OCH_3$ | $CH_2CH_2$ | H | H | CH 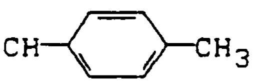 |
| $CH_3$ | $OCH_3$ | $CH_2CH_2$ | H | H | CH 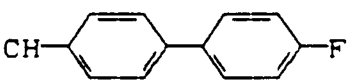 |
| $CH_3$ | $OCH_3$ | $CH_2CH_2$ | H | H | $CH-(2-F-3-Cl-C_6H_3)$ |
| $CH_3$ | $OCH_3$ | $CH_2CH_2$ | H | H | $CH-(2-Cl-3-F-C_6H_3)$ |
| $CH_3$ | $OCH_3$ | $CH_2CH_2$ | H | H | HC 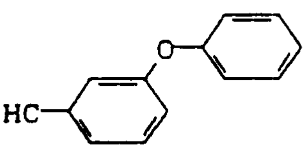 |
| $CH_3$ | $OCH_3$ | $CH_2CH_2$ | H | H | HC 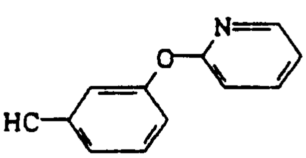 |

Tabelle 1 - Fortsetzung

| $R^1$ | $R^2$ | A | R | Q | B |
|---|---|---|---|---|---|
| $CH_3$ | $OCH_3$ | $CH_2CH_2$ | H | H | 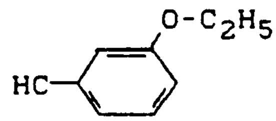 |
| $CH_3$ | $OCH_3$ | $CH_2CH_2$ | H | H | 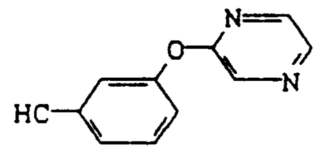 |
| $CH_3$ | $OCH_3$ | $CH_2CH_2$ | H | H | 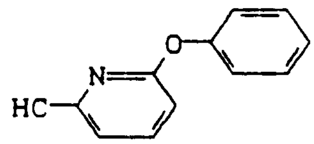 |
| $CH_3$ | $OCH_3$ | $CH_2CH_2$ | H | H | $CH-[2,4,6-(CH_3)_3C_6H_2]$ |
| $CH_3$ | $OCH_3$ | $CH_2CH_2$ | H | H | 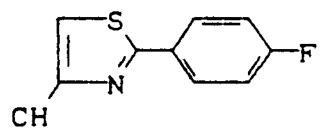 |
| $CH_2CH_3$ | $OCH_3$ | $CH_2CH_2$ | H | H | $CH-C_6H_5$ |
| $CH_2CH_3$ | $OCH_3$ | $CH_2CH_2$ | H | H | $CH-(2,4-Cl_2-C_6H_3)$ |
| $CH_2CH_3$ | $OCH_3$ | $CH_2CH_2$ | H | H | $CH-(3,4-Cl_2-C_6H_3)$ |
| $CH_3$ | $OCH_3$ | - | H | H | 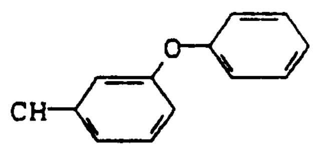 |
| $CH_3$ | $OCH_3$ | - | H | H | 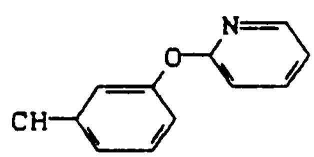 |

Tabelle 1 - Fortsetzung

| $R^1$ | $R^2$ | A | R | Q | B |
|---|---|---|---|---|---|
| $CH_3$ | $OCH_3$ | - | H | H | 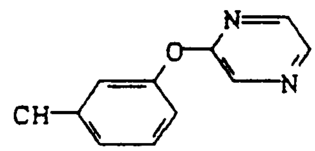 |
| $CH_3$ | $OCH_3$ | - | H | H | 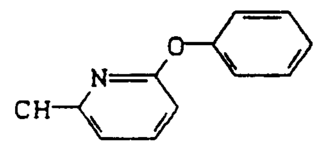 |
| $CH_3$ | $OCH_3$ | - | H | H | 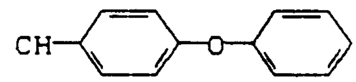 |
| $CH_3$ | $OCH_3$ | $CH_2$ | H | H | 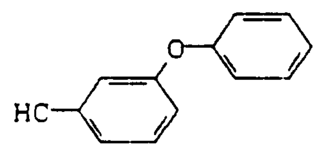 |
| $CH_3$ | $OCH_3$ | $CH_2$ | H | H | 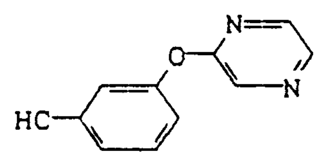 |
| $CH_3$ | $OCH_3$ | $CH_2$ | H | H | 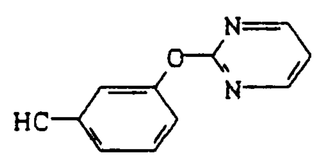 |
| $CH_3$ | $OCH_3$ | $CH_2$ | H | H | 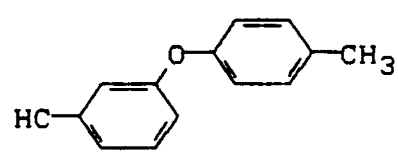 |

<u>Tabelle 1</u> - Fortsetzung

| $R^1$ | $R^2$ | A | R | Q | B |
|---|---|---|---|---|---|
| $CH_3$ | $OCH_3$ | $CH_2$ | H | H | 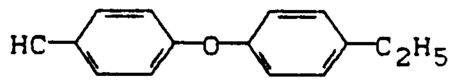 |
| $CH_3$ | $OCH_3$ | O | H | H | 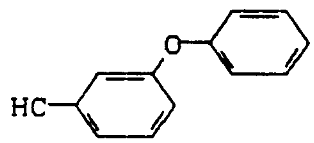 |
| $CH_3$ | $OCH_3$ | O | H | H | 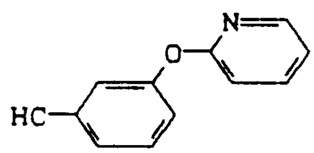 |
| $CH_3$ | $OCH_3$ | O | H | H | 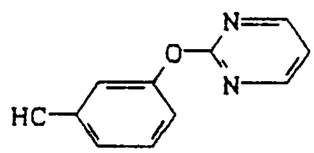 |
| $CH_3$ | $OCH_3$ | O | H | H | 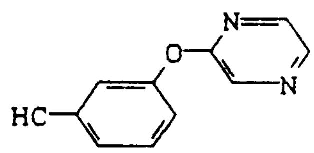 |
| $CH_3$ | $OCH_3$ | O | H | H | 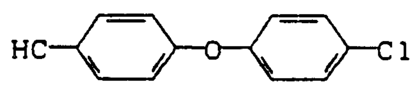 |
| $CH_3$ | $OCH_3$ | S | H | H | 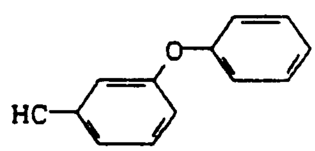 |
| $CH_3$ | $OCH_3$ | S | H | H | 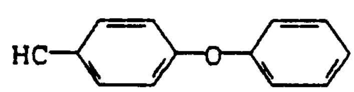 |

Tabelle 1 - Fortsetzung

| $R^1$ | $R^2$ | A | R | Q | B |
|---|---|---|---|---|---|
| $CH_3$ | $OCH_3$ | S | H | H | 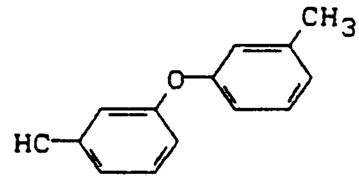 |
| $CH_3$ | $OCH_3$ | S | H | H | 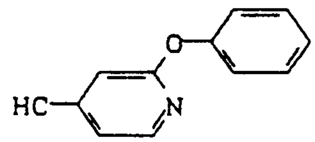 |
| $CH_3$ | $OCH_3$ | S | H | H | 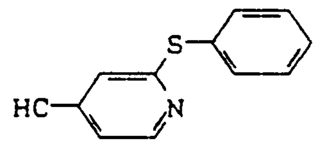 |
| $CH_3$ | $OCH_3$ | $C(CH_3)_2$ | H | H | 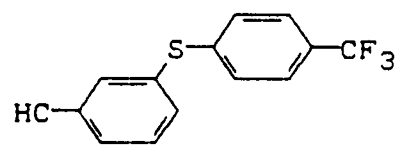 |
| $CH_3$ | $OCH_3$ | $C(CH_3)_2$ | H | H | 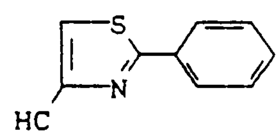 |
| $CH_3$ | $OCH_3$ | $C(CH_3)_2$ | H | H | 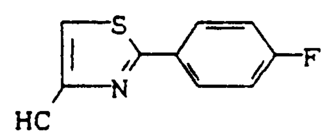 |
| $CH_3$ | $OCH_3$ | $C(CH_3)_2$ | H | H | 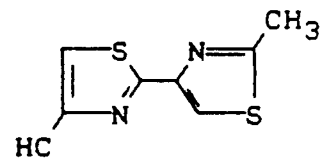 |
| $CH_3$ | $OCH_3$ | $C(CH_3)_2$ | H | H | 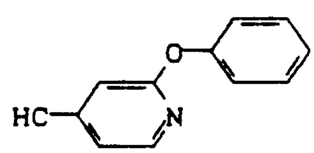 |

Tabelle 1 - Fortsetzung

| $R^1$ | $R^2$ | A | R | Q | B |
|---|---|---|---|---|---|
| $CH_3$ | $OCH_3$ | $NCH_3$ | H | H | 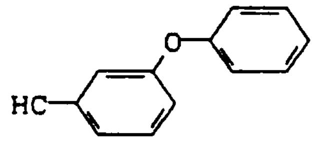 |
| $CH_3$ | $OCH_3$ | $NCOCH_3$ | H | H | 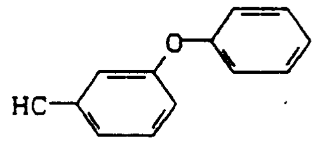 |
| $CH_3$ | $OCH_3$ | $NCOOC_2H_5$ | H | H | 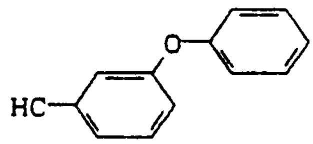 |
| $CH_3$ | $OCH_3$ | 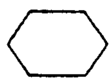 | H | H | 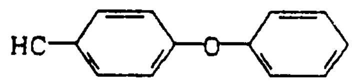 |
| $CH_3$ | $OCH_3$ | 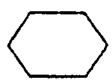 | H | H | 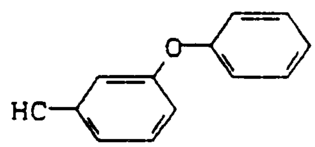 |
| $CH_3$ | $OCH_3$ | O | H | H | $CH-(2,3-Cl_2-C_6H_3)$ |
| $CH_3$ | $OCH_3$ | O | H | H | 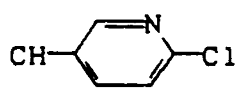 |
| $CH_3$ | $OCH_3$ | O | H | H | 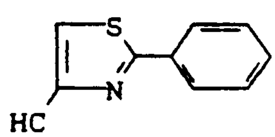 |
| $CH_3$ | $OCH_3$ | O | H | H | $CH-(3-F-4-Cl-C_6H_3)$ |

Tabelle 1 - Fortsetzung

| $R^1$ | $R^2$ | A | R | Q | B |
|---|---|---|---|---|---|
| $CH_3$ | $OCH_3$ | S | H | H | $CH-(2,6-Cl_2-C_6H_3)$ |
| $CH_3$ | $OCH_3$ | S | H | H | $CH-(2,4,6-Cl_3-C_6H_2)$ |
| $CH_3$ | $OCH_3$ | S | H | H | 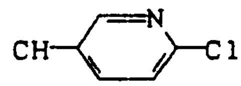 |
| $CH_3$ | $OCH_3$ | S | H | H | 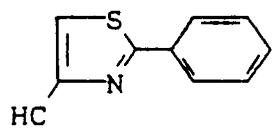 |
| $CH_3$ | $OCH_3$ | $N-COOC_2H_5$ | H | H | $CH-C_6H_5$ |
| $CH_3$ | $OCH_3$ | $N-COOC_2H_5$ | H | H | 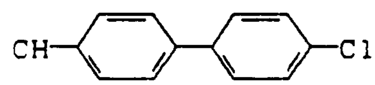 |
| $CH_3$ | $OCH_3$ | $N-COOC_2H_5$ | H | H | 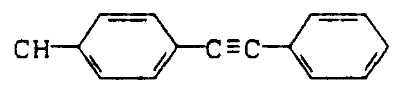 |
| $CH_3$ | $OCH_3$ | $N-COOC_2H$ | H | H | 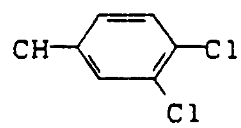 |
| $CH_3$ | $OCH_3$ | $N-CH_3$ | H | H | $CH-(2-F-C_6H_4)$ |
| $CH_3$ | $OCH_3$ | $N-CH_3$ | H | H | $CH-(3-F-C_6H_4)$ |
| $CH_3$ | $OCH_3$ | $N-CO-CH_3$ | H | H | $CH-C_6H_5$ |
| $CH_3$ | $OCH_3$ | $N-CO-CH_3$ | H | H | 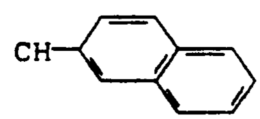 |
| $CH_3$ | $OCH_3$ | 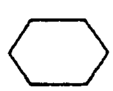 | H | H | $CH-C_6H_5$ |

Tabelle 1 - Fortsetzung

| $R^1$ | $R^2$ | A | R | Q | B |
|---|---|---|---|---|---|
| $CH_3$ | $OCH_3$ | 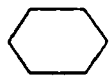 | H | H | $CH-(3,4-Cl_2-C_6H_3)$ |
| $CH_3$ | $OCH_3$ | 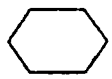 | H | H | $CH-(4-Cl-C_6H_4)$ |
| $CH_2CH_3$ | $OCH_3$ | 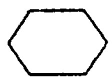 | H | H | $CH-C_6H_5$ |
| $CH_3$ | $OCH_3$ | - | H | H | $N-OCH_3$ |
| $CH_3$ | $OCH_3$ | - | H | H | $N-OCH_2-CH=CH_2$ |
| $CH_3$ | $OCH_3$ | - | H | H | $N-OCH_2-C\equiv CH$ |
| $CH_3$ | $OCH_3$ | - | H | H | $N-OCH_2-C_6H_5$ |
| $CH_3$ | $OCH_3$ | - | H | H | $N-O-C_6H_5$ |
| $CH_3$ | $OCH_3$ | - | H | H | $N-OCH_2-CH_2-CH_2CH_3$ |
| $CH_3$ | $OCH_3$ | - | H | H | $N-OCH_2-C\equiv N$ |
| $CH_2CH_3$ | $OCH_3$ | $CH_2$ | H | H | $N-OCH_3$ |
| $CH_2CH_3$ | $OCH_3$ | $CH_2$ | H | H | $N-OCH_2-CH=CH_2$ |
| $CH_3$ | $OCH_3$ | $CH_2$ | H | H | $N-OCH_2-(4-Cl-C_6H_4)$ |
| $CH_3$ | $OCH_3$ | $CH_2$ | H | H | $N-OCH_2COOCH_3$ |
| $CH_3$ | $OCH_3$ | $CH_2$ | H | H | $N-OCH_2COOC_2H_5$ |
| $CH_3$ | $OCH_3$ | $CH_2$ | H | H | $N-OCH_2-C\equiv CH$ |
| $CH_3$ | $OCH_3$ | $CH_2$ | H | H | $N-OCH_2-C\equiv N$ |
| $CH_3$ | $OCH_3$ | $CH_2CH_2$ | H | H | $NOCH_3$ |

Tabelle 1 - Fortsetzung

| $R^1$ | $R^2$ | A | R | Q | B |
|---|---|---|---|---|---|
| $CH_3$ | $OCH_3$ | $CH_2CH_2$ | H | H | $NOC_2H_5$ |
| $CH_3$ | $OCH_3$ | $CH_2CH_2$ | H | H | $NO-CH_2-CH=CH_2$ |
| $CH_3$ | $OCH_3$ | O | H | H | $NO-CH_2-CH=CH_2$ |
| $CH_3$ | $OCH_3$ | O | H | H | $NO-CH_3$ |
| $CH_3$ | $OCH_3$ | O | H | H | $NO-CH_2COOCH_3$ |
| $CH_3$ | $OCH_3$ | S | H | H | $NOCH_3$ |
| $CH_3$ | $OCH_3$ | S | H | H | $NOCH_2CH=CH_2$ |
| $CH_3$ | $OCH_3$ | S | H | H | $NOCH_2C\equiv CH$ |
| $CH_3$ | $OCH_3$ | $NCH_3$ | H | H | $NOCH_3$ |
| $CH_3$ | $OCH_3$ | $NCH_3$ | H | H | $NOCH_2CH=CH_2$ |
| $CH_3$ | $OCH_3$ | $N-C-CH_3$<br>‖<br>O | H | H | $NOCH_3$ |
| $CH_3$ | $OCH_3$ | $N-C-CH_3$<br>‖<br>O | H | H | $NOCH_2CH=CH_2$ |
| $CH_3$ | $OCH_3$ | $N-COOCH_3$ | H | H | $NOCH_3$ |
| $CH_3$ | $OCH_3$ | $N-COOC_2H_5$ | H | H | $NOCH_2CH=CH_2$ |
| $CH_3$ | $OCH_3$ | $C(CH_3)_2$ | H | H | $NOC_2H_5$ |
| $CH_3$ | $OCH_3$ | $C(CH_3)_2$ | H | H | $NOCH_2CH=CH_2$ |
| $CH_3$ | $OCH_3$ | $CH-C(CH_3)_3$ | H | H | $NO-C_3H_7-n$ |
| $CH_3$ | $OCH_3$ | $CH-C(CH_3)_3$ | H | H | $NOCH_2CH=CH_2$ |
| $CH_3$ | $OCH_3$ | 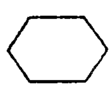 | H | H | $NO-C_3H_7-n$ |

Tabelle 1 - Fortsetzung

| $R^1$ | $R^2$ | A | R | Q | B |
|---|---|---|---|---|---|
| $CH_3$ | $OCH_3$ | 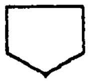 | H | H | $NO-C_3H_7$ |
| $CH_3$ | $OCH_3$ | 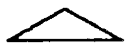 | H | H | $NOCH_2CH=CH_2$ |
| $CH_3$ | $OCH_3$ | 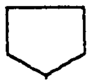 | H | H | $NOCH_2CH=CH_2$ |
| $CH_3$ | $OCH_3$ | $CH-CH_3$ | H | H | $NOCH_2CH=CH_2$ |
| $CH_3$ | $OCH_3$ | $CH-CH_3$ | H | H | $NOCH_2C≡CH$ |
| $CH_3$ | $OCH_3$ | -CH 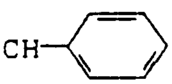 | H | H | $NOCH_2CH_3$ |

Verwendet man beispielsweise 3-Hydroxy-2-[2-(2-(2,3-difluorphenyl)-ethenyl)-cyclopenten-1-yl]-acrylsäuremethylester und Dimethylsulfat als Ausgangsstoffe, so läßt sich der Reaktionsablauf des erfindungsgemäßen Verfahrens (a) durch das folgende Reaktionsschema darstellen:

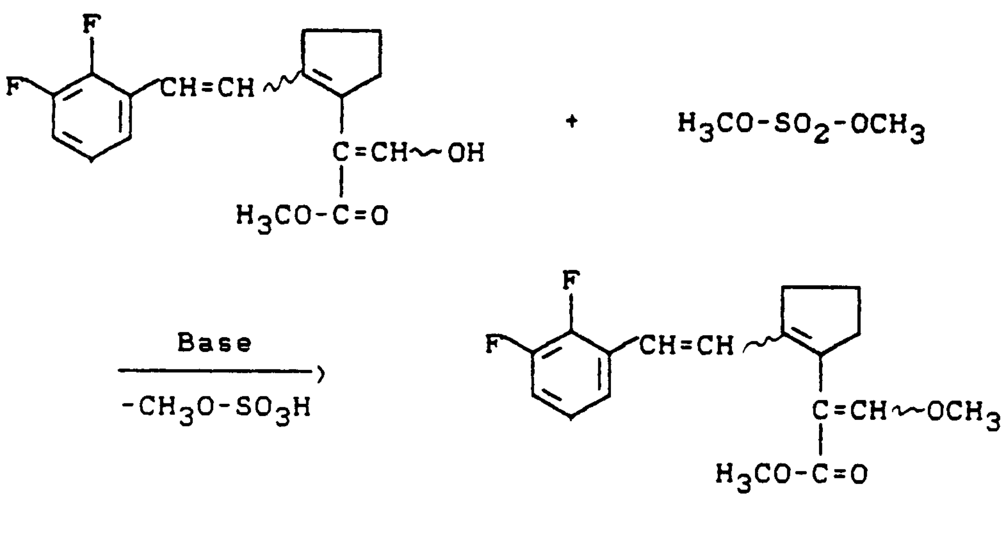


Verwendet man beispielsweise 2-[[2-(4-Chlor-3-methoxyphenyl)-ethenyl]-cyclohexen-1-yl]-essigsäuremethylester und Dimethylformamiddimethylacetal als Ausgangsstoffe, so läßt sich der Reaktionsablauf des erfindungsgemäßen Verfahrens (b) durch das folgende Reaktionsschema darstellen:

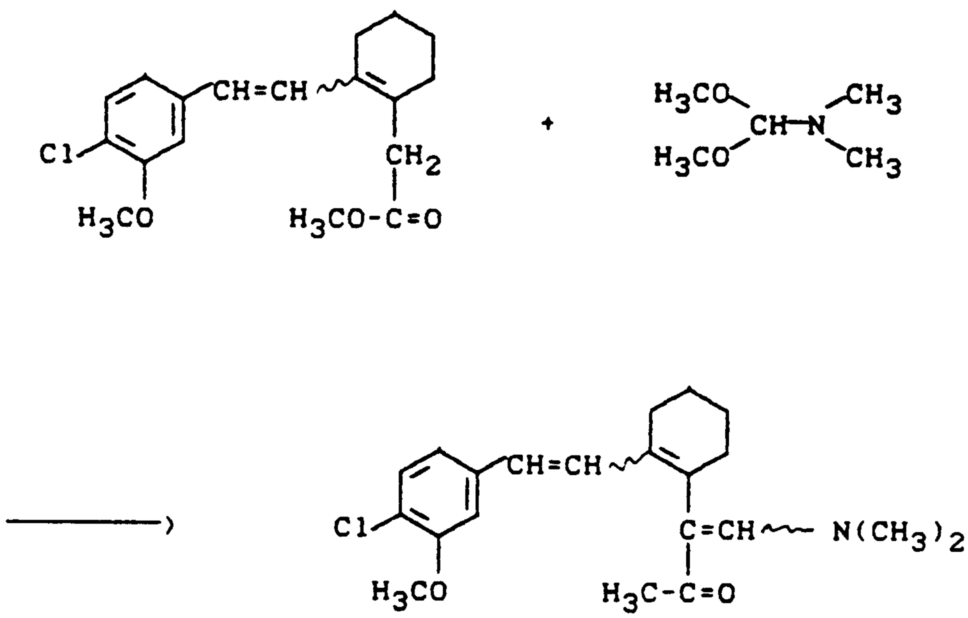

Verwendet man beispielsweise 2-Oxo[[2-(2-Phenyl)-ethenyl]-cyclohepten-1-yl]-essigsäuremethylester und [-(Methylthio)-(trimethylsilyl)]-methylenlithium als Ausgangsverbindungen, so läßt sich der Reaktionsablauf des erfindungsgemäßen Verfahrens (c) durch das folgende Formelschema darstellen:

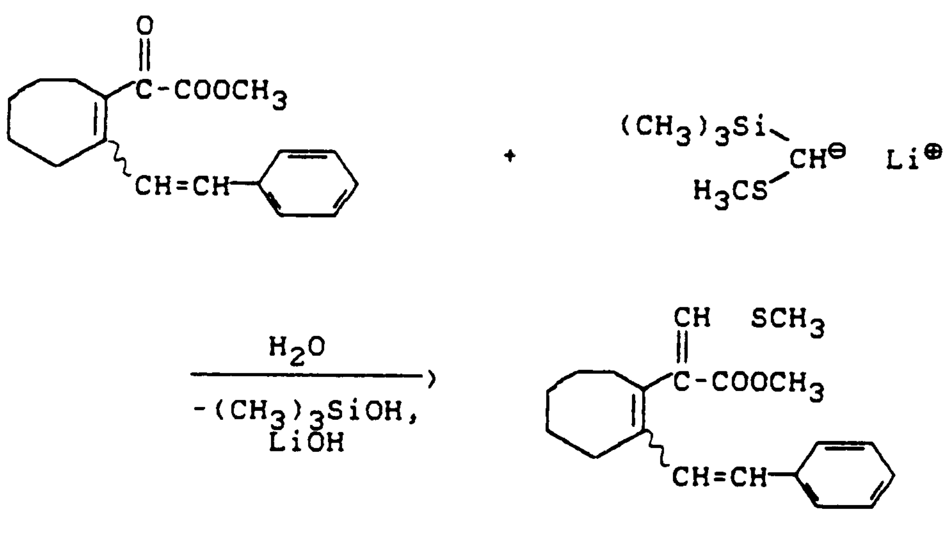


Verwendet man beispielsweise 3-Methansulfonyloxy-[[2-(Pyridin-2-yl)-ethenyl]-4-thia-cyclohexen-1-yl]-acrylsäuremethylester und Methylmercaptan als Ausgangsstoffe, so läßt sich der Reaktionsablauf des erfindungsgemäßen Verfahrens (d) durch das folgende Reaktionsschema darstellen:

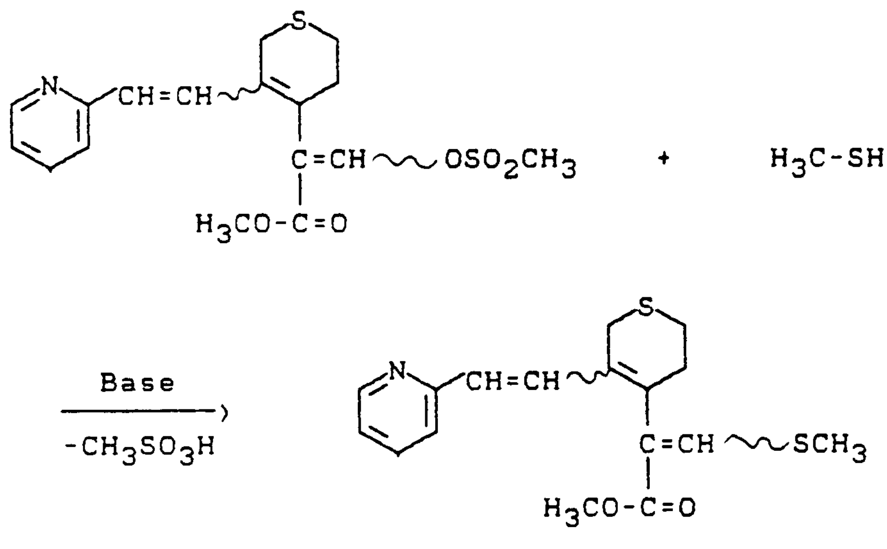


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$, A, B, R und Q vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Lithium-, Natrium- oder Kaliumkation.

Die zur Durchführung des erfindungsgemäßen Verfahrens (a) benötigten Hydroxyacrylsäureester der Formel (II) sind noch nicht bekannt und Gegenstand der Erfindung.

Man erhält sie, wenn man substituierte Essigsäureester der Formel (IV),

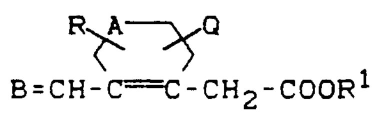
(IV)

in welcher

$R^1$, A, B, R und Q die oben angegebene Bedeutung haben,

mit Ameisensäureestern der Formel (X),

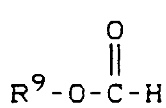
(X)

in welcher

$R^9$ für Alkyl, insbesondere für Methyl oder Ethyl steht,

gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen von -20 °C bis +50 °C umsetzt.

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester der allgemeinen Formel (IV) sind neu und Gegenstand der Erfindung. In dieser Formel (IV) stehen $R^1$, A und B vorzugsweise für diejenigen Reste, die bereits im Zusammenhang mit der Beschreibung der erfindungsgemäßen Stoffe der Formel (I) als bevorzugt für diese Substituenten genannt wurden.

Man erhält die Verbindungen der Formel (IV)

a) für den Fall, daß $R^1$, A, R und Q die oben angegebene Bedeutung haben, und

$B^1$ für die Gruppe N-O-$R^9$ steht,

wobei

$R^9$ die oben angegebene Bedeutung hat,

indem man Aldehyde der allgemeinen Formel (XI)

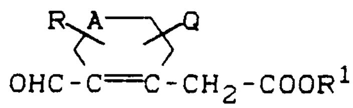

(XI)

in welcher

$R^1$, A, R und Q die oben angegebene Bedeutung haben,

mit Verbindungen der allgemeinen Formel (XII)

$R^9$-O-$NH_2$ (XII)

in welcher

$R^9$ die oben angegebene Bedeutung hat,

gegebenenfalls in Gegenwart eines Verdünnungsmittels wie beispielsweise Methanol bei Temperaturen von -20 °C bis +50 °C umsetzt (vgl. Herstellungsbeispiele).

Verbindungen der allgemeinen Formel (XII) sind allgemein bekannte Verbindungen der organischen Chemie oder erhältlich in Analogie zu allgemein bekannten Verfahren (vgl. z.B. GB 1042191; Tetrahedron Lett. 23 2955-6 (1982); DE-OS 3615473).

Man erhält sie weiterhin

b) für den Fall, daß $R^1$, A, R und Q die oben angegebene Bedeutung haben, und

$B^2$ für die Gruppe CH-$R^8$ steht, wobei

$R^8$ die oben angegebene Bedeutung hat, indem man Aldehyde der allgemeinen Formel (XI)

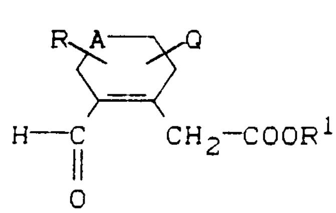

(XI)

in der

$R^1$, A, R und Q die oben angegebene Bedeutung haben,

α) mit Phosphoniumverbindungen der allgemeinen Formel (XIII)

$R^8$-$CH_2$-$P(R^{12})_3^{\oplus}X^{\ominus}$ (XIII)

in welcher

$R^8$ die oben angegebene Bedeutung hat,

$R^{12}$ für gegebenenfalls durch Methyl, Ethyl, Methoxy, Ethoxy substituiertes Aryl, bevorzugt Phenyl und

X für Halogen, bevorzugt Brom oder Chlor steht, oder

β) mit Reagenzien der allgemeinen Formel (XIV)

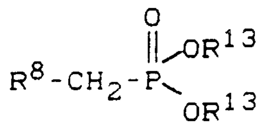

(XIV)

in welcher

$R^8$ die oben angegebene Bedeutung hat und

$R^{13}$ für $C_{1-4}$Alkyl, besonders bevorzugt Methyl, Ethyl oder n-Butyl steht,

unter Verwendung eines unter den Reaktionsbedingungen inerten Lösungsmittels, wie beispielsweise Tetrahydrofuran und gegebenenfalls eines basischen Hilfsmittels wie beispielsweise Natriumhydrid, Butyllithium oder Kalium-tert.-butylat bei Temperaturen von -80 °C bis +60 °C, im Sinne einer WITTIG-Reaktion bzw. einer HORNER-WITTIG-Reaktion umsetzt (vgl. Herstellungsbeispiele).

Sowohl die Verbindungen der allgemeinen Formel (XIII) als auch die der allgemeinen Formel (XIV) sind allgemein bekannte Verbindungen der organischen Chemie oder lassen sich in Analogie zu allgemein bekannten Verfahren synthetisieren (vgl. z.B. L.-F. Tietze, Th. Eicher "Reaktionen und Synthesen im organisch-chemischen Praktikum" S. 165 und 167, Thieme Verlag Stuttgart, New York 1981).

Die Aldehyde der allgemeinen Formel (XI) sind teilweise bekannt und/oder lassen sich in Analogie zu bekannten Verfahren synthetisieren (vgl. u.a. J.Chem.Soc. Perkin I 2005 (1974)).

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht $R^{2-1}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{2-1}$ steht ganz besonders bevorzugt für Dimethylamino oder Diethylamino.

$R^{10}$ und $R^{11}$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest, mit jeweils 1 bis 6 insbesondere mit 1 bis 4 Kohlenstoffatomen in den einzelnen geradkettigen oder verzweigten Alkylteilen.

Die Formamide der Formel (Va) und deren Derivate der Formel (Vb) sind allgemein bekannte Verbindungen der organischen Chemie.

Die zur Durchführung des erfindungsgemäßen Verfahrens (c) als Ausgangsstoffe benötigten Ketocarbonsäurederivate sind durch die Formel (VI) allgemein definiert.

In dieser Formel (VI) stehen $R^1$, A, B, R und Q vorzugsweise für diejenigen Reste, die bereits im Zusammenhang mit der Beschreibung der erfindungsgemäßen Stoffe der Formel (I) als bevorzugt für diese Substituenten genannt wurden.

Die Ketocarbonsäurederivate der allgemeinen Formel (VI) sind teilweise bekannt und/oder können nach allgemein bekannten Verfahren, wie beispielsweise durch Oxidation von Verbindungen der allgemeinen Formel (IV), mit Hilfe eines geeigneten Oxidationsmittels, wie beispielsweise Pyridiniumchlorochromat oder Pyridiniumdichromat und unter Verwendung geeigneter inerter Verdünnungsmittel, beispielsweise chlorierter Kohlenwasserstoffe, wie Dichlormethan, bei Temperaturen von +20 °C bis +100 °C hergestellt werden. Pyridiniumchlorochromat und Pyridiniumdichromat sind allgemein bekannte Verbindungen der organischen Chemie.

Die zur Durchführung des erfindungsgemäßen Verfahrens (c) weiterhin als Ausgangsstoffe benötigten metallorganischen Verbindungen sind durch die Formel (VII) allgemein definiert. In dieser Formel (VII) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J.Org.Chem. 33, 780 [1968]; J.Org.Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert. In dieser Formel (VIII) stehen $R^1$, A, B, R und Q vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy-oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (II),

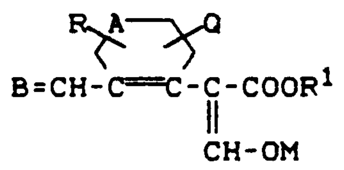

in welcher

M, $R^1$, A, B, R und Q die oben angegebene Bedeutung haben,

mit Säurechloriden der Formel (XIV),

$R^{14}$-Cl (XIV)

in welcher

$R^{14}$ für einen Acyl- oder Sulfonylrest, insbesondere für einen Acetyl-, einen Methansulfonyl- oder einen p-Toluolsulfonylrest steht,

gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen von -20 °C bis +120 °C umsetzt.

Säurechloride der Formel (XIV) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht $R^3$ vorzugsweise für diejenigen Reste, die bereits im Zusammenhang mit der Beschreibung der erfindungsgemaßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen von - 30 °C bis + 120 °C, vorzugsweise bei Temperaturen von - 20 °C bis + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol,

vorzugsweise 1.0 bis 2.0 Mol an Reaktionshilfsmittel ein.
Dabei ist es auch möglich, die als Ausgangsverbindungen zur Durchführung des erfindungsgemäßen Verfahrens (a) benötigten 3-Hydroxyacrylsaureester oder deren Alkalimetallsalze der Formel (II) in einer vorgelagerten Reaktion direkt im Reaktionsgefäß herzustellen und direkt aus dem Reaktionsgemisch heraus ohne Isolierung mit dem Alkylierungsmittel der Formel (III) gemäß dem erfindungsgemäßen Verfahren (a) weiter umzusetzen ("Eintopfverfahren").
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen von - 20 °C bis + 200 °C, vorzugsweise bei Temperaturen von 0 °C bis 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein.

Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Toluol, Xylol, Petrolether, Hexan oder Cyclohexan oder Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen von -100° C bis +10° C, vorzugsweise bei Temperaturen von -80° C bis +50° C.

Zur Durchführung des erfindungsgemäßen Verfahrens (c) setzt man pro Mol an Ketocarbonsäurederivat der Formel (VI) im allgemeinen 1.0 bis 1.5 Mol, vorzugsweise 1.0 bis 1.2 Mol an metallorganischer Verbindung der Formel (VII) ein.
Die Reaktionsdurchführung, Aufarbeitung und Isolierung der Reaktionsprodukte erfolgt nach bekannten Verfahren (vgl. z.B. J.Org.Chem. 33, 780 [1968]; J.Org.Chem. 37, 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemaßen Verfahrens (d) in einem größeren Bereich variiert werden. Im allgemeinen arbeitet man bei Temperaturen von -20° C bis 180° C, vorzugsweise bei Temperaturen von 0° C bis 150° C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.

Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide und Insektizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur protektiven Bekämpfung von Venturia-Arten an Äpfeln oder zur protektiven Bekämpfung von Reiskrankheiten, wie beispielsweise gegen den Erreger der Reisfleckenkrankheit (Pyricularia oryzae) oder zur protektiven Bekämpfung von Erysiphe an Gerste oder Weizen, oder zur protektiven Bekämpfung von Leptosphaeria nodorum an Weizen oder zur protektiven Bekämpfung von Cochliobolus sativus und Pyrenophora teres an Gerste einsetzen.

Darüberhinaus zeigen die erfindungsgemäßen Wirkstoffe außerdem eine fungizide Wirkung gegen Plasmopara, Cercosporella und Sclerotinia sowie eine gute in vitro Wirksamkeit.

Auch die neuen Zwischenprodukte der Formel (IV) zeigen in gewissem Umfang ebenfalls fungizide Wirkung.

Desweiteren eignen sich die Wirkstoffe zur Bekämpfung von tierischen Schädlingen, vorzugsweise Arthropoden und Nematoden, ins besondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehoren:
Aus der Ordnung der Isopoda z.B. Oniscus asellus, Armadillidium vulgare, Porcellio scaber.

Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.

Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globedera ssp., Meloidogyne spp., Aphelenchoides spp., Longidorus spp., Xiphinema spp., Trichodorus spp..

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,1 bis 95 Gewichtsprozent Wirkstoff, vorzugsweise 0,5 bis 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1

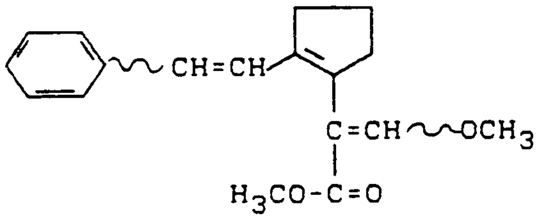

Zu einer gerührten Suspension von 1,24 g (41.26 mmol) Natriumhydrid (80%ige Suspension) in 30 ml Dimethylformamid läßt man bei 0-10 °C eine Lösung von 5,00 g (20.63 mmol) 1-(2-Phenyl-ethenyl)-cyclopenten-2-essigsäuremethylester in 30 ml Ameisensäuremethylester tropfen. Die anfänglich zinnoberrote Suspension geht im weiteren Verlauf der Reaktion in Lösung. Nach ca. 4 Stunden Nachrührzeit ist die Bildung des Enols vollständig. Bei ca. 20 ° C läßt man nun unter Rühren 5.20 g (41.23 mmol) Dimethylsulfat zutropfen und rührt bis zur völligen Umsetzung nach.

Zur Aufarbeitung verrührt man mit überschüssiger gesättigter Natriumhydrogencarbonatlösung, extrahiert mit Essigester, trocknet die vereinigten Extrakte über wasserfreiem Magnesiumsulfat, filtriert, engt ein und reinigt den verbleibenden Rückstand durch Säulenchromatographie an Silicagel (Laufmittel: Dichlormethan/n-Hexan 1:1). Man erhält 2.00 g (34.1% d. Theorie) E-3-Methoxy-2-[2(2-Phenyl-ethenyl)-cyclopenten-1-yl]-acrylsäuremethylester vom Fp. 107 ° C und einem cis/trans-Verhältnis von ca 1:7 nach $^{1}$H-NMR.

In analoger Weise zu dem im Beispiel 1 beschriebenen Verfahren und unter Berücksichtigung der Angaben in den Beschreibungen zu den erfindungsgemäßen Verfahren, werden die in der nachfolgenden Tabelle 2 aufgeführten Endprodukte der Formel (I)

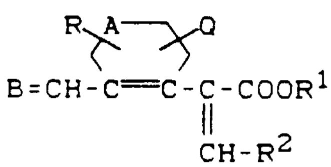

(I)

erhalten:

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 2 | H | H | $CH_3$ | $OCH_3$ | $CH_2$ | 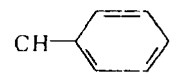 | | 1 : 7 | Fp: 88-92° C |
| 3 | H | H | $CH_3$ | $OCH_3$ | $CH_2$ | 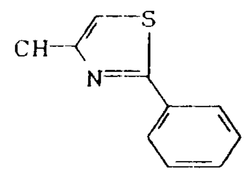 | E | E | Fp: 114-117° C |
| 4 | H | H | $CH_3$ | $OCH_3$ | $CH_2$ | 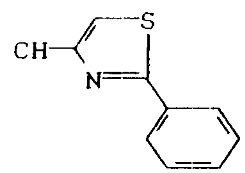 | Z | E | $^1$H-NMR α) |
| 5 | H | H | $CH_3$ | $OCH_3$ | $CH_2$ | 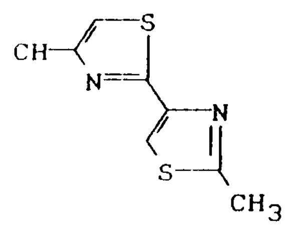 | E | E | Fp: 118-119° C |
| 6 | H | H | $CH_3$ | $OCH_3$ | $CH_2$ | 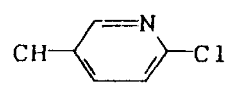 | E | 1 : 5 | Fp: 87-89° C |
| 7 | H | H | $CH_3$ | $OCH_3$ | $CH_2$ | 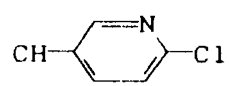 | Z | 1 : 5 | Fp: 105-107° C |

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 8 | H | H | $CH_3$ | $OCH_3$ | - | 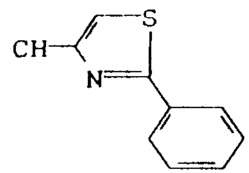 | E | E | Fp:105-106° C |
| 9 | H | H | $CH_3$ | $OCH_3$ | - | 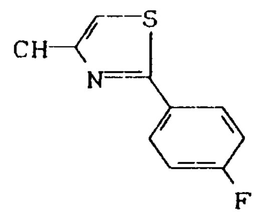 | E | E | $^1H$-NMR β) |
| 10 | H | H | $CH_3$ | $OCH_3$ | $CH_2-CH_3$ | 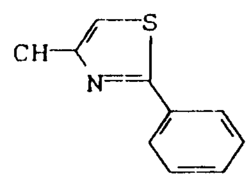 | | | |
| 11 | H | H | $CH_3$ | $OCH_3$ | - | 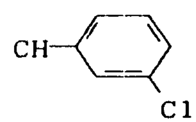 | E | E | Fp.: 110° C |
| 12 | H | H | $CH_3$ | $OCH_3$ | - | 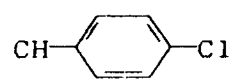 | E | E | δ=7,50 |

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 13 | H | H | $CH_3$ | $OCH_3$ | - | 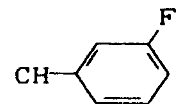 | E | E | Fp.: 100-102° C |
| 14 | H | H | $CH_3$ | $OCH_3$ | - | 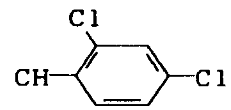 | E | E | Fp.: 56-58° C |
| 15 | H | H | $CH_3$ | $OCH_3$ | - | 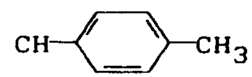 | E | E | δ=7,45 |
| 16 | H | H | $CH_3$ | $OCH_3$ | - | 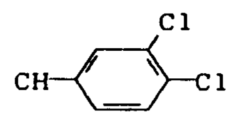 | E | E | Fp.: 86-87° C |
| 17 | H | H | $CH_3$ | $OCH_3$ | - | 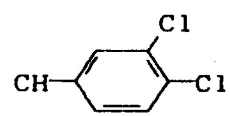 | E | E | Fp.: 74-76° C |
| 18 | H | H | $CH_3$ | $OCH_3$ | - | 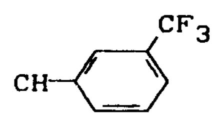 | E | E | Fp.: 76-77° C |

Tabelle 2

| Bsp.-Nr | R | Q | $R^1$ | $R^2$ | A | B | E/Z 1) | E/Z 2) | physikal. Konstanten |
|---|---|---|---|---|---|---|---|---|---|
| 19 | H | H | $CH_3$ | $OCH_3$ | - | 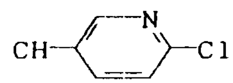 | E | 4:1 | Fp.:124-127° C |
| 20 | H | H | $CH_3$ | $OCH_3$ | - | 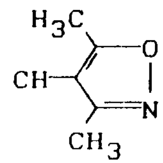 | 5:2 | 4:1 | ms:m/e=303 |
| 21 | H | H | $CH_3$ | $OCH_3$ | - | 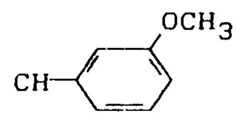 | E | 6.1 | δ=3,65 |
| 22 | H | H | $CH_3$ | $OCH_3$ | - | 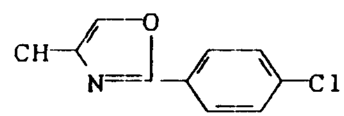 | E | E | ms:m/e=385 |
| 23 | H | H | $CH_3$ | $OCH_3$ | - | 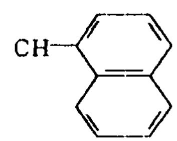 | E | 5:1 | ms:m/e=334 |
| 24 | H | H | $CH_3$ | $OCH_3$ | - | 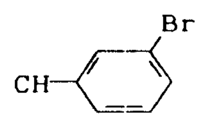 | E | E | ms:m/e=364 |

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 25 | H | H | $CH_3$ | $OCH_3$ | - | 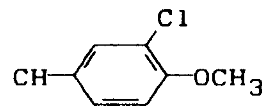 | E | E | ms:m/e=348 |
| 26 | H | H | $CH_3$ | $OCH_3$ | - | 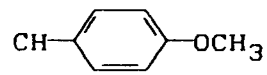 | E | E | Fp.:104-106° C |
| 27 | H | H | $CH_3$ | $OCH_3$ | - | 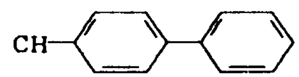 | E | E | Fp.:162-165° C |
| 28 | H | H | $CH_3$ | $OCH_3$ | - | 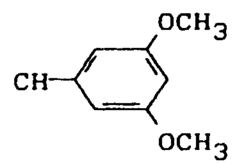 | E | 8:1 | ms:m/e=344 |
| 29 | H | H | $CH_3$ | $OCH_3$ | - | 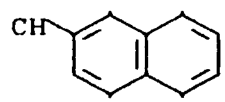 | E | E | ms:m/e=334 |
| 30 | H | H | $CH_3$ | $OCH_3$ | - | 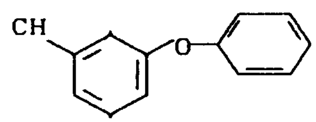 | E | E | ms:m/e=376 |

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 31 | H | H | $CH_3$ | $OCH_3$ | - | 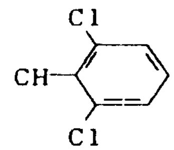 | E | E | Fp.: 96-99° C |
| 32 | H | H | $CH_3$ | $OCH_3$ | - | $CH-C_3H_7n$ | E | E | ms:m/e=250 |
| 33 | H | H | $CH_3$ | $OCH_3$ | - | 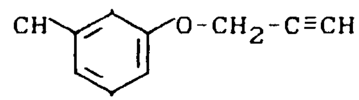 | | | ms:m/e=338 |
| 34 | H | H | $CH_3$ | $OCH_3$ | - | 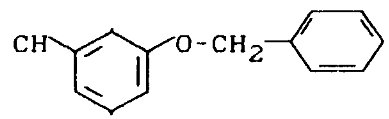 | E | E | δ=5,05 |
| 35 | H | H | $CH_3$ | $OCH_3$ | - | 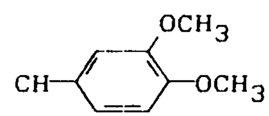 | E | E | Fp.:108-109° C |
| 36 | H | H | $CH_3$ | $OCH_3$ | - | 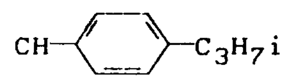 | E | E | ms:m/e=326 |
| 37 | H | H | $CH_3$ | $OCH_3$ | - | 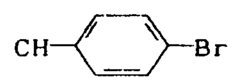 | E | E | Fp.:124-125° C |

Tabelle 2

| Bsp.-Nr | R | Q | $R^1$ | $R^2$ | A | B | E/Z 1) | E/Z 2) | physikal. Konstanten |
|---|---|---|---|---|---|---|---|---|---|
| 38 | H | H | $CH_3$ | $OCH_3$ | - | 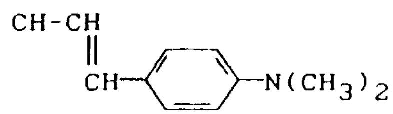 | E | E | Fp.:194-196° C |
| 39 | H | H | $CH_3$ | $OCH_3$ | - | 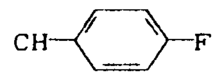 | E | E | Fp.:112-113° C |
| 40 | H | H | $CH_3$ | $OCH_3$ | - | 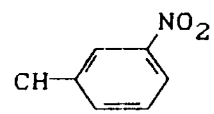 | E | E | ms:m/e=329 |
| 41 | H | H | $CH_3$ | $OCH_3$ | - | 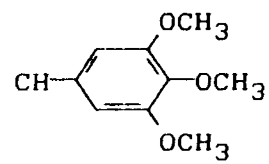 | E | E | Fp.:144-146° C |
| 42 | H | H | $CH_3$ | $OCH_3$ | - | 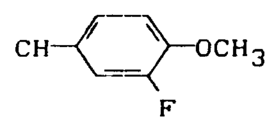 | E | E | Fp.:124-125° C |

Tabelle 2

| Bsp.-Nr | R | Q | $R^1$ | $R^2$ | A | B | E/Z[1)] | E/Z[2)] | physikal. Konstanten |
|---|---|---|---|---|---|---|---|---|---|
| 43 | H | H | $CH_3$ | $OCH_3$ | - | 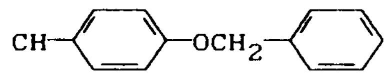 | E | E | Fp.:130-131° C |
| 44 | H | H | $CH_3$ | $OCH_3$ | - | 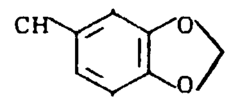 | E | E | Fp.:118-119° C |
| 45 | H | H | $CH_3$ | $OCH_3$ | - | 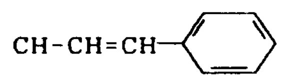 | E | E | Fp.: 76-77° C |
| 46 | H | H | $CH_3$ | $OCH_3$ | - | 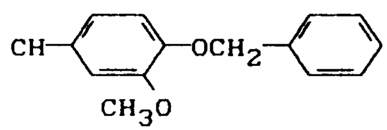 | E | E | Fp.: 98-99° C |
| 47 | H | H | $CH_3$ | $OCH_3$ | - | 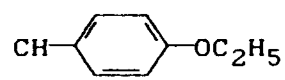 | E | E | Fp.:114-116° C |
| 48 | H | H | $CH_3$ | $OCH_3$ | - | 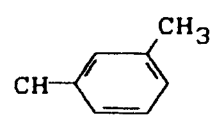 | E | E | Fp.: 72-742° C |

Tabelle 2

| Bsp.-Nr | R | Q | $R^1$ | $R^2$ | A | B | E/Z 1) | E/Z 2) | physikal. Konstanten |
|---|---|---|---|---|---|---|---|---|---|
| 49 | H | H | $CH_3$ | $OCH_3$ | - | 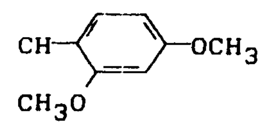 | E | E | δ= 3,65, 3,7 |
| 50 | H | H | $CH_3$ | $OCH_3$ | - | 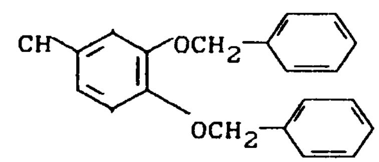 | E | E | Fp.:121-122° C |
| 51 | H | H | $CH_3$ | $OCH_3$ | - | 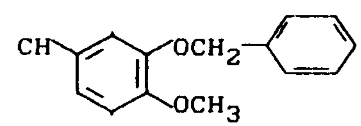 | E | E | Fp.: 64-65° C |
| 52 | H | H | $CH_3$ | $OCH_3$ | $CH_2$ | 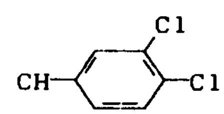 | E | E | Fp.: 80-81° C |
| 53 | H | H | $CH_3$ | $OCH_3$ | $CH_2$ | 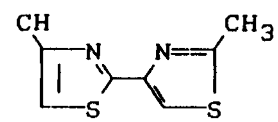 | E | E | Fp.:122-123° C |

Tabelle 2

| Bsp.-Nr | R | Q | $R^1$ | $R^2$ | A | B | E/Z 1) | E/Z 2) | physikal. Konstanten |
|---|---|---|---|---|---|---|---|---|---|
| 54 | H | H | $CH_3$ | $OCH_3$ | $CH_2$ | 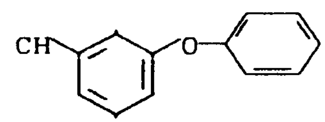 | E | 8:1 | δ=3,7 |
| 55 | H | H | $CH_3$ | $OCH_3$ | O | 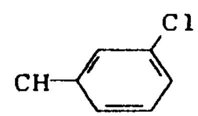 | E | E | δ= 6,2 |
| 56 | H | H | $CH_3$ | $OCH_3$ | O | 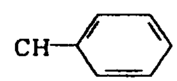 | E | 12:1 | δ=6,3 |
| 57 | H | H | $CH_3$ | $OCH_3$ | S | 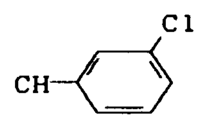 | E | 5:1 | Öl |

<u>Tabelle 2</u> - Erläuterungen

$^1$H-NMR-Daten:

α) ($CDCl_3$, δ/ppm) : δ= 3,75 (s, 3H, $OCH_3$), 3,89 (s, 3H, $OCH_3$), 6,41 (s, 1H), 6,58 (d, 1H), 7,05 (s, 1H), 7,50 (d, 1H)

β) ($CDCl_3$, δ/ppm) : δ= 3,75 (s, 3H, $OCH_3$, 3,85 (s, 3H, $OCH_3$), 6,49 (d, 1H), 7,03 (s, 1H)

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

1) an der Doppelbindung mit dem Substituenten $R^2$

2) an der Doppelbindung mit dem Substituenten B

Herstellung der Vorprodukte

Beispiel (IV-1)

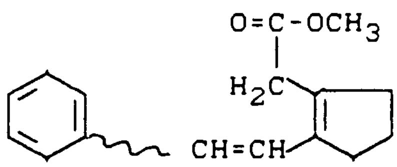

In ein unter Argon bei 0° C gerührtes Gemisch aus 280 ml Methanol, 140 ml Tetrahydrofuran und 8.12 g (150.1 mmol) Natriummethanolat werden portionsweise insgesamt 43.12 g (110.9 mmol) Benzyltriphenylphosphoniumchlorid gegeben. Man läßt ca. 30 Minuten lang nachrühren und fügt danach tropfenweise eine Lösung con 14.00 g (83.2 mmol) 2-Formyl-cyclopent-1-en-1-yl-essigsäuremethylester in 10 ml Tetrahydrofuran unter Kühlung während ca. 30 Minuten zu. Nach beendetem Zutropfen läßt man auf Raumtemperatur kommen und rührt bis zur beendeten Umsetzung nach. Zur Aufarbeitung versetzt man mit Eiswasser, extrahiert mit Essigsäureethylester, trocknet den Extrakt über wasserfreiem Magnesiumsulfat, engt ein und reinigt den Rückstand durch Chromatographiert an Silicagel (Laufmittel: Dichlormethan).

Man erhält 10.50 g (52.1% der Theorie) 1-(2-Phenyl-ethenyl)-cyclopenten-2-essigsäuremethylester mit einem nach $^1$H-NMR bestimmten cis/trans-Verhältnis von 1:3 und einem Gehalt von 95.4% nach GC.

$^1$H-NMR ($CDCl_3$, $\delta$/ppm): 3.62 (s, 3H, $COOCH_3$/cis-Isomer), 3,70 (s, 3H, $COOCH_3$/ trans-Isomere, 6,22 (d, 1H, CH=/cis-Isomer), 6,48 (d, 1H, CH=/trans-Isomer), 6,51 (d, 1H, CH=/cis-Isomer), 7.03 (d, 1H, CH=/trans-Isomer); $J_{CH=CH(cis)}$ = 12,1 Hz, $J_{CH=CH(trans)}$ = 15,9 Hz.

In analoger Weise zu Beispiel (IV-1) und unter Berücksichtigung der Angaben in den Beschreibungen zu den erfindungsgemäßen Verfahren werden die in der nachfolgenden Tabelle 3 aufgeführten Vorprodukte der Formel (IV)

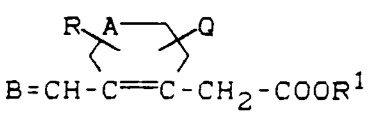

(IV)

erhalten.

Tabelle 3

| Bsp.-Nr | R | Q | $R^1$ | A | B | cis/ trans | physikal. Konstanten (Massenspektroskopie) |
|---|---|---|---|---|---|---|---|
| IV-2 | H | H | $CH_3$ | - | 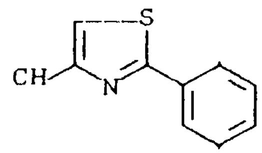 | trans | MS: 326($M^{\oplus}$), 252(Basispeak) |
| IV-3 | H | H | $CH_3$ | - | 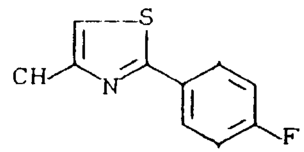 | trans | MS: 343($M^{\oplus}$), 270(Basispeak), 193 |
| IV-4 | H | H | $CH_3$ | - | 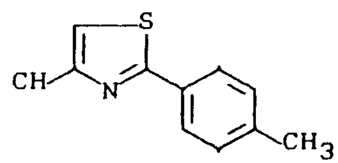 | trans | MS: 339($M^{\oplus}$), 266(Basispeak), 189, 135 |
| IV-5 | H | H | $CH_3$ | - | 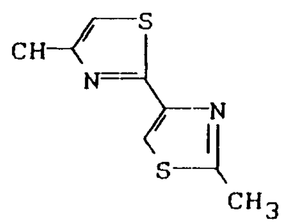 | trans | MS: 346($M^{\oplus}$), 313, 273 (Basispeak) |
| IV-6 | H | H | $CH_3$ | $CH_2$ | 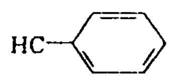 | 32 : 1 | MS: 256($M^{\oplus}$) und (Basispeak) |

Tabelle 3 - Fortsetzung

| Bsp.-Nr | R | Q | $R^1$ | A | B | cis/ trans | physikal.Konstante (Massenspektroskopie |
|---|---|---|---|---|---|---|---|
| IV-7 | H | H | $CH_3$ | $CH_2$ | 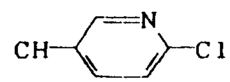 | 5 : 3 | MS : 291/293($M^{\oplus}$), 258, 232, 202 |
| IV-8 | H | H | $CH_3$ | $CH_2$ | 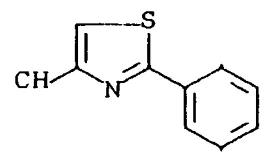 | trans | MS: 339($M^{\oplus}$) |
| IV-9 | H | H | $CH_3$ | $CH_2$ | 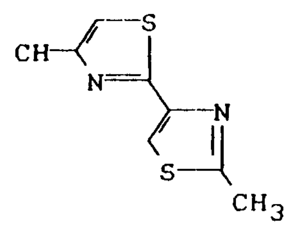 | trans | MS: 360($M^{\oplus}$), 287 (Basis-peak), 218, 142 |
| IV-10 | H | H | $CH_3$ | $CH_2CH_2$ | 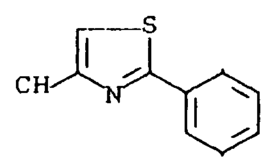 | trans | MS: 353($M^{\oplus}$), 280(Basispeak), 179, 121 |

Beispiel (XI-1)

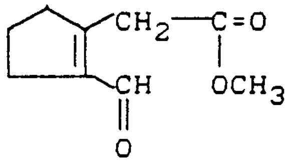

Ein Gemisch aus 29.0 g (0.1 mol) 2-(Benzoyl-methylen)cyclopentanol-1-essigsäuremethylester, 300 $cm^3$ Ether und 150 $cm^3$ 33%iger wäßriger Schwefelsäure wird bis zur vollständigen Umsetzung intensiv verrührt. Man trennt die organische Phase ab und extrahiert mit Ether. Die vereinigten Extrakte werden mit gesättigter Kochsalzlösung, gesättigter Natriumcarbonatlösung (bis zur deutlich alkalischen Reaktion der Waschlösung) und abermals mit Kochsalzlösung nachgewaschen, über wasserfreiem Magnesiumsulfat getrocknet und eingeengt.

Man erhält 14.1 g (83.8% der Theorie) (2-Formylcyclopent-1-en-1-yl)essigsäuremethylester.

Beispiel (XI-2)

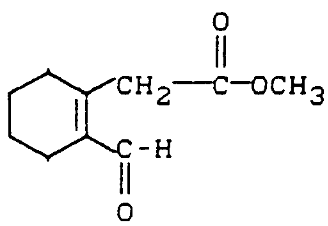

In analoger Weise zu Beispiel (XI-1) erhält man (2-Formyl-cyclohex-1-en-1-yl)essigsäuremethylester in einer Ausbeute von 75% der Theorie.

Herstellung der Ausgangsprodukte

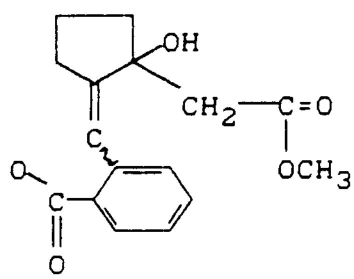

Ein gerührtes Gemisch aus 43.00 g (198.9 mmol) 2-(Benzoyloxy-methylen)-cyclopentanon, 72.28 g (472.5 mmol) Bromessigsäuremethylester, 62.64 g (958.2 mmol) Zinkpulver und 400 $cm^3$ Ether wird unter Ultrabeschallung und Rückfluß solange erwärmt, bis komplette Umsetzung eingetreten ist.

Zur Aufarbeitung läßt man abkühlen, versetzt vorsichtig mit einer Lösung von 25 $cm^3$ Essigsäure in 100 ml Ether, rührt noch 15 Minuten nach und neutralisiert durch Verrühren mit einer Suspension überschüssigen Natriumhydrogencarbonats in wenig Wasser. Die Etherphase wird dekantiert und die wäßrige Suspension der Zinkrückstände mehrfach gründlich mit Essigsäureethylester extrahiert.

Die vereinigten organischen Lösungen werden über wasserfreiem Magnesiumsulfat getrocknet und eingeengt. Der kristalline Rückstand wird in Diisopropylether aufgeschlämmt, abgesaugt und unter vermindertem Druck getrocknet. Aus der Mutterlauge kristallisiert weiteres Produkt aus.

Man erhält 29.9 g (52% der Theorie) 2-(Benzoyloxymethylen)-cyclopentanol-1-essigsäuremethylester vom Schmelzpunkt Fp: 69-70° C.

Auf analoge Weise erhält man 2-(Benzoyloxy-methylen)-cyclohexanol-1-essigsäuremethylester (Ausbeute: 64% der Theorie) vom Schmelzpunkt Fp: 92-94° C.

Auf analoge Weise erhält man 2-(Benzoyloxy-methylen)cycloheptanol-1-essigsäuremethylester (Ausbeute: 59.8% der Theorie).

Allgemeine Arbeitsvorschrift zur Benzoylierung der Hydroxymethylencycloalkanone.

Zu einem Gemisch aus 0.250 mol des Hydroxymethylencycloalkanons, 21.75 g (0.275 mol) Pyridin und 250 $cm^3$ Ether läßt man unter Rühren bei 0-10° C eine Lösung von 35.14 g (0.25 mol) Benzoylchlorid in 100 $cm^3$ Ether tropfen. Nach beendeter Zugabe läßt man noch 3-4 Stunden bei Raumtemperatur nachrühren, erwärmt bis zum Rückfluß und filtriert warm. Der verbleibende Rückstand wird durch mehrfaches Aufschlämmen in warmem Ether und Abfiltrieren extrahiert und die vereinigten organischen Lösungen werden zur Trockene eingeengt. Der verbleibende Rückstand wird in Diisopropylether aufgeschlämmt und abgesaugt.

Auf diese Weise werden 2-(Benzoyl-methylen)-cyclopentanon vom Schmelzpunkt Fp: 75-77° C (Ausbeute 80,6% der Theorie), 2-(Benzoyl-methylen)-cyclohexanon vom Schmelzpunkt Fp: 91-92° C (Ausbeute 80% der Theorie) und 2-(Benzoyl-methylen)-cycloheptanon vom Schmelzpunkt Fp: 102-104° C (Ausbeute: 77.2% der Theorie) erhalten.

Allgemeine Arbeitsvorschrift zur Herstellung von Oxymethylencycloalkanen

Ein Gemisch aus 1.02 mol des Ketons, 76.0 g Ameisensäureethylester und 800 $cm^3$ Ether wird unter Rühren bei Raumtemperatur portionsweise mit 1.02 mol Natriumhydrid (als Suspension) versetzt. Anfänglich muß häufig unter Rückfluß erwärmt werden, nach erfolgtem Anspringen der Reaktion kann gegebenenfalls äußere Kühlung erforderlich werden. Das schließlich erstarrende Reaktionsgemisch wird über Nacht bei Raumtemperatur stehen gelassen.

Zur Aufarbeitung versetzt man mit Eiswasser (ca. 500 ml), trennt die organische Phase ab und wäscht die wäßrige Lösung mit 250 $cm^3$ Ether nach. Die verbliebene wäßrige Phase wird mit 60 g Essigsäure versetzt und mehrfach mit Ether extrahiert. Die vereinigten Extrakte werden über Magnesiumsulfat getrocknet, filtriert, eingeengt und der Rückstand unter vermindertem Druck destilliert.

Auf diese Weise werden Oxymethylencyclopentanon $Kp_{12-14}$ 78-84° C, (Ausbeute: 30% der Theorie), Oxymethylencyclohexanon $Kp_{12-14}$ 80-84° C (Ausbeute: 58% der Theorie) und Oxymethylencycloheptanon $Kp_{12-14}$ 82-88° C (Ausbeute: 43,6% der Theorie) erhalten.

Anwendungsbeispiele

In den folgenden Anwendungsbeispielen wurde die nachstehend aufgeführte Verbindung als Vergleichssubstanz eingesetzt:

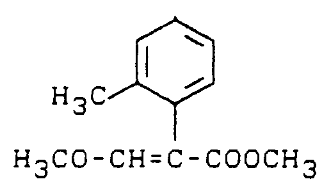

(A)

3-Methoxy-2-(2-methylphenyl)-acrylsäuremethylester
(bekannt aus EP 178 816)

Beispiel A

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß Herstellungsbeispiel 1 und 2.

Beispiel B

Pyricularia-Test (Reis) /protektiv

Lösungsmittel: 12.5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen 1, 2 und 3

Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20° C und einer relativen Luftfeuchtigkeit von ca 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindungen gemäß Herstellungsbeispiel (1).

Beispiel D

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den folgenden Herstellungsbeispielen (1) und (2).

Beispiel E

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den folgenden Herstellungsbeispielen (8) und (9).

Beispiel F

Cochliobolus sativus-Test (Gerste)/protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den folgenden Herstellungsbeispielen (1), (8) und (9).

Beispiel G

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von

Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den folgenden Herstellungsbeispielen (1), (2), (3), (5) und (8).

**Patentansprüche**

**Patentansprüche für folgende Vertragsstaaten : AT, BE, CH, DE, DK, FR, GB, GR, IT, LI, NL**

**1.** Substituierte Acrylsäureester der allgemeinen Formel (I),

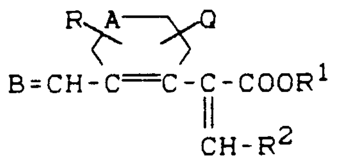

(I)

in welcher

| | |
|---|---|
| $R^1$ | für Alkyl oder für unsubstituiertes oder substituiertes Aralkyl steht, |
| $R^2$ | für Dialkylamino oder für einen Rest $-Z-R^3$ steht, |
| $R^3$ | für Alkyl oder für unsubstituiertes oder substituiertes Aralkyl steht, |
| Z | für Sauerstoff oder Schwefel steht, |
| A | für Sauerstoff, Schwefel oder für eine der folgenden Gruppierungen steht $-(CH_2)-_n$, |

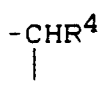, 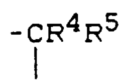 oder 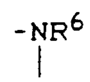,

wobei

| | |
|---|---|
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und |
| $R^6$ | für Alkyl oder für einen Rest |

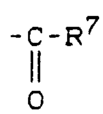

steht,

wobei

| | |
|---|---|
| $R^7$ | für Alkyl, Alkoxy oder Dialkylamino steht, |
| B | für die Gruppe $CH-R^8$ oder $NO-R^9$ steht, wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen. |

2. Acrylsäureester der Formel (I) gemäß Anspruch 1, bei welchen

$R^1$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

$R^2$ für Dialkylamino mit jeweils 1 bis 6 Kohlenstoffatomen in den einzelnen geradkettigen oder verzweigten Alkylteilen oder für einen Rest -Z-$R^3$ steht,

wobei

$R^3$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

Z für Sauerstoff oder Schwefel steht,

A für Sauerstoff, Schwefel oder für eine der folgenden Gruppierungen steht

$-(CH_2)-n$,

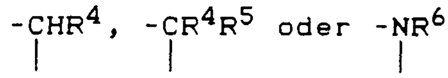

wobei

n für eine Zahl 0 bis 3 steht,

$R^4$ und $R^5$ jeweils unabhängig voneinander für geradkettiges oder verzweigtes Alkyl mit 1 bis 6 Kohlenstoffatomen stehen oder gemeinsam für eine Alkylkette mit 2 bis 7 Kohlenstoffatomen stehen und

$R^6$ für geradkettiges oder verzweigtes Alkyl mit 1 bis 6 Kohlenstoffatomen oder für einen Rest

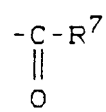

steht,

wobei

$R^7$ für geradkettiges oder verzweigtes Alkyl mit 1 bis 6 Kohlenstoffatomen, geradkettiges oder verzweigtes Alkoxy mit 1 bis 6 Kohlenstoffatomen oder geradkettiges oder verzweigtes Dialkylamino mit jeweils 1 bis 6 Kohlenstoffatomen in den einzelnen Alkylteilen steht,

B für die Gruppe CH-$R^8$ oder NO-$R^9$ steht,

wobei,

$R^8$ für unsubstituiertes oder substituiertes Aryl mit 6 bis 10 Kohlenstoffatomen im Arylteil oder für Heteroaryl mit 2 bis 9 Kohlenstoffatomen und 1 bis 4 gleichen oder verschiedenen Heteroatomen, - insbesondere Stickstoff, Sauerstoff und/oder Schwefel - im Heteroarylteil steht,

wobei als Aryl- und Heteroarylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, -insbesondere Stickstoff, Sauerstoff und/oder Schwefel - im Heteroarylteil und gegebenenfalls 1 bis 4 Kohlenstoffatomen im geradkettigen oder verzweigten Alkylteil,

$R^9$ für jeweils unsubstituiertes oder substituiertes Alkyl mit 1 bis 8 Kohlenstoffatomen, unsubstituiertes oder substituiertes Alkenyl mit 2 bis 8 Kohlenstoffatomen oder unsubstituiertes oder substituiertes Alkinyl mit 2 bis 8 Kohlenstoffatomen steht, wobei als Substituenten jeweils infrage kommen: Cyano, Alkoxy mit 1 bis 4 Kohlenstoffatomen oder Alkoxycarbonyl mit 1 bis 4 Kohlenstoffatomen im Alkoxyteil,
weiterhin
für unsubstituiertes oder einfach bis mehrfach, gleich oder verschieden substituiertes Aryl mit 6 bis 10 Kohlenstoffatomen, unsubstituiertes oder einfach bis mehrfach, gleich oder verschieden substituiertes Aralkyl mit 6 bis 10 Kohlenstoffatomen im Arylteil und 1 bis 2 Kohlenstoffatomen im Alkylteil steht, wobei als Substituenten genannt seien: Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen oder Alkylidendioxy mit 1 bis 2 Kohlenstoffatomen,
ferner
für Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil steht und

R und Q unabhängig voneinander für Wasserstoff, Alkyl mit 1 bis 4 Kohlenstoffatomen, Alkoxy mit 1 bis 4 Kohlenstoffatomen oder Halogenalkyl mit 1 bis 4 Kohlenstoffatomen und 1 bis 9 gleichen oder verschiedenen Halogenatomen stehen.

**3.** Acrylsäureester der Formel (I) gemäß Anspruch 1, bei welchen

$R^1$ für Methyl, Ethyl, n- oder i-Propyl, n-, i-, s-oder t-Butyl oder für gegebenenfalls ein-bis dreifach, gleich oder verschieden substituiertes Benzyl steht, wobei als Substituenten die bei $R^8$ genannten infrage kommen,

$R^2$ für Dialkylamino mit jeweils 1 bis 4 Kohlenstoffatomen in den einzelnen geradkettigen oder verzweigten Alkylteilen steht oder für einen Rest $-Z-R^3$ steht,
wobei

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^8$ genannten infrage kommen und

Z für Sauerstoff oder Schwefel steht;

A für Sauerstoff, Schwefel oder für eine der folgenden Gruppierungen steht
$-(CH_2)_n-$,

$$-\underset{|}{C}HR^4,\quad -\underset{|}{C}R^4R^5 \text{ oder } -\underset{|}{N}R^6,$$

wobei

n für die Zahlen 0, 1, 2 und 3 steht,

$R^4$ und $R^5$ jeweils unabhängig voneinander für geradkettiges oder verzweigtes Alkyl mit 1 bis 4 Kohlenstoffatomen stehen oder gemeinsam für eine Alkylkette mit 2 bis 5 Kohlenstoff-

atomen stehen und

$R^6$ für geradkettiges oder verzweigtes Alkyl mit 1 bis 4 Kohlenstoffatomen oder für einen Rest

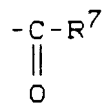

steht,
wobei
$R^7$ für geradkettiges oder verzweigtes Alkyl mit 1 bis 4 Kohlenstoffatomen, geradkettiges oder verzweigtes Alkoxy mit 1 bis 4 Kohlenstoffatomen oder geradkettiges oder verzweigtes Dialkylamino mit jeweils 1 bis 4 Kohlenstoffatomen in den einzelnen Alkylresten steht,

B für die Gruppe CH-$R^8$ oder NO-$R^9$ steht,
wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,
wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Benzyloxy, Phenylthio oder Benzylthio;

$R^9$ für jeweils unsubstituiertes oder substituiertes Alkyl mit 1 bis 6 Kohlenstoffatomen, unsubstituiertes oder substituiertes Alkenyl mit 2 bis 6 Kohlenstoffatomen, unsubstituiertes oder substituiertes Alkinyl mit 2 bis 6 Kohlenstoffatomen, wobei als Substituenten jeweils infrage kommen:
Cyano, Methoxy, Ethoxy, n- und i-Propoxy, n-, i-, s- und t-Butoxy, Methoxycarbonyl, Ethoxycarbonyl, n- und i-Propoxycarbonyl, n-, i-, s- und t-Butoxycarbonyl,
weiterhin
für unsubstituiertes oder einfach bis dreifach, gleich oder verschieden substituiertes Phenyl oder Benzyl steht, wobei als Substituenten genannt seien:
Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Halogenalkyl mit 1 oder 2 Kohlenstoffatomen und 1 bis 5 gleichen oder verschiedenen Fluor- oder Chloratomen, Methylidendioxy und Ethylidendioxy steht und

R und Q unabhängig voneinander für Wasserstoff, Methyl, Ethyl, n- oder i-Propyl, Methoxy, Ethoxy oder Halogenalkyl mit 1 oder 2 Kohlenstoffatomen und 1 bis 5 gleichen oder verschiedenen Fluor- oder Chloratomen steht.

**4.** Acrylsäureester der Formel (I) gemäß Anspruch 1, bei welchen

$R^1$ für Methyl, Ethyl oder Benzyl steht, wobei als Benzyl-Substituenten die bei $R^8$ genannten infrage kommen,

$R^2$ für Dimethylamino, Diethylamino oder für einen Rest -Z-$R^3$ steht,
wobei

$R^3$ für Methyl, Ethyl, n- oder i-Propyl oder Benzyl steht,

Z für Sauerstoff oder Schwefel steht;

A für Sauerstoff, Schwefel oder für eine der folgenden Gruppierungen steht,
$-(CH_2)_n-$, $>CH-CH_3$, $>CH-C_2H_5$,
$>CH-CH(CH_3)_2$, $>CH-C(CH_3)_3$, $>C(CH_3)_2$,

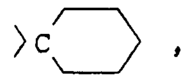

$>N-COOCH_3$, $>N-COOC_2H_5$,

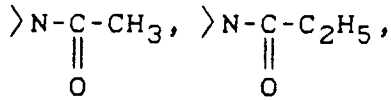

wobei

n für die Zahlen 0, 1, 2 und 3 steht,

B für die Gruppe $CH-R^8$ oder $NO-R^9$ steht,

wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,

wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl, Benzyloxy, Phenylthio oder Benzylthio;

$R^9$ für jeweils unsubstituiertes oder substituiertes Alkyl mit 1-6 Kohlenstoffatomen, unsubstituiertes oder substituiertes Alkenyl mit 2 bis 4 Kohlenstoffatomen und unsubstituiertes oder substituiertes Alkinyl mit 2 bis 4 Kohlenstoffatomen steht,

wobei als Substituenten infrage kommen, Cyano, Methoxycarbonyl, Ethoxycarbonyl, Methoxy oder Alkoxy;

weiterhin für unsubstituiertes oder einfach oder zweifach, gleich oder verschieden substituiertes Phenyl oder Benzyl steht,

wobei als Substituenten infrage kommen:

Brom, Fluor, Chlor, Methoxy, Ethoxy, Methylendioxy, Methyl, Ethyl, Trifluormethyl und

R und Q unabhängig voneinander für Wasserstoff oder Methyl stehen.

**5.** Acrylsäureester der Formel (I) gemäß Anspruch 1, bei welchen

$R^1$ für Methyl oder Ethyl steht,

$R^2$ für Methoxy, Ethoxy, Methylthio oder Dimethylamino steht,

A für Sauerstoff, Schwefel oder für eine der folgenden Gruppierungen steht

$-(CH_2)-_n$,

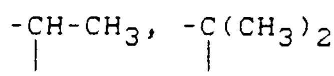

wobei

n für die Zahlen 0, 1 oder 2 steht,

B für die Gruppe $CH-R^8$ oder $NO-R^9$ steht,

wobei

$R^8$ für jeweils unsubstituiertes ein- bis dreifach, gleich oder verschieden substituiertes Phenyl, Naphthyl, Pyridinyl, Pyrimidinyl, Thienyl, Furyl, Thiazolyl, Oxazolyl, Isoxazolyl steht,

wobei als Substituenten infrage kommen: Fluor, Chlor, Brom, Cyano, Nitro, Methyl,

| | |
|---|---|
| | Ethyl, Methoxy, Ethoxy, Methylendioxy, Methylthio, Trifluormethyl, Methoxycarbonyl, Ethoxycarbonyl, Cyclopentyl, Cyclohexyl, Phenoxy, Phenylthio, Benzyloxy, Benzylthio; |
| $R^9$ | für Methyl, Ethyl, n- und i-Propyl, n-, i- und t-Butyl, n- und iso-Amyl, Allyl, Methallyl, Propargyl, Benzyl, o-, m- und p-Chlorbenzyl, o-, m- und p-Methoxy-benzyl, o-, m- und p-Methyl-benzyl oder o-, m- und p-Fluorbenzyl steht und |
| R und Q | für Wasserstoff stehen. |

**6.** Verfahren zur Herstellung von Acrylsäureestern der allgemeinen Formel (I),

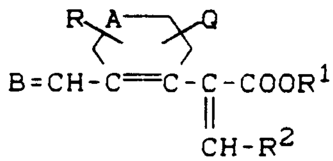

(I)

in welcher

| | |
|---|---|
| $R^1$ | für Alkyl oder für unsubstituiertes oder substituiertes Aralkyl steht, |
| $R^2$ | für Dialkylamino oder für einen Rest $-Z-R^3$ steht, |
| $R^3$ | für Alkyl oder für unsubstituiertes oder substituiertes Aralkyl steht, |
| Z | für Sauerstoff oder Schwefel steht, |
| A | für Sauerstoff, Schwefel oder für eine der folgenden Gruppierungen steht $-(CH_2)-_n$, |

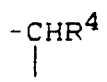, 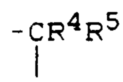 oder 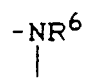,

| | |
|---|---|
| | wobei |
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und |
| $R^6$ | für Alkyl oder für einen Rest |

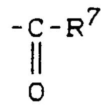

| | |
|---|---|
| | steht, |
| | wobei |
| $R^7$ | für Alkyl, Alkoxy oder Dialkylamino steht, |
| B | für die Gruppe $CH-R^8$ oder $NO-R^9$ steht, |
| | wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen, |

dadurch gekennzeichnet, daß man

a) substituierte Acrylsäureester der Formel (Ia),

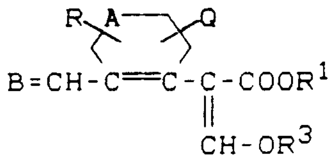

(Ia)

in welcher

$R^1$, $R^3$, A, B, R und Q die oben angegebene Bedeutung haben,

erhält,

wenn man Hydroxyacrylsäureester oder deren Alkalimetallsalze der Formel (II),

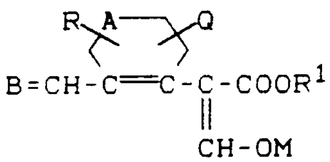

(II)

in welcher

M für Wasserstoff oder für ein Alkalimetallkation steht und

$R^1$, A, B, R und Q die oben angegebene Bedeutung haben,

mit Alkylierungsmitteln der Formel (III)

$R^3$-$E^1$ (III)

in welcher

$E^1$ für eine elektronenanziehende Abgangsgruppe steht und

$R^3$ die oben angegebene Bedeutung hat,

gegebenenfalls in Gegenwart eines Verdünnungsmittels und gegebenenfalls in Gegenwart eines Reaktionshilfsmittels umsetzt;

b) substituierte Acrylsäureester der Formel (Ib),

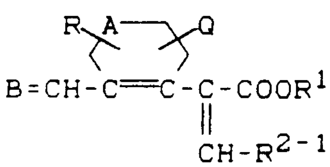

(Ib)

in welcher

$R^{2-1}$ für Dialkylamino steht und

$R^1$, A, B, R und Q die oben angegebene Bedeutung haben,

erhält,

wenn man substituierte Essigsäureester der Formel (IV),

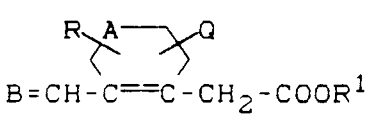

(IV)

in welcher

$R^1$, A, B, R und Q die oben angegebene Bedeutung haben,
mit Formamiden der Formel (Va)

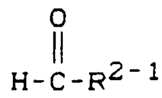 (Va)

in welcher
$R^{2-1}$ die oben angegebene Bedeutung hat,
oder mit Formamid-Derivaten der Formel (Vb)

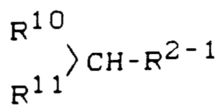 (Vb)

in welcher
$R^{10}$ und $R^{11}$ unabhängig voneinander für Alkoxy oder Dialkylamino stehen und
$R^{2-1}$ die oben angegebene Bedeutung hat, gegebenenfalls in Gegenwart eines Verdünnungsmittels umsetzt;

c) substituierte Acrylsäureester der Formel (Ic),

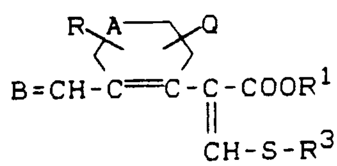 (Ic)

in welcher
$R^1$, $R^3$, A, B, R und Q die oben angegebene Bedeutung haben,
erhält,
wenn man Ketocarbonsäurederivate der Formel (VI)

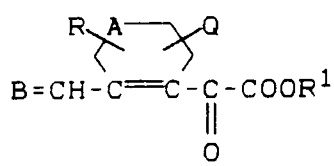 (VI)

in welcher
$R^1$, A, B, R und Q die oben angegebene Bedeutung haben,
mit metallorganischen Verbindungen der Formel (VII),

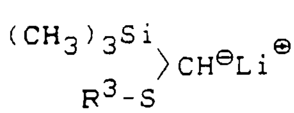 (VII)

in welcher

$R^3$ die oben angegebene Bedeutung hat,

gegebenenfalls in Gegenwart eines Verdünnungsmittels umsetzt;

d) substituierte Acrylsäureester der Formel (Ic) erhält,

wenn man substituierte Acrylsäureester der Formel (VIII),

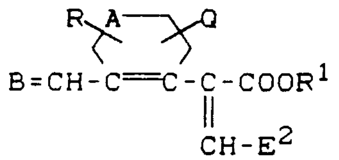

(VIII)

in welcher

$R^1$, A, B, R und Q die oben angegebene Bedeutung haben und

$E^2$ für eine elektronenanziehende Abgangsgruppe steht,

mit Thiolen der Formel (IX),

$R^3$-SH (IX)

in welcher

$R^3$ die oben angegebene Bedeutung hat,

gegebenenfalls in Gegenwart eines Verdünnungsmittels und gegebenenfalls in Gegenwart eines Reaktionshilfsmittels umsetzt.

**7.** Schädlingsbekämpfungsmittel, gekennzeichnet durch einen Gehalt an mindestens einem Acrylsäureester der Formel (I) nach den Ansprüchen 1 bis 6.

**8.** Verwendung von Acrylsäureestern der Formel (I) nach den Ansprüchen 1 bis 6 zur Bekämpfung von Schädlingen.

**9.** Verfahren zur Bekämpfung von Schädlingen, dadurch gekennzeichnet, daß man Acrylsäureester der Formel (I) nach den Ansprüchen 1 bis 6 auf Schädlinge und/oder ihren Lebensraum einwirken läßt.

**10.** Verfahren zur Herstellung von Schädlingsbekämpfungsmitteln, dadurch gekennzeichnet, daß man Acrylsäureester der Formel (I) nach den Ansprüchen 1 bis 6 mit Streckmitteln und/oder oberflächenaktiven Mitteln vermischt.

**11.** Hydroxyacrylsäureester der allgemeinen Formel (II)

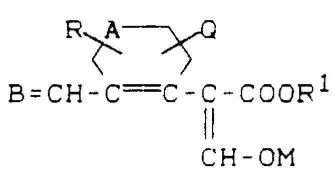

(II)

in welcher

$R^1$ für Alkyl oder für unsubstituiertes oder substituiertes Aralkyl steht,

A für Sauerstoff, Schwefel oder für eine der folgenden Gruppierungen steht

$-(CH_2)-_n$,

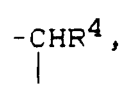

$-CR^4R^5$ oder

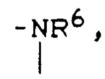

wobei

n für eine Zahl 0 bis 6 steht,

$R^4$ und $R^5$ jeweils unabhängig voneinander für Alkyl stehen oder gemeinsam für eine Alkylkette mit 2 bis 7 Kohlenstoffatomen stehen und

$R^6$ für Alkyl oder für einen Rest

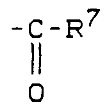

steht,

wobei

$R^7$ für Alkyl, Alkoxy oder Dialkylamino steht,

B für die Gruppe $CH-R^8$ oder $NO-R^9$ steht,

wobei

$R^8$ für unsubstituiertes oder substituiertes Aryl oder Hetaryl steht und

$R^9$ für unsubstituiertes oder substituiertes Alkyl, Alkenyl, Alkinyl, Aralkyl, Aryl oder Hetaryl steht und

R und Q unabhängig voneinander für Wasserstoff, Alkyl, Halogenalkyl oder Alkoxy stehen und

M für Wasserstoff oder für ein Alkalimetallkation steht.

**12.** Verfahren zur Herstellung von Hydroxyacrylsäureestern der Formel (II) gemäß Anspruch 11, dadurch gekennzeichnet, daß man substituierte Essigsäureester der Formel (IV)

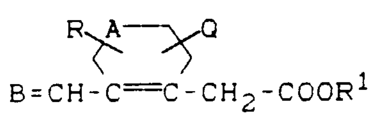

(IV)

in welcher

$R^1$, A, B, R und Q die oben angegebene Bedeutung haben,

mit Ameisensäureestern der Formel (X),

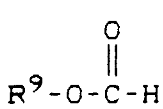

(X)

in welcher

$R^9$ für Alkyl, insbesondere für Methyl oder Ethyl, steht,

gegebenenfalls in Gegenwart eines Verdünnungsmittels und gegebenenfalls in Gegenwart eines basischen Reaktionshilfsmittels bei Temperaturen von -20° C bis +50° C umsetzt.

**13.** Essigsäureester der allgemeinen Formel (IV)

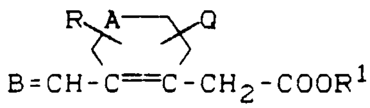 (IV)

in welcher

R[1] für Alkyl oder für unsubstituiertes oder substituiertes Aralkyl steht,

A für Sauerstoff, Schwefel oder für eine der folgenden Gruppierungen steht $-(CH_2)-_n$,

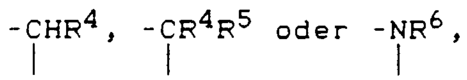

wobei

n für eine Zahl 0 bis 6 steht,

$R^4$ und $R^5$ jeweils unabhängig voneinander für Alkyl stehen oder gemeinsam für eine Alkylkette mit 2 bis 7 Kohlenstoffatomen stehen und

$R^6$ für Alkyl oder für einen Rest

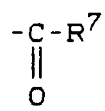

steht,

wobei

$R^7$ für Alkyl, Alkoxy oder Dialkylamino steht,

B für die Gruppe $CH\text{-}R^8$ oder $NO\text{-}R^9$ steht,

wobei

$R^8$ für unsubstituiertes oder substituiertes Aryl oder Hetaryl steht und

$R^9$ für unsubstituiertes oder substituiertes Alkyl, Alkenyl, Alkinyl, Aralkyl, Aryl oder Hetaryl steht und

R und Q unabhängig voneinander für Wasserstoff, Alkyl, Halogenalkyl oder Alkoxy stehen.

**14.** Verfahren zur Herstellung von Essigsäureestern der Formel (IV) gemäß Anspruch 13, dadurch gekennzeichnet, daß

a) für den Fall, daß $R^1$, A, R und Q die oben angegebene Bedeutung haben, und

$B^1$ für die Gruppe $N\text{-}O\text{-}R^9$ steht,

wobei

$R^9$ die oben angegebene Bedeutung hat,

man Aldehyde der allgemeinen Formel (XI),

 (XI)

in welcher

$R^1$, A, R und Q die oben angegebene Bedeutung haben,

mit Verbindungen der allgemeinen Formel (XII)

$R^9$-O-$NH_2$ (XII)

in welcher

$R^9$ die oben angegebene Bedeutung hat,

gegebenenfalls in Gegenwart eines Verdünnungsmittels bei Temperaturen von -20° C bis +50° C umsetzt,

oder

b) für den Fall, daß $R^1$, A, R und Q die oben angegebene Bedeutung haben, und

$B^2$ für die Gruppe CH-$R^8$ steht,

wobei

$R^8$ die oben angegebene Bedeutung hat,

indem man Aldehyde der allgemeinen Formel (XI)

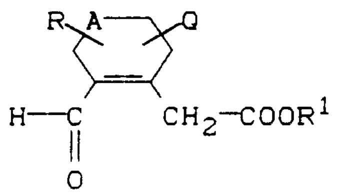

(XI)

in der

$R^1$, A, R und Q die oben angegebene Bedeutung haben,

α) mit Phosphoniumverbindungen der allgemeinen Formel (XIII)

$R^8$-$CH_2$-$P(R^{12})_3{}^{\oplus}X^{\ominus}$ (XIII)

in welcher

$R^8$ die oben angegebene Bedeutung hat,

$R^{12}$ für gegebenenfalls durch Methyl, Ethyl, Methoxy, Ethoxy substituiertes Aryl, bevorzugt Phenyl und

X für Halogen, bevorzugt Brom oder

Chlor steht, oder

β) mit Reagenzien der allgemeinen Formel (XIV)

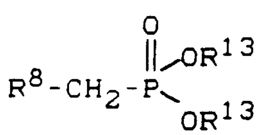

(XIV)

in welcher

$R^8$ die oben angegebene Bedeutung hat und

$R^{13}$ für $C_{1-4}$Alkyl, besonders bevorzugt Methyl, Ethyl oder n-Butyl steht,

unter Verwendung eines unter den Reaktionsbedingungen inerten Lösungsmittels, und gegebenenfalls eines basischen Hilfsmittels bei Temperaturen von -80° C bis +60° C, im Sinne einer WITTIG-Reaktion bzw. einer HORNER-WITTIG-Reaktion umsetzt.

**15.** Acrylsäureester der allgemeinen Formel (VIII)

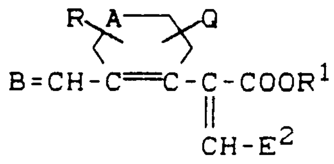 (VIII)

in welcher

| | |
|---|---|
| $R^1$ | für Alkyl oder für unsubstituiertes oder substituiertes Aralkyl steht, |
| A | für Sauerstoff, Schwefel oder für eine der folgenden Gruppierungen steht |

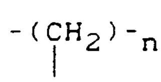 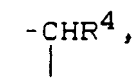

$-CR^4R^5$ oder

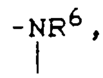

wobei

| | |
|---|---|
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und |
| $R^6$ | für Alkyl oder für einen Rest |

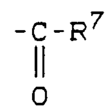

steht,

wobei

| | |
|---|---|
| $R^7$ | für Alkyl, Alkoxy oder Dialkylamino steht, |
| B | für die Gruppe $CH-R^8$ oder $NO-R^9$ steht, wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen und |
| $E^2$ | für eine elektronenanziehende Abgangsgruppe steht. |

**16.** Verfahren zur Herstellung von Acrylsäureester der Formel (VIII) gemäß Anspruch 15, dadurch gekennzeichnet, daß man Hydroxyacrylsäureester der Formel (II),

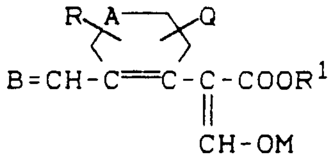

(II)

in welcher

$R^1$, A, B, R und Q die oben angegebene Bedeutung haben und M für Wasserstoff oder ein Alkalimetallkation steht,

mit Säurechloriden der Formel (XIV),

$R^{14}$-Cl (XIV)

in welcher

$R^{14}$ für einen Acyl- oder Sulfonylrest, insbesondere für einen Acetyl-, einen Methansulfonyl-oder einen p-Toluolsulfonylrest steht,

gegebenenfalls in Gegenwart eines Verdünnungsmittels und gegebenenfalls in Gegenwart eines Säurebindemittels bei Temperaturen von -20° C bis +120° C umsetzt.

**Patentansprüche für folgenden Vertragsstaat : ES**

**1.** Verfahren zur Herstellung von Acrylsäureestern der allgemeinen Formel (I),

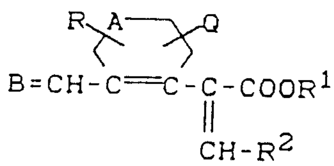

(I)

in welcher

$R^1$ für Alkyl oder für unsubstituiertes oder substituiertes Aralkyl steht,

$R^2$ für Dialkylamino oder für einen Rest -Z-$R^3$ steht,

$R^3$ für Alkyl oder für unsubstituiertes oder substituiertes Aralkyl steht,

Z für Sauerstoff oder Schwefel steht,

A für Sauerstoff, Schwefel oder für eine der folgenden Gruppierungen steht $-(CH_2)-_n$,

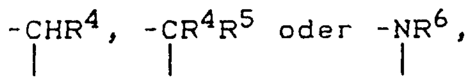

wobei

n für eine Zahl 0 bis 6 steht,

$R^4$ und $R^5$ jeweils unabhängig voneinander für Alkyl stehen oder gemeinsam für eine Alkylkette mit 2 bis 7 Kohlenstoffatomen stehen und $R^6$ für Alkyl oder für einen Rest

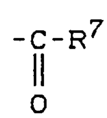

steht,

wobei

$R^7$ für Alkyl, Alkoxy oder Dialkylamino steht,

B für die Gruppe CH-$R^8$ oder NO-$R^9$ steht,

wobei

$R^8$ für unsubstituiertes oder substituiertes Aryl oder Hetaryl steht und

$R^9$ für unsubstituiertes oder substituiertes Alkyl, Alkenyl, Alkinyl, Aralkyl, Aryl oder Hetaryl steht und

R und Q unabhängig voneinander für Wasserstoff, Alkyl, Halogenalkyl oder Alkoxy stehen,

dadurch gekennzeichnet, daß man

a) substituierte Acrylsäureester der Formel (Ia),

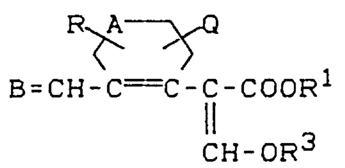

(Ia)

in welcher

$R^1$, $R^3$, A, B, R und Q die oben angegebene Bedeutung haben,

erhält,

wenn man Hydroxyacrylsäureester oder deren Alkalimetallsalze der Formel (II),

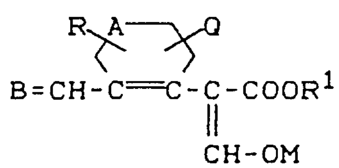

(II)

in welcher

M für Wasserstoff oder für ein Alkalimetallkation steht und

$R^1$, A, B, R und Q die oben angegebene Bedeutung haben,

mit Alkylierungsmitteln der Formel (III)

$R^3$-$E^1$ (III)

in welcher

$E^1$ für eine elektronenanziehende Abgangsgruppe steht und

$R^3$ die oben angegebene Bedeutung hat,

gegebenenfalls in Gegenwart eines Verdünnungsmittels und gegebenenfalls in Gegenwart eines Reaktionshilfsmittels umsetzt;

b) substituierte Acrylsäureester der Formel (Ib),

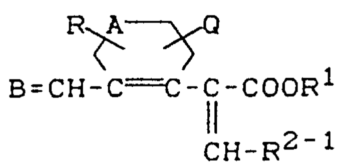

(Ib)

in welcher

$R^{2-1}$ für Dialkylamino steht und

$R^1$, A, B, R und Q die oben angegebene Bedeutung haben,

erhält,

wenn man substituierte Essigsäureester der Formel (IV),

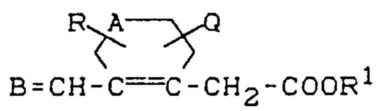

(IV)

in welcher

$R^1$, A, B, R und Q die oben angegebene Bedeutung haben,

mit Formamiden der Formel (Va)

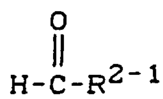

(Va)

in welcher

$R^{2-1}$ die oben angegebene Bedeutung hat,

oder mit Formamid-Derivaten der Formel (Vb)

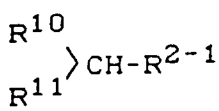

(Vb)

in welcher

$R^{10}$ und $R^{11}$ unabhängig voneinander für Alkoxy oder Dialkylamino stehen und

$R^{2-1}$ die oben angegebene Bedeutung hat,

gegebenenfalls in Gegenwart eines Verdünnungsmittels umsetzt;

c) substituierte Acrylsäureester der Formel (Ic),

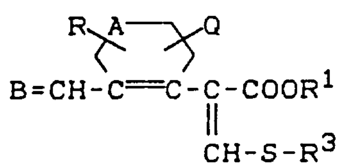

(Ic)

in welcher

$R^1$, $R^3$, A, B, R und Q die oben angegebene Bedeutung haben,

erhält,

wenn man Ketocarbonsäurederivate der Formel (VI)

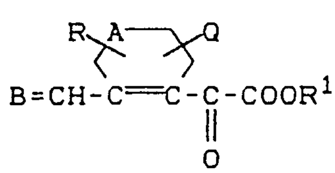

(VI)

in welcher

$R^1$, A, B, R und Q die oben angegebene Bedeutung haben,

mit metallorganischen Verbindungen der Formel (VII),

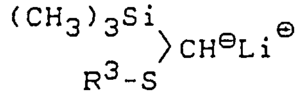 (VII)

in welcher

$R^3$ die oben angegebene Bedeutung hat,

gegebenenfalls in Gegenwart eines Verdünnungsmittels umsetzt;

d) substituierte Acrylsäureester der Formel (Ic) erhält,

wenn man substituierte Acrylsäureester der Formel (VIII),

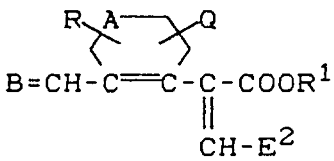 (VIII)

in welcher

$R^1$, A, B, R und Q die oben angegebene Bedeutung haben und

$E^2$ für eine elektronenanziehende Abgangsgruppe steht,

mit Thiolen der Formel (IX),

$R^3$-SH (IX)

in welcher

$R^3$ die oben angegebene Bedeutung hat,

gegebenenfalls in Gegenwart eines Verdünnungsmittels und gegebenenfalls in Gegenwart eines Reaktionshilfsmittels umsetzt.

**2.** Verfahren gemäß Anspruch 1, bei welchen

$R^1$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

$R^2$ für Dialkylamino mit jeweils 1 bis 6 Kohlenstoffatomen in den einzelnen geradkettigen oder verzweigten Alkylteilen oder für einen Rest -Z-$R^3$ steht,

wobei

$R^3$ für geradkettiges oder verzweigtes Alkyl mit 1 bis 6 Kohlenstoffatomen oder für unsubstituiertes oder im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^8$ genannten infrage kommen,

Z für Sauerstoff oder Schwefel steht,

A für Sauerstoff, Schwefel oder für eine der folgenden Gruppierungen steht

$-(CH_2)_n-$,

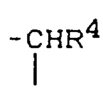, 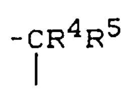 oder 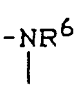

wobei

n für eine Zahl 0 bis 3 steht,

$R^4$ und $R^5$ jeweils unabhängig voneinander für geradkettiges oder verzweigtes Alkyl mit 1 bis 6 Kohlenstoffatomen stehen oder gemeinsam für eine Alkylkette mit 2 bis 7 Kohlenstoffatomen stehen und

$R^6$ für geradkettiges oder verzweigtes Alkyl mit 1 bis 6 Kohlenstoffatomen oder für einen Rest

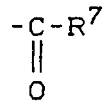

steht,

wobei

$R^7$ für geradkettiges oder verzweigtes Alkyl mit 1 bis 6 Kohlenstoffatomen, geradkettiges oder verzweigtes Alkoxy mit 1 bis 6 Kohlenstoffatomen oder geradkettiges oder verzweigtes Dialkylamino mit jeweils 1 bis 6 Kohlenstoffatomen in den einzelnen Alkylteilen steht,

B für die Gruppe CH-$R^8$ oder NO-$R^9$ steht,

wobei,

$R^8$ für unsubstituiertes oder substituiertes Aryl mit 6 bis 10 Kohlenstoffatomen im Arylteil oder für Heteroaryl mit 2 bis 9 Kohlenstoffatomen und 1 bis 4 gleichen oder verschiedenen Heteroatomen, - insbesondere Stickstoff, Sauerstoff und/oder Schwefel - im Heteroarylteil steht,

wobei als Aryl- und Heteroarylsubstituenten jeweils infrage kommen;

Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, -insbesondere Stickstoff, Sauerstoff und/oder Schwefel - im Heteroarylteil und gegebenenfalls 1 bis 4 Kohlenstoffatomen im geradkettigen oder verzweigten Alkylteil,

$R^9$ für jeweils unsubstituiertes oder substituiertes Alkyl mit 1 bis 8 Kohlenstoffatomen, unsubstituiertes oder substituiertes Alkenyl mit 2 bis 8 Kohlenstoffatomen oder unsubstituiertes oder substituiertes Alkinyl mit 2 bis 8 Kohlenstoffatomen steht, wobei als Substituenten jeweils infrage kommen; Cyano, Alkoxy mit 1 bis 4 Kohlenstoffatomen oder Alkoxycarbonyl mit 1 bis 4 Kohlenstoffatomen im Alkoxyteil,

weiterhin

für unsubstituiertes oder einfach bis mehrfach, gleich oder verschieden substituiertes Aryl mit 6 bis 10 Kohlenstoffatomen, unsubstituiertes oder einfach bis mehrfach, gleich oder verschieden substituiertes Aralkyl mit 6 bis 10 Kohlenstoffatomen im Arylteil und 1 bis 2 Kohlenstoffatomen im Alkylteil steht, wobei als Substituenten genannt seien; Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen

oder verschiedenen Halogenatomen oder Alkylidendioxy mit 1 bis 2 Kohlenstoffatomen,
ferner
für Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil steht und

R und Q unabhängig voneinander für Wasserstoff, Alkyl mit 1 bis 4 Kohlenstoffatomen, Alkoxy mit 1 bis 4 Kohlenstoffatomen oder Halogenalkyl mit 1 bis 4 Kohlenstoffatomen und 1 bis 9 gleichen oder verschiedenen Halogenatomen stehen.

**3.** Verfahren gemäß Anspruch 1, bei welchen

$R^1$ für Methyl, Ethyl, n- oder i-Propyl, n-, i-, s-oder t-Butyl oder für gegebenenfalls einbis dreifach, gleich oder verschieden substituiertes Benzyl steht, wobei als Substituenten die bei $R^8$ genannten infrage kommen,

$R^2$ für Dialkylamino mit jeweils 1 bis 4 Kohlenstoffatomen in den einzelnen geradkettigen oder verzweigten Alkylteilen steht oder für einen Rest $-Z-R^3$ steht,
wobei

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^8$ genannten infrage kommen und

Z für Sauerstoff oder Schwefel steht;

A für Sauerstoff, Schwefel oder für eine der folgenden Gruppierungen steht
$-(CH_2)-_n$,

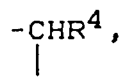, 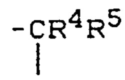 oder 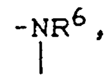,

wobei

n für die Zahlen 0, 1, 2 und 3 steht,

$R^4$ und $R^5$ jeweils unabhängig voneinander für geradkettiges oder verzweigtes Alkyl mit 1 bis 4 Kohlenstoffatomen stehen oder gemeinsam für eine Alkylkette mit 2 bis 5 Kohlenstoffatomen stehen und

$R^6$ für geradkettiges oder verzweigtes Alkyl mit 1 bis 4 Kohlenstoffatomen oder für einen Rest

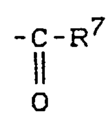

steht,
wobei
$R^7$ für geradkettiges oder verzweigtes Alkyl mit 1 bis 4 Kohlenstoffatomen, geradkettiges oder verzweigtes Alkoxy mit 1 bis 4 Kohlenstoffatomen oder geradkettiges oder verzweigtes Dialkylamino mit jeweils 1 bis 4 Kohlenstoffatomen in den einzelnen Alkylresten steht,

B für die Gruppe $CH-R^8$ oder $NO-R^9$ steht,
wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,
wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Benzyloxy, Phenylthio oder Benzylthio;

$R^9$ für jeweils unsubstituiertes oder substituiertes Alkyl mit 1 bis 6 Kohlenstoffatomen, unsubstituiertes oder substituiertes Alkenyl mit 2 bis 6 Kohlenstoffatomen, unsubstituiertes oder substituiertes Alkinyl mit 2 bis 6 Kohlenstoffatomen, wobei als Substituenten jeweils infrage kommen:
Cyano, Methoxy, Ethoxy, n- und i-Propoxy, n-, i-, s- und t-Butoxy, Methoxycarbonyl, Ethoxycarbonyl, n- und i-Propoxycarbonyl, n-, i-, s- und t-Butoxycarbonyl,
weiterhin
für unsubstituiertes oder einfach bis dreifach, gleich oder verschieden substituiertes Phenyl oder Benzyl steht, wobei als Substituenten genannt seien:
Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Halogenalkyl mit 1 oder 2 Kohlenstoffatomen und 1 bis 5 gleichen oder verschiedenen Fluor- oder Chloratomen, Methylidendioxy und Ethylidendioxy steht und

R und Q unabhängig voneinander für Wasserstoff, Methyl, Ethyl, n- oder i-Propyl, Methoxy, Ethoxy oder Halogenalkyl mit 1 oder 2 Kohlenstoffatomen und 1 bis 5 gleichen oder verschiedenen Fluor- oder Chloratomen steht.

**4.** Verfahren gemäß Anspruch 1, bei welchen

$R^1$ für Methyl, Ethyl oder Benzyl steht, wobei als Benzyl-Substituenten die bei $R^8$ genannten infrage kommen,

$R^2$ für Dimethylamino, Diethylamino oder für einen Rest $-Z-R^3$ steht,
wobei

$R^3$ für Methyl, Ethyl, n- oder i-Propyl oder Benzyl steht,

Z für Sauerstoff oder Schwefel steht;

A für Sauerstoff, Schwefel oder für eine der folgenden Gruppierungen steht,
$-(CH_2)$-n, $>CH-CH_3$, $>CH-C_2H_5$,
$>CH-CH(CH_3)_2$, $>CH-C(CH_3)_3$, $>C(CH_3)_2$,

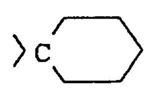 ,

$>N-COOCH_3$, $>N-COOC_2H_5$,

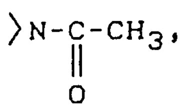 , 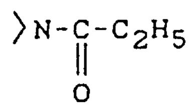 ,

wobei

n für die Zahlen 0, 1, 2 und 3 steht,

B für die Gruppe $CH-R^8$ oder $NO-R^9$ steht,
wobei

$R^8$ für jeweils unsubstituiertes oder jeweils ein-bis dreifach, gleich oder verschieden substituiertes Phenyl, Naphthyl, Pyridinyl, Pyrimidinyl, Pyrazinyl, Pyridazinyl, Thienyl, Furyl, Pyrrolyl, Thiazolyl, Isothiazolyl, Oxazolyl, Isoxazolyl steht,
wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl, Benzyloxy, Phenylthio oder Benzylthio;

$R^9$ für jeweils unsubstituiertes oder substituiertes Alkyl mit 1-6 Kohlenstoffatomen, unsubstituiertes oder substituiertes Alkenyl mit 2 bis 4 Kohlenstoffatomen und unsubstituiertes oder substituiertes Alkinyl mit 2 bis 4 Kohlenstoffatomen steht,
wobei als Substituenten infrage kommen, Cyano, Methoxycarbonyl, Ethoxycarbonyl, Methoxy oder Alkoxy;
weiterhin für unsubstituiertes oder einfach oder zweifach, gleich oder verschieden substituiertes Phenyl oder Benzyl steht,
wobei als Substituenten infrage kommen; Brom, Fluor, Chlor, Methoxy, Ethoxy, Methylendioxy, Methyl, Ethyl, Trifluormethyl und

R und Q unabhängig voneinander für Wasserstoff oder Methyl stehen.

**5.** Verfahren gemäß Anspruch 1, bei welchen

$R^1$ für Methyl oder Ethyl steht,

$R^2$ für Methoxy, Ethoxy, Methylthio oder Dimethylamino steht,

A für Sauerstoff, Schwefel oder für eine der folgenden Gruppierungen steht
$-(CH_2)-_n$,

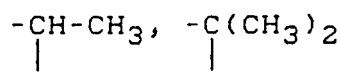

wobei

n für die Zahlen 0, 1 oder 2 steht,

B für die Gruppe $CH-R^8$ oder $NO-R^9$ steht,
wobei

$R^8$ für jeweils unsubstituiertes ein- bis dreifach, gleich oder verschieden substituiertes Phenyl, Naphthyl, Pyridinyl, Pyrimidinyl, Thienyl, Furyl, Thiazolyl, Oxazolyl, Isoxazolyl steht,
wobei als Substituenten infrage kommen: Fluor, Chlor, Brom, Cyano, Nitro, Methyl, Ethyl, Methoxy, Ethoxy, Methylendioxy, Methylthio, Trifluormethyl, Methoxycarbonyl, Ethoxycarbonyl, Cyclopentyl, Cyclohexyl, Phenoxy, Phenylthio, Benzyloxy, Benzylthio;

$R^9$ für Methyl, Ethyl, n- und i-Propyl, n-, i- und t-Butyl, n- und iso-Amyl, Allyl, Methallyl, Propargyl, Benzyl, o-, m- und p-Chlorbenzyl, o-, m- und p-Methoxy-benzyl, o-, m- und p-Methyl-benzyl oder o-, m- und p-Fluorbenzyl steht und

R und Q für Wasserstoff stehen.

**6.** Schädlingsbekämpfungsmittel, gekennzeichnet durch einen Gehalt an mindestens einem Acrylsäureester der Formel (I) nach den Ansprüchen 1 bis 5.

**7.** Verwendung von Acrylsäureestern der Formel (I) nach den Ansprüchen 1 bis 5 zur Bekämpfung von Schädlingen.

**8.** Verfahren zur Bekämpfung von Schädlingen, dadurch gekennzeichnet, daß man Acrylsäureester der Formel (I) nach den Ansprüchen 1 bis 5 auf Schädlinge und/oder ihren Lebensraum einwirken läßt.

**9.** Verfahren zur Herstellung von Schädlingsbekämpfungsmitteln, dadurch gekennzeichnet, daß man Acrylsäureester der Formel (I) nach den Ansprüchen 1 bis 5 mit Streckmitteln und/oder oberflächenaktiven Mitteln vermischt.

**10.** Verfahren zur Herstellung von Hydroxyacrylsäureestern der allgemeinen Formel (II)

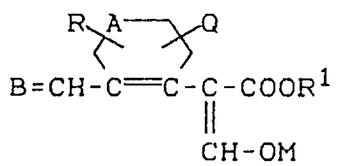

(II)

in welcher

| | |
|---|---|
| $R^1$ | für Alkyl oder für unsubstituiertes oder substituiertes Aralkyl steht, |
| A | für Sauerstoff, Schwefel oder für eine der folgenden Gruppierungen steht $-(CH_2)-_n$, 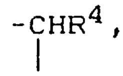 $-CR^4R^5$ oder 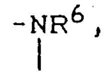 wobei |
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und $R^6$ für Alkyl oder für einen Rest 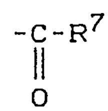 steht, wobei |
| $R^7$ | für Alkyl, Alkoxy oder Dialkylamino steht, |
| B | für die Gruppe $CH-R^8$ oder $NO-R^9$ steht, wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen und |
| M | für Wasserstoff oder für ein Alkalimetallkation steht, |

dadurch gekennzeichnet, daß man substituierte Essigsäureester der Formel (IV)

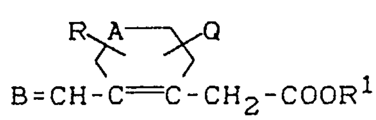

(IV)

in welcher

$R^1$, A, B, R und Q die oben angegebene Bedeutung haben,

mit Ameisensäureestern der Formel (X),

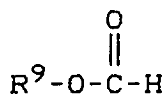 (X)

in welcher

$R^9$ für Alkyl, insbesondere für Methyl oder Ethyl, steht,

gegebenenfalls in Gegenwart eines Verdünnungsmittels und gegebenenfalls in Gegenwart eines basischen Reaktionshilfsmittels bei Temperaturen von -20 °C bis +50 °C umsetzt.

**11.** Verfahren zur Herstellung von Essigsäureestern der allgemeinen Formel (IV)

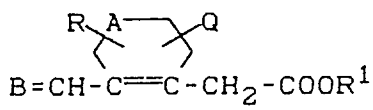 (IV)

in welcher

$R^1$ für Alkyl oder für unsubstituiertes oder substituiertes Aralkyl steht,

A für Sauerstoff, Schwefel oder für eine der folgenden Gruppierungen steht

$-(CH_2)-_n$,

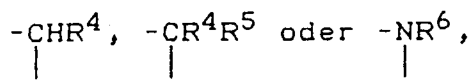

wobei

n für eine Zahl 0 bis 6 steht,

$R^4$ und $R^5$ jeweils unabhängig voneinander für Alkyl stehen oder gemeinsam für eine Alkylkette mit 2 bis 7 Kohlenstoffatomen stehen und

$R^6$ für Alkyl oder für einen Rest

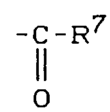

steht,

wobei

$R^7$ für Alkyl, Alkoxy oder Dialkylamino steht,

B für die Gruppe CH-$R^8$ oder NO-$R^9$ steht,

wobei

$R^8$ für unsubstituiertes oder substituiertes Aryl oder Hetaryl steht und

$R^9$ für unsubstituiertes oder substituiertes Alkyl, Alkenyl, Alkinyl, Aralkyl, Aryl oder Hetaryl steht und

R und Q unabhängig voneinander für Wasserstoff, Alkyl, Halogenalkyl oder Alkoxy stehen,

dadurch gekennzeichnet, daß

a) für den Fall, daß $R^1$, A, R und Q die oben angegebene Bedeutung haben, und

$B^1$ für die Gruppe N-O-$R^9$ steht,

wobei

$R^9$ die oben angegebene Bedeutung hat,

man Aldehyde der allgemeinen Formel (XI),

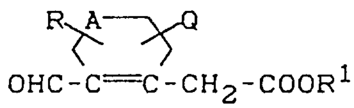 (XI)

in welcher

$R^1$, A, R und Q die oben angegebene Bedeutung haben,

mit Verbindungen der allgemeinen Formel (XII)

$R^9$-O-$NH_2$ (XII)

in welcher

$R^9$ die oben angegebene Bedeutung hat,

gegebenenfalls in Gegenwart eines Verdünnungsmittels bei Temperaturen von -20 °C bis +50 °C umsetzt,

oder

b) für den Fall, daß $R^1$, A, R und Q die oben angegebene Bedeutung haben, und

$B^2$ für die Gruppe CH-$R^8$ steht,

wobei

$R^8$ die oben angegebene Bedeutung hat, indem man Aldehyde der allgemeinen Formel (XI)

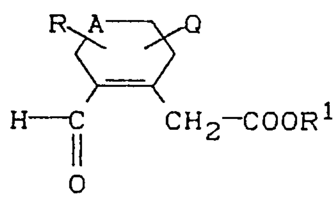 (XI)

in der

$R^1$, A, R und Q die oben angegebene Bedeutung haben,

α) mit Phosphoniumverbindungen der allgemeinen Formel (XIII)

$R^8$-$CH_2$-$P(R^{12})_3{}^{\oplus}X^{\ominus}$ (XIII)

in welcher

$R^8$ die oben angegebene Bedeutung hat,

$R^{12}$ für gegebenenfalls durch Methyl, Ethyl, Methoxy, Ethoxy substituiertes Aryl, bevorzugt Phenyl und

X für Halogen, bevorzugt Brom oder Chlor steht, oder

β) mit Reagenzien der allgemeinen Formel (XIV)

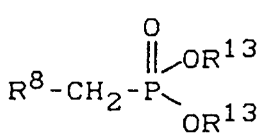 (XIV)

in welcher

$R^8$ die oben angegebene Bedeutung hat und

$R^{13}$ für $C_{1-4}$Alkyl, besonders bevorzugt Methyl, Ethyl oder n-Butyl steht,

unter Verwendung eines unter den Reaktionsbedingungen inerten Lösungsmittels, und gegebenenfalls eines basischen Hilfsmittels bei Temperaturen von -80 °C bis +60 °C, im Sinne einer WITTIG-Reaktion bzw. einer HORNER-WITTIG-Reaktion umsetzt.

**12.** Verfahren zur Herstellung von Acrylsäureestern der allgemeinen Formel (VIII)

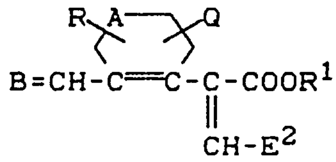 (VIII)

in welcher

| | |
|---|---|
| $R^1$ | für Alkyl oder für unsubstituiertes oder substituiertes Aralkyl steht, |
| A | für Sauerstoff, Schwefel oder für eine der folgenden Gruppierungen steht |

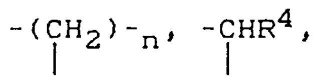

-$CR^4R^5$ oder

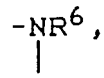

wobei

| | |
|---|---|
| n | für eine Zahl 0 bis 6 steht, |
| $R^4$ und $R^5$ | jeweils unabhängig voneinander für Alkyl stehen oder gemeinsam für eine Alkylkette mit 2 bis 7 Kohlenstoffatomen stehen und |
| $R^6$ | für Alkyl oder für einen Rest |

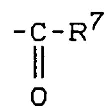

steht,
wobei

| | |
|---|---|
| $R^7$ | für Alkyl, Alkoxy oder Dialkylamino steht, |
| B | für die Gruppe CH-$R^8$ oder NO-$R^9$ steht, wobei |
| $R^8$ | für unsubstituiertes oder substituiertes Aryl oder Hetaryl steht und |
| $R^9$ | für unsubstituiertes oder substituiertes Alkyl, Alkenyl, Alkinyl, Aralkyl, Aryl oder Hetaryl steht und |
| R und Q | unabhängig voneinander für Wasserstoff, Alkyl, Halogenalkyl oder Alkoxy stehen und |
| $E^2$ | für eine elektronenanziehende Abgangsgruppe steht, |

dadurch gekennzeichnet, daß man Hydroxyacrylsäureester der Formel (II),

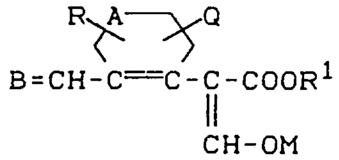

(II)

in welcher

$R^1$, A, B, R und Q die oben angegebene Bedeutung haben und M für Wasserstoff oder ein Alkalimetallkation steht,

mit Säurechloriden der Formel (XIV),

$R^{14}$-Cl (XIV)

in welcher

$R^{14}$ für einen Acyl- oder Sulfonylrest, insbesondere für einen Acetyl-, einen Methansulfonyl- oder einen p-Toluolsulfonylrest steht,

gegebenenfalls in Gegenwart eines Verdünnungsmittels und gegebenenfalls in Gegenwart eines Säurebindemittels bei Temperaturen von -20 °C bis +120 °C umsetzt.

**Claims**

**Claims for the following Contracting States : AT, BE, CH, DE, DK, FR, GB, GR, IT, LI, NL**

**1.** Substituted acrylic esters of the general formula (I)

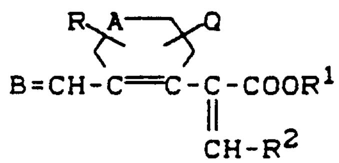

(I)

in which

| | |
|---|---|
| $R^1$ | represents alkyl, or represents unsubstituted or substituted aralkyl, |
| $R^2$ | represents dialkylamino, or represents a radical $-Z-R^3$, |
| $R^3$ | represents alkyl, or represents unsubstituted or substituted aralkyl, |
| Z | represents oxygen or sulphur, |
| A | represents oxygen or sulphur, or represents one of the following groups $-(CH_2)-_n$, |

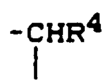, 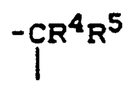 or 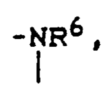,

where

| | |
|---|---|
| n | represents a number from 0 to 6, |
| $R^4$ and $R^5$ | in each case independently of one another represent alkyl, or together represent an alkyl chain having 2 to 7 carbon atoms, and |
| $R^6$ | represents alkyl, or represents a radical |

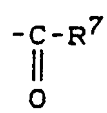

where

$R^7$ represents alkyl, alkoxy or dialkylamino,

B represents the group $CH-R^8$ or $NO-R^9$,

where

$R^8$ represents unsubstituted or substituted aryl or hetaryl and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy.

**2.** Acrylic esters of the formula (I) according to Claim 1, in which

$R^1$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is unsubstituted or monosubstituted or polysubstituted in the aryl moiety by identical or different substituents, suitable aryl substituents being those mentioned in the case of $R^8$,

$R^2$ represents dialkylamino having 1 to 6 carbon atoms in each of the individual straight-chain or branched alkyl moieties, or represents a radical $-Z-R^3$,

where

$R^3$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is unsubstituted or monosubstituted or polysubstituted in the aryl moiety by identical or different substituents, suitable aryl substituents being those mentioned in the case of $R^8$,

Z represents oxygen or sulphur,

A represents oxygen or sulphur, or represents one of the following groups

$-(CH_2)_n-$,

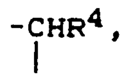 , 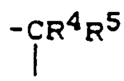 or 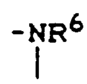

where

n represents a number from 0 to 3,

$R^4$ and $R^5$ in each case independently of one another repre sent straight-chain or branched alkyl having 1 to 6 carbon atoms, or together represent an alkyl chain having 2 to 7 carbon atoms, and

$R^6$ represents straight-chain or branched alkyl having 1 to 6 carbon atoms, or represents a radical

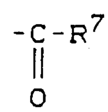

where

$R^7$ represents straight-chain or branched alkyl having 1 to 6 carbon atoms, straight-chain or branched alkoxy having 1 to 6 carbon atoms, or straight-chain or branched dialkylamino having 1 to 6 carbon atoms in each of the individual alkyl moieties,

B represents the group $CH-R^8$ or $NO-R^9$,

where

$R^8$ represents unsubstituted or substituted aryl having 6 to 10 carbon atoms in the aryl moiety, or represents heteroaryl having 2 to 9 carbon atoms and 1 to 4 identical or different hetero atoms - in particular nitrogen, oxygen and/or sulphur - in the heteroaryl moiety,

suitable aryl and heteroaryl substituents in each case being:

halogen, cyano, nitro, in each case straight-chain or branched alkyl, alkoxy or

alkylthio, each of which has 1 to 4 carbon atoms, alkylidenedioxy having 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, cycloalkyl having 3 to 7 carbon atoms, divalent alkanediyl having 3 to 5 carbon atoms, or aryl, aralkyl, aryloxy, arylthio, aralkyloxy or aralkylthio, each of which has 6 to 10 carbon atoms in the aryl moiety and where appropriate 1 to 4 carbon atoms in the straight-chain or branched alkyl moiety and each of which is optionally monosubstituted or polysubstituted in the aryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms, or represents heteroarylalkyl, heteroaryloxy, heteroarylthio or heteroaryl, each of which has 2 to 9 carbon atoms and 1 to 4 identical or different hetero atoms - in particular nitrogen, oxygen and/or sulphur - in the heteroaryl moiety and where appropriate 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms,

$R^9$ represents in each case unsubstituted or substituted alkyl having 1 to 8 carbon atoms, unsubstituted or substituted alkenyl having 2 to 8 carbon atoms or unsubstituted or substituted alkinyl having 2 to 8 carbon atoms, suitable substituents in each case being: cyano, alkoxy having 1 to 4 carbon atoms or alkoxycarbonyl having 1 to 4 carbon atoms in the alkoxy moiety,

furthermore

$R^9$ represents aryl which has 6 to 10 carbon atoms which is unsubstituted or monosubstituted to polysubstituted by identical or different substituents, aralkyl which has 6 to 10 carbon atoms in the aryl moiety and 1 to 2 carbon atoms in the alkyl moiety and which is unsubstituted or monosubstituted to polysubstituted by identical or different substituents, suitable substituents which may be mentioned being: halogen, alkyl having 1 to 4 carbon atoms, alkoxy having 1 to 4 carbon atoms, halogenoalkyl having 1 to 4 carbon atoms and 1 to 9 identical or different halogen atoms, or alkylidenedioxy having 1 to 2 carbon atoms,

$R^9$ also

represents heteroaryl having in each case 2 to 9 carbon atoms and 1 to 4 identical or different hetero atoms - in particular nitrogen, oxygen and/or sulphur - in the heteroaryl moiety, and

R and Q independently of one another represent hydrogen, alkyl having 1 to 4 carbon atoms, alkoxy having 1 to 4 carbon atoms or halogenoalkyl having 1 to 4 carbon atoms and 1 to 9 identical or different halogen atoms.

**3.** Acrylic esters of the formula (I) according to Claim 1, in which

$R^1$ represents methyl, ethyl, n- or i-propyl or n-, i-, s- or t-butyl, or represents benzyl which is optionally monosubstituted to trisubstituted by identical or different substituents, suitable substituents being those mentioned in the case of $R^8$,

$R^2$ represents dialkylamino having 1 to 4 carbon atoms in each of the individual straight-chain or branched alkyl moieties, or represents a radical $-Z-R^3$,

where

$R^3$ represents methyl, ethyl, n- or i-propyl or n-, i-, s- or t-butyl, or represents benzyl which is optionally monosubstituted to trisubstituted by identical or different substituents, suitable substituents being those mentioned in the case of $R^8$, and

Z represents oxygen or sulphur;

A represents oxygen or sulphur, or represents one of the following groups

$-(CH_2)-_n$,

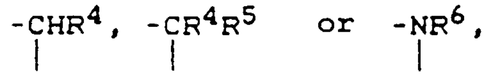

or

where

n represents the numbers 0, 1, 2 and 3,

$R^4$ and $R^5$ in each case independently of one another represent straight-chain or branched alkyl having 1 to 4 carbon atoms, or together represent an alkyl chain having 2 to 5 carbon atoms, and

$R^6$ represents straight-chain or branched alkyl having 1 to 4 carbon atoms, or represents a radical

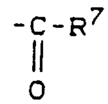

where

$R^7$ represents straight-chain or branched alkyl having 1 to 4 carbon atoms, straight-chain or branched alkoxy having 1 to 4 carbon atoms, or straight-chain or branched dialkylamino having 1 to 4 carbon atoms in each of the individual alkyl radicals,

B represents the group $CH-R^8$ or $NO-R^9$,

where

$R^8$ represents phenyl, naphthyl, pyridinyl, pyrimidinyl, pyrazinyl, pyridazinyl, thienyl, furyl, pyrrolyl, thiazolyl, isothiazolyl, oxazolyl and isoxazolyl, in each case unsubstituted or in each case monosubstituted to trisubstituted by identical or different substituents,

suitable substituents in each case being:

fluorine, chlorine, bromine, cyano, nitro, methyl, ethyl, n- or i-propyl, n-, i-, s- or t-butyl, methoxy, ethoxy, n- or i-propoxy, methylenedioxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, benzyl, phenoxy, benzyloxy, phenylthio or benzylthio, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio;

$R^9$ represents in each case unsubstituted or substituted alkyl having 1 to 6 carbon atoms, unsubstituted or substituted alkenyl having 2 to 6 carbon atoms, and unsubstituted or substituted alkinyl having 2 to 6 carbon atoms, suitable substituents in each case being:

cyano, methoxy, ethoxy, n- and i-propoxy, n-, i-, s- and t-butoxy, methoxycarbonyl, ethoxycarbonyl, n- and i-propoxycarbonyl, n-, i-, s- and t-butoxycarbonyl,

$R^9$ furthermore

represents phenyl or benzyl, in each case unsubstituted or monosubstituted to trisubstituted by identical or different substituents, substituents which may be mentioned being: fluorine, chlorine, bromine, methyl, ethyl, n- or i-propyl, n-, i-, s- or t-butyl, methoxy, ethoxy, n- or i-propoxy, n-, i-, s- or t-butoxy, halogenoalkyl having 1 or 2 carbon atoms and 1 to 5 identical or different fluorine or chlorine atoms, methylidenedioxy and ethylidenedioxy, and

R and Q independently of one another represent hydrogen, methyl, ethyl, n- or i-propyl, methoxy, ethoxy or halogenoalkyl having 1 or 2 carbon atoms and 1 to 5 identical or different fluorine or chlorine atoms.

**4.** Acrylic esters of the formula (I) according to Claim 1, in which

$R^1$ represents methyl, ethyl or benzyl, suitable benzyl substituents being those mentioned in the case of $R^8$,

$R^2$ represents dimethylamino or diethylamino, or represents a radical $-Z-R^3$, where

$R^3$ represents methyl, ethyl, n- or i-propyl or benzyl,

Z represents oxygen or sulphur;

A represents oxygen or sulphur, or represents one of the following groups

$-(CH_2)-_n$, $>CH-CH_3$, $>CH-C_2H_5$,
$>CH-CH(CH_3)_2$, $>CH-C(CH_3)_3$, $>C(CH_3)_2$,

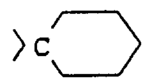,

$>N-COOCH_3$, $>N-COOC_2H_5$,

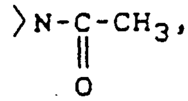, 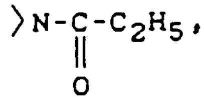,

where

n represents the numbers 0, 1, 2 and 3,

B represents the group $CH-R^8$ or $NO-R^9$, where

$R^8$ represents phenyl, naphthyl, pyridinyl, pyrimidinyl, pyrazinyl, pyridazinyl, thienyl, furyl, pyrrolyl, thiazolyl, isothiazolyl, oxazolyl or isoxazolyl, in each case unsubstituted or in each case monosubstituted to trisubstituted by identical or different substituents,
suitable substituents in each case being:
fluorine, chlorine, bromine, cyano, nitro, methyl, ethyl, n- or i-propyl, n-, i-, s- or t-butyl, methoxy, ethoxy, n- or i-propoxy, methylenedioxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, phenoxy, benzyl, benzyloxy, phenylthio or benzylthio, each of which is optionally monosubstituted or disubstituted by identical or different substituents from the series comprising fluorine, chlorine, bromine, methyl and/or ethyl;

$R^9$ represents in each case unsubstituted or substituted alkyl having 1 - 6 carbon atoms, unsubstituted or substituted alkenyl having 2 to 4 carbon atoms and unsubstituted or substituted alkinyl having 2 to 4 carbon atoms,
suitable substituents in each case being cyano, methoxycarbonyl, ethoxycarbonyl, methoxy or alkoxy;
$R^9$ furthermore represents phenyl or benzyl, in each case unsubstituted or monosubstituted or disubstituted by identical or different substituents,
suitable substituents being: bromine, fluorine, chlorine, methoxy, ethoxy, methylenedioxy, methyl, ethyl and trifluoromethyl, and

R and Q independently of one another represent hydrogen or methyl.

**5.** Acrylic esters of the formula (I) according to Claim 1, in which

$R^1$ represents methyl or ethyl,

$R^2$ represents methoxy, ethoxy, methylthio or dimethylamino,

A represents oxygen or sulphur, or represents one of the groups below

$-(CH_2)-_n$,

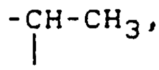, 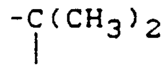

where

n represents the numbers 0, 1 or 2,

B represents the group CH-$R^8$ or NO-$R^9$,

where

$R^8$ represents phenyl, naphthyl, pyridinyl, pyrimidinyl, thienyl, furyl, thiazolyl, oxazolyl or isoxazolyl, each of which is unsubstituted or monosubstituted to trisubstituted by identical or different substituents,
suitable substituents being:
fluorine, chlorine, bromine, cyano, nitro, methyl, ethyl, methoxy, ethoxy, methylenedioxy, methylthio, trifluoromethyl, methoxycarbonyl, ethoxycarbonyl, cyclopentyl, cyclohexyl, phenoxy, phenylthio, benzyloxy or benzylthio;

$R^9$ represents methyl, ethyl, n- and i-propyl, n-, i- and t-butyl, n- and iso-amyl, allyl, methallyl, propargyl, benzyl, o-, m- and p-chlorobenzyl, o-, m- and p-methoxy-benzyl, o-, m- and p-methylbenzyl or o-, m- and p-fluorobenzyl, and

R and Q represent hydrogen.

**6.** Process for the preparation of acrylic esters of the general formula (I)

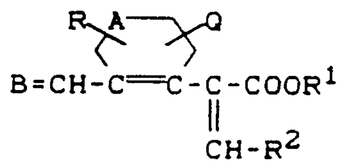

(I)

in which

$R^1$ represents alkyl, or represents unsubstituted or substituted aralkyl,

$R^2$ represents dialkylamino, or represents a radical -Z-$R^3$,

$R^3$ represents alkyl, or represents unsubstituted or substituted aralkyl,

Z represents oxygen or sulphur,

A represents oxygen or sulphur, or represents one of the following groups
$-(CH_2)-_n$,

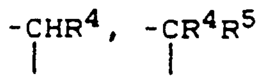 or 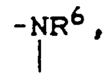,

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

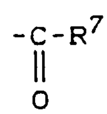

where

$R^7$ represents alkyl, alkoxy or dialkylamino,

B represents the group CH-$R^8$ or NO-$R^9$,
where
$R^8$ represents unsubstituted or substituted aryl or hetaryl and
$R^9$ represents unsubstituted or substituted alkyl, alkenyl, alkinyl, aralkyl, aryl or hetaryl, and
R and Q independently of one another represent hydrogen, alkyl, halogenoalkyl or alkoxy,

characterized in that

a) substituted acrylic esters of the formula (Ia)

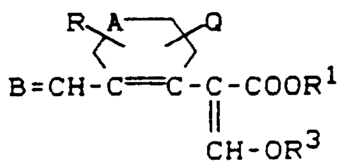

(Ia)

in which
$R^1$, $R^3$, A, B, R and Q have the abovementioned meaning,
are obtained when hydroxyacrylic esters or their alkali metal salts of the formula (II)

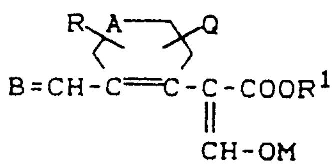

(II)

in which
M represents hydrogen, or represents an alkali metal cation, and
$R^1$, A, B, R and Q have the abovementioned meaning,
are reacted with alkylating agents of the formula (III)

$R^3$-$E^1$ (III)

in which
$E^1$ represents an electron-attracting leaving group and
$R^3$ has the abovementioned meaning,
if appropriate in the presence of a diluent and if appropriate in the presence of a reaction auxiliary;

b) substituted acrylic esters of the formula (Ib)

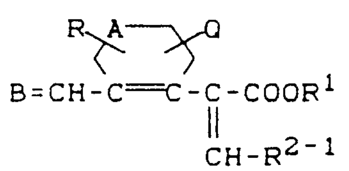

(Ib)

in which
$R^{2-1}$ represents dialkylamino and
$R^1$, A, B, R and Q have the abovementioned meaning,
are obtained when substituted acetic esters of the formula (IV)

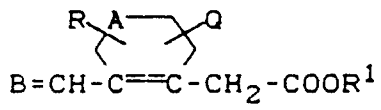

(IV)

in which

$R^1$, A, B, R and Q have the abovementioned meaning,

are reacted with formamides of the formula (Va)

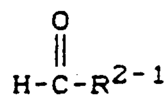

(Va)

in which

$R^{2-1}$ has the abovementioned meaning,

or with formamide derivatives of the formula (Vb)

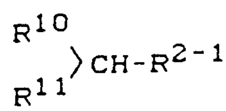

(Vb)

in which

$R^{10}$ and $R^{11}$ independently of one another represent alkoxy or dialkylamino and

$R^{2-1}$ has the abovementioned meaning,

if appropriate in the presence of a diluent;

c) substituted acrylic esters of the formula (Ic)

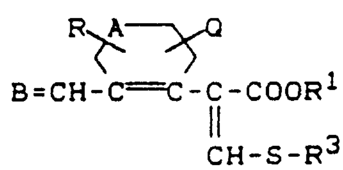

(Ic)

in which

$R^1$, $R^3$, A, B, R and Q have the abovementioned meaning,

are obtained when ketocarboxylic acid derivatives of the formula (VI)

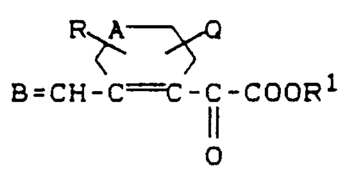

(VI)

in which

$R^1$, A, B, R and Q have the abovementioned meaning,

are reacted with organometallic compounds of the formula (VII)

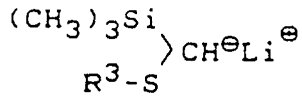 (VII)

in which

$R^3$ has the abovementioned meaning,

if appropriate in the presence of a diluent;

d) substituted acrylic esters of the formula (Ic) are obtained when substituted acrylic esters of the formula (VIII)

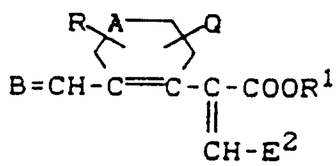 (VIII)

in which

$R^1$, A, B, R and Q have the abovementioned meaning and

$E^2$ represents an electron-attracting leaving group,

are reacted with thiols of the formula (IX)

$R^3$-SH (IX)

in which

$R^3$ has the abovementioned meaning,

if appropriate in the presence of a diluent and if appropriate in the presence of a reaction auxiliary.

**7.** Pesticides, characterized in that they contain at least one acrylic ester of the formula (I) according to Claims 1 to 6.

**8.** Use of acrylic esters of the formula (I) according to Claims 1 to 6 for combating pests.

**9.** Method of combating pests, characterized in that acrylic esters of the formula (I) according to Claims 1 to 6 are allowed to act on pests and/or their habitat.

**10.** Process for the preparation of pesticides, characterized in that acrylic esters of the formula (I) according to Claims 1 to 6 are mixed with extenders and/or surface-active agents.

**11.** Hydroxyacrylic esters of the general formula (II)

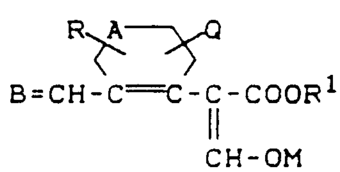 (II)

in which

$R^1$ represents alkyl, or represents unsubstituted or substituted aralkyl,

A represents oxygen or sulphur, or represents one of the following groups

$-(CH_2)_n-$,

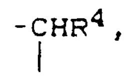

$-CR^4R^5$ or

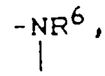

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

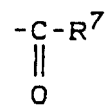

where

$R^7$ represents alkyl, alkoxy or dialkylamino,

B represents the group $CH-R^8$ or $NO-R^9$,

where

$R^8$ represents unsubstituted or substituted aryl or hetaryl, and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy, and

M represents hydrogen, or represents an alkali metal cation.

**12.** Process for the preparation of hydroxyacrylic esters of the formula (II) according to Claim 11, characterized in that substituted acetic esters of the formula (IV)

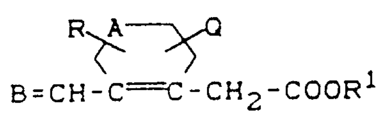

(IV)

in which

$R^1$, A, B, R and Q have the abovementioned meaning,

are reacted with formic esters of the formula (X)

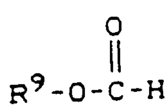

(X)

in which

$R^9$ represents alkyl, in particular methyl or ethyl,

if appropriate in the presence of a diluent and if appropriate in the presence of a basic reaction auxiliary, at temperatures of from -20 °C to +50 °C.

**13.** Acetic esters of the general formula (IV)

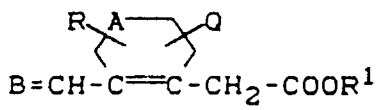 (IV)

in which

| | |
|---|---|
| $R^1$ | represents alkyl, or represents unsubstituted or substituted aralkyl, |
| A | represents oxygen or sulphur, or represents one of the following groups $-(CH_2)-_n$, |

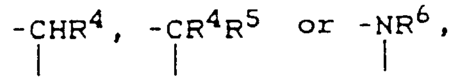

where

| | |
|---|---|
| n | represents a number from 0 to 6, |
| $R^4$ and $R^5$ | in each case independently of one another represent alkyl, or together represent an alkyl chain having 2 to 7 carbon atoms, and |
| $R^6$ | represents alkyl, or represents a radical |

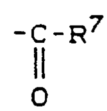

where

| | |
|---|---|
| $R^7$ | represents alkyl, alkoxy or dialkylamino, |
| B | represents the group $CH-R^8$ or $NO-R^9$, where |
| $R^8$ | represents unsubstituted or substituted aryl or hetaryl and |
| $R^9$ | represents unsubstituted or substituted alkyl, alkenyl, alkinyl, aralkyl, aryl or hetaryl, and |
| R and Q | independently of one another represent hydrogen, alkyl, halogenoalkyl or alkoxy. |

**14.** Process for the preparation of acetic esters of the formula (IV) according to Claim 13, characterized in that

a) in the event that $R^1$, A, R and Q have the above-mentioned meaning and

| | |
|---|---|
| $B^1$ | represents the group $N-O-R^9$, where |
| $R^9$ | has the abovementioned meaning, aldehydes of the general formula (XI) |

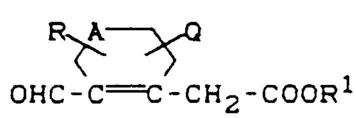 (XI)

in which

| | |
|---|---|
| $R^1$, A, R and Q | have the abovementioned meaning, |

are reacted with compounds of the general formula (XII)

$R^9-O-NH_2$ (XII)

in which

$R^9$ has the abovementioned meaning,

if appropriate in the presence of a diluent, at temperatures of from -20 °C to +50 °C,

or

b) in the event that $R^1$, A, R and Q have the abovementioned meaning and

$B^2$ represents the group $CH-R^8$,
where

$R^8$ has the abovementioned meaning,

aldehydes of the general formula (XI)

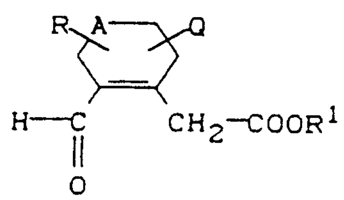

(XI)

in which

$R^1$, A, R and Q have the abovementioned meaning,

α) are reacted with phosphonium compounds of the general formula (XIII)

$R^8-CH_2-P(R^{12})_3{}^{\oplus}X^{\ominus}$ (XIII)

in which

$R^8$ has the abovementioned meaning,

$R^{12}$ represents aryl which is optionally substituted by methyl, ethyl, methoxy or ethoxy, preferably phenyl, and

X represents halogen, preferably bromine or chlorine, or

β) are reacted with reagents of the general formula (XIV)

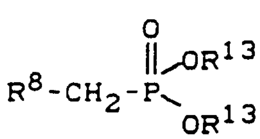

(XIV)

in which

$R^8$ has the abovementioned meaning and

$R^{13}$ represents $C_{1-4}$-alkyl, particularly preferably methyl, ethyl or n-butyl,

using a solvent which is inert under the reaction conditions and, if appropriate, a basic auxiliary, at temperatures of from -80 °C to +60 °C, in the sense of a WITTIG reaction or of a HORNER-WITTIG reaction.

**15.** Acrylic esters of the general formula (VIII)

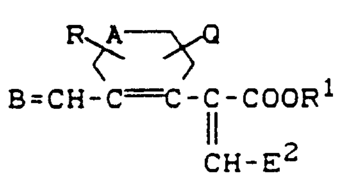

(VIII)

in which

$R^1$ represents alkyl, or represents unsubstituted or substituted aralkyl,

A represents oxygen or sulphur, or represents one of the following groups

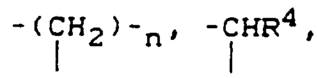

$-CR^4R^5$ or

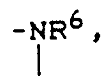

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

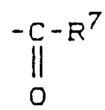

where

$R^7$ represents alkyl, alkoxy or dialkylamino,

B represents the group $CH-R^8$ or $NO-R^9$,

where

$R^8$ represents unsubstituted or substituted aryl or hetaryl, and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy, and

$E^2$ represents an electron-attracting leaving group.

**16.** Process for the preparation of acrylic esters of the formula (VIII) according to Claim 15, characterized in that hydroxyacrylic esters of the formula (II)

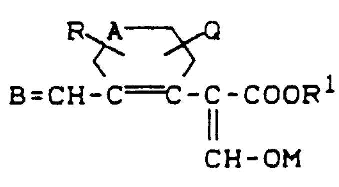

(II)

in which

$R^1$, A, B, R and Q have the abovementioned meaning and M represents hydrogen or an alkali metal cation,

are reacted with acid chlorides of the formula (XIV)

$R^{14}-Cl$ (XIV)

in which

$R^{14}$ represents an acyl or sulphonyl radical, in particular an acetyl radical, a methanesulphonyl radical or a p-toluenesulphonyl radical,

if appropriate in the presence of a diluent and if appropriate in the presence of an acid-binding agent, at temperatures of from -20 °C to +120 °C.

**Claims for the following Contracting State : ES**

1. Process for the preparation of acrylic esters of the general formula (I)

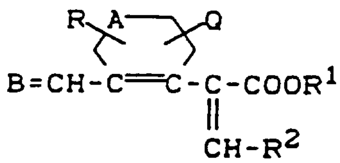

(I)

in which

$R^1$ represents alkyl, or represents unsubstituted or substituted aralkyl,

$R^2$ represents dialkylamino, or represents a radical $-Z-R^3$,

$R^3$ represents alkyl, or represents unsubstituted or substituted aralkyl,

Z represents oxygen or sulphur,

A represents oxygen or sulphur, or represents one of the following groups $-(CH_2)-_n$,

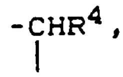, 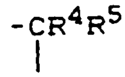 or 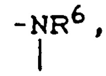

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

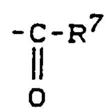

where

$R^7$ represents alkyl, alkoxy or dialkylamino,

B represents the group $CH-R^8$ or $NO-R^9$,

where

$R^8$ represents unsubstituted or substituted aryl or hetaryl and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy,

characterized in that

a) substituted acrylic esters of the formula (Ia)

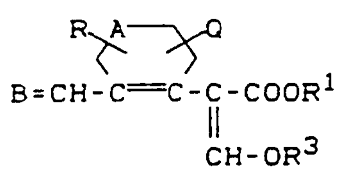

(Ia)

in which

$R^1$, $R^3$, A, B, R and Q have the abovementioned meaning,

are obtained when hydroxyacrylic esters or their alkali metal salts of the formula (II)

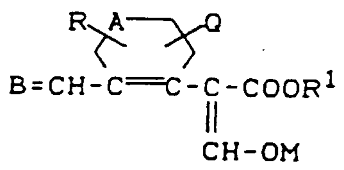

(II)

in which

M represents hydrogen, or represents an alkali metal cation, and

$R^1$, A, B, R and Q have the abovementioned meaning,

are reacted with alkylating agents of the formula (III)

$R^3$-$E^1$ (III)

in which

$E^1$ represents an electron-attracting leaving group and

$R^3$ has the abovementioned meaning,

if appropriate in the presence of a diluent and if appropriate in the presence of a reaction auxiliary;

b) substituted acrylic esters of the formula (Ib)

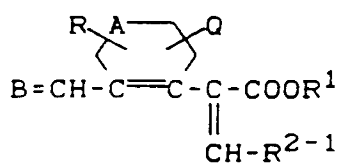

(Ib)

in which

$R^{2-1}$ represents dialkylamino and

$R^1$, A, B, R and Q have the abovementioned meaning,

are obtained when substituted acetic esters of the formula (IV)

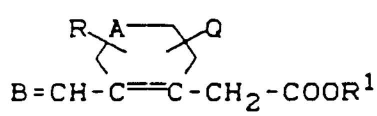

(IV)

in which

$R^1$, A, B, R and Q have the abovementioned meaning,

are reacted with formamides of the formula (Va)

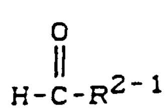

(Va)

in which

$R^{2-1}$ has the abovementioned meaning,

or with formamide derivatives of the formula (Vb)

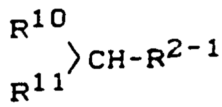

(Vb)

in which

$R^{10}$ and $R^{11}$ independently of one another represent alkoxy or dialkylamino and

$R^{2-1}$ has the abovementioned meaning,

if appropriate in the presence of a diluent;

c) substituted acrylic eaters of the formula (Ic)

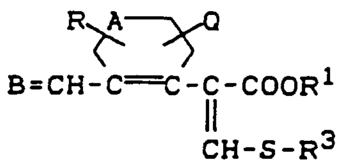

(Ic)

in which

$R^1$, $R^3$, A, B, R and Q have the abovementioned meaning,

are obtained when ketocarboxylic acid derivatives of the formula (VI)

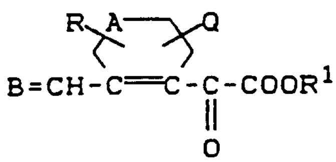

(VI)

in which

$R^1$, A, B, R and Q have the abovementioned meaning,

are reacted with organometallic compounds of the formula (VII)

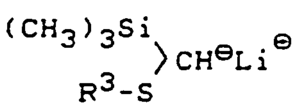

(VII)

in which

$R^3$ has the abovementioned meaning,

if appropriate in the presence of a diluent;

d) substituted acrylic esters of the formula (Ic) are obtained when substituted acrylic esters of the formula (VIII)

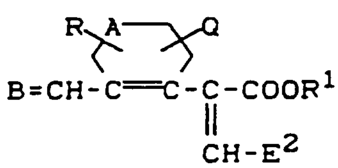

(VIII)

in which

$R^1$, A, B, R and Q have the abovementioned meaning and
$E^2$ represents an electron-attracting leaving group,

are reacted with thiols of the formula (IX)

$R^3$-SH (IX)

in which
$R^3$ has the abovementioned meaning,

if appropriate in the presence of a diluent and if appropriate in the presence of a reaction auxiliary.

**2.** Process according to Claim 1, in which

$R^1$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is unsubstituted or monosubstituted or polysubstituted in the aryl moiety by identical or different substituents, suitable aryl substituents being those mentioned in the case of $R^8$,

$R^2$ represents dialkylamino having 1 to 6 carbon atoms in each of the individual straight-chain or branched alkyl moieties, or represents a radical -Z-$R^3$,
where

$R^3$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is unsubstituted or monosubstituted or polysubstituted in the aryl moiety by identical or different substituents, suitable aryl substituents being those mentioned in the case of $R^8$,

Z represents oxygen or sulphur,

A represents oxygen or sulphur, or represents one of the following groups
$-(CH_2)_n-$,

$$-\underset{|}{C}HR^4,\quad -\underset{|}{C}R^4R^5 \text{ or } -\underset{|}{N}R^6$$

where

n represents a number from 0 to 3,

$R^4$ and $R^5$ in each case independently of one another represent straight-chain or branched alkyl having 1 to 6 carbon atoms, or together represent an alkyl chain having 2 to 7 carbon atoms, and

$R^6$ represents straight-chain or branched alkyl having 1 to 6 carbon atoms, or represents a radical

$$-\underset{\underset{O}{\|}}{C}-R^7$$

where

$R^7$ represents straight-chain or branched alkyl having 1 to 6 carbon atoms, straight-chain or branched alkoxy having 1 to 6 carbon atoms, or straight-chain or branched dialkylamino having 1 to 6 carbon atoms in each of the individual alkyl moieties,

B represents the group CH-$R^8$ or NO-$R^9$,
where

$R^8$ represents unsubstituted or substituted aryl having 6 to 10 carbon atoms in the aryl moiety, or represents heteroaryl having 2 to 9 carbon atoms and 1 to 4 identical or different hetero atoms - in particular nitrogen, oxygen and/or sulphur - in the heteroaryl moiety,

suitable aryl and heteroaryl substituents in each case being: halogen, cyano, nitro, in each case straight-chain or branched alkyl, alkoxy or alkylthio, each of which has 1 to 4 carbon atoms, alkylidenedioxy having 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, cycloalkyl having 3 to 7 carbon atoms, divalent alkanediyl having 3 to 5 carbon atoms, or aryl, aralkyl, aryloxy, arylthio, aralkyloxy or aralkylthio, each of which has 6 to 10 carbon atoms in the aryl moiety and where appropriate 1 to 4 carbon atoms in the straight-chain or branched alkyl moiety and each of which is optionally monosubstituted or polysubstituted in the aryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms, or represents heteroarylalkyl, heteroaryloxy, heteroarylthio or heteroaryl, each of which has 2 to 9 carbon atoms and 1 to 4 identical or different hetero atoms -in particular nitrogen, oxygen and/or sulphur - in the heteroaryl moiety and where appropriate 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms,

$R^9$ represents in each case unsubstituted or substituted alkyl having 1 to 8 carbon atoms, unsubstituted or substituted alkenyl having 2 to 8 carbon atoms or unsubstituted or substituted alkinyl having 2 to 8 carbon atoms, suitable substituents in each case being: cyano, alkoxy having 1 to 4 carbon atoms or alkoxycarbonyl having 1 to 4 carbon atoms in the alkoxy moiety,

furthermore

$R^9$ represents aryl which has 6 to 10 carbon atoms which is unsubstituted or monosubstituted to polysubstituted by identical or different substituents, aralkyl which has 6 to 10 carbon atoms in the aryl moiety and 1 to 2 carbon atoms in the alkyl moiety and which is unsubstituted or monosubstituted to polysubstituted by identical or different substituents, suitable substituents which may be mentioned being: halogen, alkyl having 1 to 4 carbon atoms, alkoxy having 1 to 4 carbon atoms, halogenoalkyl having 1 to 4 carbon atoms and 1 to 9 identical or different halogen atoms, or alkylidenedioxy having 1 to 2 carbon atoms,

$R^9$ also

represents heteroaryl having in each case 2 to 9 carbon atoms and 1 to 4 identical or different hetero atoms - in particular nitrogen, oxygen and/or sulphur - in the heteroaryl moiety, and

R and Q independently of one another represent hy drogen, alkyl having 1 to 4 carbon atoms, alkoxy having 1 to 4 carbon atoms or halogenoalkyl having 1 to 4 carbon atoms and 1 to 9 identical or different halogen atoms.

**3.** Process according to Claim 1, in which

$R^1$ represents methyl, ethyl, n- or i-propyl or n-, i-, s- or t-butyl, or represents benzyl which is optionally monosubstituted to trisubstituted by identical or different substituents, suitable substituents being those mentioned in the case of $R^8$,

$R^2$ represents dialkylamino having 1 to 4 carbon atoms in each of the individual straight-chain or branched alkyl moieties, or represents a radical $-Z-R^3$,

where

$R^3$ represents methyl, ethyl, n- or i-propyl or n-, i-, s- or t-butyl, or represents benzyl which is optionally monosubstituted to trisubstituted by identical or different substituents, suitable substituents being those mentioned in the case of $R^8$, and

Z represents oxygen or sulphur;

A represents oxygen or sulphur, or represents one of the following groups

$-(CH_2)-_n$,

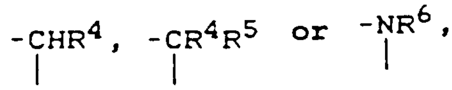

where

n represents the numbers 0, 1, 2 and 3,

$R^4$ and $R^5$ in each case independently of one another represent straight-chain or branched alkyl having 1 to 4 carbon atoms, or together represent an alkyl chain having 2 to 5 carbon atoms, and

$R^6$ represents straight-chain or branched alkyl having 1 to 4 carbon atoms, or represents a radical

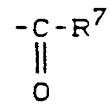

where

$R^7$ represents straight-chain or branched alkyl having 1 to 4 carbon atoms, straight-chain or branched alkoxy having 1 to 4 carbon atoms, or straight-chain or branched dialkylamino having 1 to 4 carbon atoms in each of the individual alkyl radicals,

B represents the group CH-$R^8$ or NO-$R^9$,

where

$R^8$ represents phenyl, naphthyl, pyridinyl, pyrimidinyl, pyrazinyl, pyridazinyl, thienyl, furyl, pyrrolyl, thiazolyl, isothiazolyl, oxazolyl and isoxazolyl, in each case unsubstituted or in each case monosubstituted to trisubstituted by identical or different substituents,

suitable substituents in each case being:

fluorine, chlorine, bromine, cyano, nitro, methyl, ethyl, n- or i-propyl, n-, i-, s- or t-butyl, methoxy, ethoxy, n- or i-propoxy, methylenedioxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, benzyl, phenoxy, benzyloxy, phenylthio or benzylthio, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio;

$R^9$ represents in each case unsubstituted or substituted alkyl having 1 to 6 carbon atoms, unsubstituted or substituted alkenyl having 2 to 6 carbon atoms, and unsubstituted or substituted alkinyl having 2 to 6 carbon atoms, suitable substituents in each case being: cyano, methoxy, ethoxy, n- and i-propoxy, n-, i-, s- and t-butoxy, methoxycarbonyl, ethoxycarbonyl, n- and i-propoxycarbonyl, n-, i-, s- and t-butoxycarbonyl,

$R^9$ furthermore

represents phenyl or benzyl, in each case unsubstituted or monosubstituted to trisubstituted by identical or different substituents, substituents which may be mentioned being:

fluorine, chlorine, bromine, methyl, ethyl, n- or i-propyl, n-, i-, s- or t-butyl, methoxy, ethoxy, n- or i-propoxy, n-, i-, s- or t-butoxy, halogenoalkyl having 1 or 2 carbon atoms and 1 to 5 identical or different fluorine or chlorine atoms, methylidenedioxy and ethylidenedioxy, and

R and Q independently of one another represent hydrogen, methyl, ethyl, n- or i-propyl, methoxy, ethoxy or halogenoalkyl having 1 or 2 carbon atoms and 1 to 5 identical or different fluorine or chlorine atoms.

**4.** Process according to Claim 1, in which

$R^1$ represents methyl, ethyl or benzyl, suitable benzyl substituents being those mentioned in the case of $R^8$,

$R^2$ represents dimethylamino or diethylamino, or represents a radical $-Z-R^3$,

where

$R^3$ represents methyl, ethyl, n- or i-propyl or benzyl,

Z represents oxygen or sulphur;

A represents oxygen or sulphur, or represents one of the following groups

$-(CH_2)-_n$, $>CH-CH_3$, $>CH-C_2H_5$,

$>CH-CH(CH_3)_2$, $>CH-C(CH_3)_3$, $>C(CH_3)_2$,

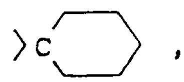 ,

$>N-COOCH_3$, $>N-COOC_2H_5$,

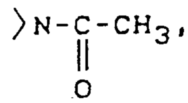 , 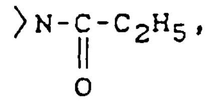 ,

where

n represents the numbers 0, 1, 2 and 3,

B represents the group $CH-R^8$ or $NO-R^9$,

where

$R^8$ represents phenyl, naphthyl, pyridinyl, pyrimidinyl, pyrazinyl, pyridazinyl, thienyl, furyl, pyrrolyl, thiazolyl, isothiazolyl, oxazolyl or isoxazolyl, in each case unsubstituted or in each case monosubstituted to trisubstituted by identical or different substituents,

suitable substituents in each case being:

fluorine, chlorine, bromine, cyano, nitro, methyl, ethyl, n- or i-propyl, n-, i-, s- or t-butyl, methoxy, ethoxy, n- or i-propoxy, methylenedioxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, phenoxy, benzyl, benzyloxy, phenylthio or benzylthio, each of which is optionally monosubstituted or disubstituted by identical or different substituents from the series comprising fluorine, chlorine, bromine, methyl and/or ethyl;

$R^9$ represents in each case unsubstituted or substituted alkyl having 1 - 6 carbon atoms, unsubstituted or substituted alkenyl having 2 to 4 carbon atoms and unsubstituted or substituted alkinyl having 2 to 4 carbon atoms,

suitable substituents in each case being cyano, methoxycarbonyl, ethoxycarbonyl, methoxy or alkoxy;

$R^9$ furthermore represents phenyl or benzyl, in each case unsubstituted or monosubstituted or disubstituted by identical or different substituents,

suitable substituents being: bromine, fluorine, chlorine, methoxy, ethoxy, methylenedioxy, methyl, ethyl and trifluoromethyl, and

R and Q independently of one another represent hydrogen or methyl.

**5.** Process according to Claim 1, in which

$R^1$ represents methyl or ethyl,

$R^2$ represents methoxy, ethoxy, methylthio or dimethylamino,

A represents oxygen or sulphur, or represents one of the groups below

$-(CH_2)-_n$,

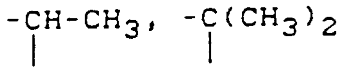

where

| | |
|---|---|
| n | represents the numbers 0, 1 or 2, |
| B | represents the group CH-$R^8$ or NO-$R^9$, where |
| $R^8$ | represents phenyl, naphthyl, pyridinyl, pyrimidinyl, thienyl, furyl, thiazolyl, oxazolyl or isoxazolyl, each of which is unsubstituted or monosubstituted to trisubstituted by identical or different substituents, suitable substituents being: fluorine, chlorine, bromine, cyano, nitro, methyl, ethyl, methoxy, ethoxy, methylenedioxy, methylthio, trifluoromethyl, methoxycarbonyl, ethoxycarbonyl, cyclopentyl, cyclohexyl, phenoxy, phenylthio, benzyloxy or benzylthio; |
| $R^9$ | represents methyl, ethyl, n- and i-propyl, n-, i- and t-butyl, n- and iso-amyl, allyl, methallyl, propargyl, benzyl, o-, m- and p-chlorobenzyl, o-, m- and p-methoxy-benzyl, o-, m- and p-methyl-benzyl or o-, m- and p-fluorobenzyl, and |
| R and Q | represent hydrogen. |

**6.** Pesticides, characterized in that they contain at least one acrylic ester of the formula (I) according to Claims 1 to 5.

**7.** Use of acrylic esters of the formula (I) according to Claims 1 to 5 for combating pests.

**8.** Method of combating pests, characterized in that acrylic esters of the formula (I) according to Claims 1 to 5 are allowed to act on pests and/or their habitat.

**9.** Process for the preparation of pesticides, characterized in that acrylic esters of the formula (I) according to Claims 1 to 5 are mixed with extenders and/or surface-active agents.

**10.** Process for the preparation of hydroxyacrylic esters of the general formula (II)

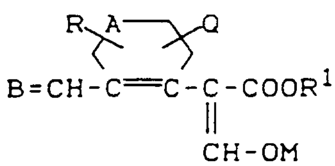

(II)

in which

| | |
|---|---|
| $R^1$ | represents alkyl, or represents unsubstituted or substituted aralkyl, |
| A | represents oxygen or sulphur, or represents one of the following groups |

$-(CH_2)-_n$,

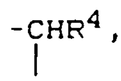

$-CH^4R^5$ or

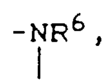

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

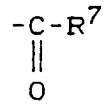

where

$R^7$ represents alkyl, alkoxy or dialkylamino,

B represents the group $CH-R^8$ or $NO-R^9$,

where

$R^8$ represents unsubstituted or substituted aryl or hetaryl, and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy, and

M represents hydrogen, or represents an alkali metal cation,

characterized in that substituted acetic esters of the formula (IV)

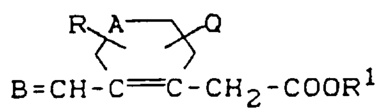
(IV)

in which

$R^1$, A, B, R and Q have the abovementioned meaning,

are reacted with formic esters of the formula (X)

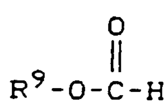
(X)

in which

$R^9$ represents alkyl, in particular methyl or ethyl,

if appropriate in the presence of a diluent and if appropriate in the presence of a basic reaction auxiliary, at temperatures of from -20 °C to +50 °C.

**11.** Process for the preparation of acetic esters of the general formula (IV)

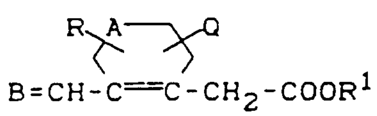
(IV)

in which

$R^1$ represents alkyl, or represents unsubstituted or substituted aralkyl,

A represents oxygen or sulphur, or represents one of the following groups

$-(CH_2)-_n$,

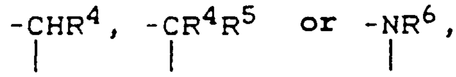

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

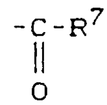

where

$R^7$ represents alkyl, alkoxy or dialkylamino,

B represents the group $CH-R^8$ or $NO-R^9$,

where

$R^8$ represents unsubstituted or substituted aryl or hetaryl and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy,

characterized in that

a) in the event that $R^1$, A, R and Q have the abovementioned meaning and

$B^1$ represents the group $N-O-R^9$,

where

$R^9$ has the abovementioned meaning,

aldehydes of the general formula (XI)

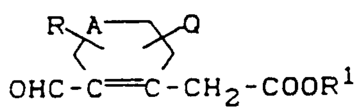 (XI)

in which

$R^1$, A, R and Q have the abovementioned meaning,

are reacted with compounds of the general formula (XII)

$R^9-O-NH_2$ (XII)

in which

$R^9$ has the abovementioned meaning,

if appropriate in the presence of a diluent, at temperatures of from -20 °C to +50 °C,

or

b) in the event that $R^1$, A, R and Q have the abovementioned meaning and

$B^2$ represents the group $CH-R^8$,

where

$R^8$ has the abovementioned meaning,

aldehydes of the general formula (XI)

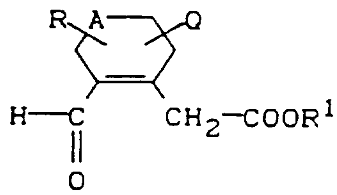

(XI)

in which

$R^1$, A, R and Q have the abovementioned meaning,

α) are reacted with phosphonium compounds of the general formula (XIII)

$R^8\text{-}CH_2\text{-}P(R^{12})_3{}^{\oplus}X^{\ominus}$ (XIII)

in which

R $^8$ has the abovementioned meaning,

$R^{12}$ represents aryl which is optionally substituted by methyl, ethyl, methoxy or ethoxy, preferably phenyl, and

X represents halogen, preferably bromine or chlorine, or

β) are reacted with reagents of the general formula (XIV)

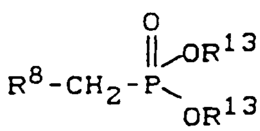

(XIV)

in which

$R^8$ has the abovementioned meaning and

$R^{13}$ represents $C_{1-4}$-alkyl, particularly preferably methyl, ethyl or n-butyl,

using a solvent which is inert under the reaction conditions and, if appropriate, a basic auxiliary, at temperatures of from -80 °C to +60 °C, in the sense of a WITTIG reaction or of a HORNER-WITTIG reaction.

**12.** Process for the preparation of acrylic esters of the general formula (VIII)

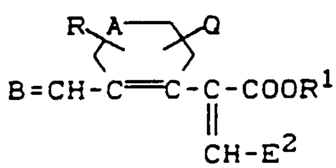

(VIII)

in which

$R^1$ represents alkyl, or represents unsubstituted or substituted aralkyl,

A represents oxygen or sulphur, or represents one of the following groups

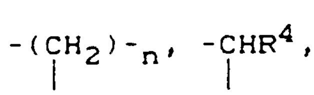

$-CR^4R^5$ or

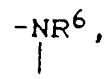

where

n represents a number from 0 to 6,

$R^4$ and $R^5$ in each case independently of one another represent alkyl, or together represent an alkyl chain having 2 to 7 carbon atoms, and

$R^6$ represents alkyl, or represents a radical

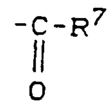

where

$R^7$ represents alkyl, alkoxy or dialkylamino,

B represents the group CH-$R^8$ or NO-$R^9$,

where

$R^8$ represents unsubstituted or substituted aryl or hetaryl, and

$R^9$ represents unsubstituted or substituted alkyl, alkenyl, alkinyl, aralkyl, aryl or hetaryl, and

R and Q independently of one another represent hydrogen, alkyl, halogenoalkyl or alkoxy, and

$E^2$ represents an electron-attracting leaving group,

characterized in that hydroxyacrylic esters of the formula (II)

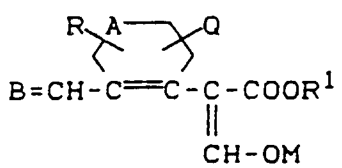

(II)

in which

$R^1$, A, B, R and Q have the abovementioned meaning and M represents hydrogen or an alkali metal cation,

are reacted with acid chlorides of the formula (XIV)

$R^{14}$-Cl (XIV)

in which

$R^{14}$ represents an acyl or sulphonyl radical, in particular an acetyl radical, a methanesulphonyl radical or a p-toluenesulphonyl radical,

if appropriate in the presence of a diluent and if appropriate in the presence of an acid-binding agent, at temperatures of from -20 °C to +120 °C.

**Revendications**

**Revendications pour les Etats contractants suivants : AT, BE, CH, DE, DK, FR, GB, GR, IT, LI, NL**

**1.** Esters d'acides acryliques substitués de formule générale (I)

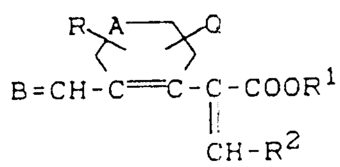

(I)

dans laquelle

| | |
|---|---|
| $R^1$ | est un groupe alkyle ou un groupe aralkyle substitué ou non substitué, |
| $R^2$ | est un groupe dialkylamino ou un reste $-Z-R^3$, |
| $R^3$ | est un groupe alkyle ou un groupe aralkyle non substitué ou substitué, |
| Z | est de l'oxygène ou du soufre |
| A | est de l'oxygène, du soufre ou l'un des groupements suivants : $-(CH_2)-_n$, |

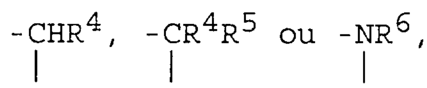

ou

dans lesquels

| | |
|---|---|
| n | est un nombre de 0 à 6, |
| $R^4$ et $R^5$ | représentent chacun, indépendamment l'un de l'autre, un groupe alkyle ou forment conjointement une chaîne alkylique de 2 à 7 atomes de carbone et |
| $R^6$ | est un groupe alkyle ou un reste |

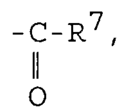

dans lequel

| | |
|---|---|
| $R^7$ | est un groupe alkyle, alkoxy ou dialkylamino, |
| B | est le groupe $CH-R^8$ ou $NO-R^9$, où |
| $R^8$ | est un groupe aryle ou hétaryle non substitué ou substitué et |
| $R^9$ | est un groupe alkyle, alcényle, alcynyle, aralkyle, aryle ou hétaryle non substitué ou substitué et |
| R et Q | représentent, indépendamment l'un de l'autre, de l'hydrogène, un groupe alkyle, halogénalkyle ou alkoxy. |

**2.** Esters d'acides acryliques de formule (I) suivant la revendication 1, dans lesquels

| | |
|---|---|
| $R^1$ | est un groupe alkyle linéaire ou ramifié ayant 1 à 6 atomes de carbone ou un groupe aralkyle non substitué ou portant dans la partie aryle un ou plusieurs substituants identiques ou différents et ayant 1 à 4 atomes de carbone dans la partie alkyle linéaire ou ramifiée et 6 à 10 atomes de carbone dans la partie aryle, en considérant comme substituants du groupe aryle les substituants mentionnés pour $R^8$, |
| $R^2$ | est un groupe dialkylamino ayant 1 à 6 atomes de carbone dans chacune des parties alkyle individuelles linéaires ou ramifiées ou un reste $-Z-R^3$, dans lequel |
| $R^3$ | est un groupe alkyle linéaire ou ramifié ayant 1 à 6 atomes de carbone ou un groupe |

aralkyle non substitué ou portant dans la partie aryle un ou plusieurs substituants identiques ou différents, ayant 1 à 4 atomes de carbone dans la partie alkyle linéaire ou ramifiée et 6 à 10 atomes de carbone dans la partie aryle, en considérant comme substituants du groupe aryle les substituants mentionnés pour $R^8$,

Z est de l'oxygène ou du soufre,

A est de l'oxygène, du soufre ou l'un des groupements suivants :
$-(CH_2)-_n$,

$$-\underset{|}{C}HR^4,\quad -\underset{|}{C}R^4R^5 \text{ ou } -\underset{|}{N}R^6,$$

dans lesquels

n est un nombre de 0 à 3,

$R^4$ et $R^5$ représentent chacun, indépendamment l'un de l'autre, un groupe alkyle linéaire ou ramifié ayant 1 à 6 atomes de carbone ou forment conjointement une chaîne alkylique de 2 à 7 atomes de carbone et

$R^6$ est un groupe alkyle linéaire ou ramifié ayant 1 à 6 atomes de carbone ou un reste

$$-\underset{\underset{O}{\|}}{C}-R^7$$

dans lequel

$R^7$ est un groupe alkyle linéaire ou ramifié ayant 1 à 6 atomes de carbone, un groupe alkoxy linéaire ou ramifié ayant 1 à 6 atomes de carbone ou un groupe dialkylamino linéaire ou ramifié ayant 1 à 6 atomes de carbone dans chacune des parties alkyle individuelles,

B est le groupe $CH-R^8$ ou $NO-R^9$,
dans lequel

$R^8$ est un groupe aryle non substitué ou substitué ayant 6 à 10 atomes de carbone dans la partie aryle ou un groupe hétéroaryle ayant 2 à 9 atomes de carbone et 1 à 4 hétéroatomes identiques ou différents - notamment azote, oxygène et/ou soufre - dans la partie hétéroaryle,
en considérant dans chaque cas comme substituants de la partie aryle et de la partie hétéroaryle :
un halogène, un radical cyano, nitro, un radical alkyle, alkoxy ou alkylthio linéaire ou ramifié ayant chacun 1 à 4 atomes de carbone, un radical alkylidènedioxy ayant 1 à 6 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un radical alkoxycarbonyle ou alkoximinoalkyle linéaire ou ramifié ayant chacun 1 à 4 atomes de carbone dans les parties alkyle individuelles, un radical cycloalkyle de 3 à 7 atomes de carbone, un radical alcanediyle divalent ayant 3 à 5 atomes de carbone, un radical aryle, aralkyle, aryloxy, arylthio, aralkyloxy ou aralkylthio ayant chacun 6 à 10 atomes de carbone dans la partie aryle et le cas échéant 1 à 4 atomes de carbone dans la partie alkyle linéaire ou ramifiée, chacun portant le cas échéant dans la partie aryle un ou plusieurs substituants halogéno, alkyle, alkoxy, alkylthio, halogénalkyle, halogénalkoxy ou halogénalkylthio identiques ou différents ayant chacun 1 à 4 atomes de carbone et le cas échéant 1 à 9 atomes d'halogènes identiques ou différents, ou un radical hétéroarylalkyle, hétéroaryloxy, hétéroarylthio ou hétéroaryle ayant chacun 2 à 9 atomes de carbone et 1 à 4 hétéroatomes - notamment azote, oxygène et/ou soufre - identiques ou différents dans la partie hétéroaryle et le cas échéant 1 à 4 atomes de carbone dans la partie alkyle linéaire ou ramifiée, portant chacun le cas échéant dans la partie hétéroaryle un ou plusieurs substituants halogéno, alkyle, alkoxy, alkylthio, halogénalkyle, halogénalkoxy ou halogénalkylthio identiques ou différents ayant chacun 1 à 4 atomes de carbone et

le cas échéant 1 à 9 atomes d'halogènes identiques ou différents,

$R^9$ est un groupe alkyle non substitué ou substitué ayant 1 à 8 atomes de carbone, un groupe alcényle non substitué ou substitué ayant 2 à 8 atomes de carbone ou un groupe alcynyle non substitué ou substitué ayant 2 à 8 atomes de carbone, en considérant dans chaque cas comme substituants : un radical cyano, alkoxy ayant 1 à 4 atomes de carbone ou alkoxycarbonyle ayant 1 à 4 atomes de carbone dans la partie alkoxy,
en outre
un groupe aryle de 6 à 10 atomes de carbone non substitué ou portant un ou plusieurs substituants identiques ou différents, un groupe aralkyle de 6 à 10 atomes de carbone dans la partie aryle et 1 ou 2 atomes de carbone dans la partie alkyle non substitué ou portant un ou plusieurs substituants identiques ou différents, en considérant comme substituants : un radical halogéno, alkyle ayant 1 à 4 atomes de carbone, alkoxy ayant 1 à 4 atomes de carbone, halogénalkyle ayant 1 à 4 atomes de carbone et 1 à 9 atomes d'halogènes identiques ou différents ou alkylidènedioxy ayant 1 ou 2 atomes de carbone,
en outre
un groupe hétéroaryle ayant 2 à 9 atomes de carbone et 1 à 4 hétéroatomes identiques ou différents - en particulier azote, oxygène et/ou soufre - dans la partie hétéroaryle et

R et Q représentent, indépendamment l'un de l'autre, de l'hydrogène, un groupe alkyle ayant 1 à 4 atomes de carbone, alkoxy ayant 1 à 4 atomes de carbone ou halogénalkyle ayant 1 à 4 atomes de carbone et 1 à 9 atomes d'halogènes identiques ou différents.

**3.** Esters d'acides acryliques de formule (I) suivant la revendication 1, dans lesquels

$R^1$ est un groupe méthyle, éthyle, n-propyle, isopropyle, n-butyle, isobutyle, sec.-butyle, tertio-butyle ou un groupe benzyle portant le cas échéant 1 à 3 substituants identiques ou différents, en considérant comme substituants ceux qui sont mentionnés pour $R^8$,

$R^2$ est un groupe dialkylamino ayant 1 à 4 atomes de carbone dans les parties alkyle individuelles linéaires ou ramifiées ou un reste $-Z-R^3$,
dans lequel

$R^3$ est un groupe méthyle, éthyle, n-propyle, isopropyle, n-butyle, isobutyle, sec.-butyle ou tertio-butyle ou un groupe benzyle portant le cas échéant 1 à 3 substituants identiques ou différents, en considérant comme substituants ceux qui ont été mentionnés pour $R^8$, et

Z est de l'oxygène ou du soufre ;

A est de l'oxygène, du soufre ou l'un des groupements suivants
$-(CH_2)-_n$,

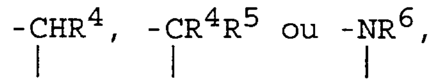

dans lesquels

n représente les nombres 0, 1, 2 et 3,

$R^4$ et $R^5$ représentent chacun indépendamment l'un de l'autre un groupe alkyle linéaire ou ramifié ayant 1 à 4 atomes de carbone ou forment conjointement une chaîne alkylique de 2 à 5 atomes de carbone et

$R^6$ est un groupe alkyle linéaire ou ramifié ayant 1 à 4 atomes de carbone ou un reste

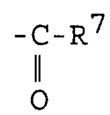

dans lequel

$R^7$ est un groupe alkyle linéaire ou ramifié ayant 1 à 4 atomes de carbone, un groupe

alkoxy linéaire ou ramifié ayant 1 à 4 atomes de carbone ou un groupe dialkylamino linéaire ou ramifié ayant 1 à 4 atomes de carbone dans chacun des restes alkyle individuels,

B représente le groupe $CH-R^8$ ou $NO-R^9$ dans lequel

$R^8$ est un groupe phényle, naphtyle, pyridinyle, pyrimidinyle, pyrazinyle, pyridazinyle, thiényle, furyle, pyrrolyle, thiazolyle, isothiazolyle, oxazolyle, isoxazolyle non substitué ou portant 1 à 3 substituants identiques ou différents, en considérant dans chaque cas comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylènedioxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, benzyle, phénoxy, benzyloxy, phénylthio ou benzylthio portant chacun le cas échéant dans la partie phényle 1 à 3 substituants fluoro, chloro, bromo, méthyle, éthyle, méthoxy, éthoxy, méthylthio, trifluorométhyle, difluorométhoxy, trifluorométhoxy et/ou trifluorométhylthio identiques ou différents ;

$R^9$ est un groupe alkyle non substitué ou substitué ayant 1 à 6 atomes de carbone, un groupe alcényle non substitué ou substitué ayant 2 à 6 atomes de carbone, un groupe alcynyle non substitué ou substitué ayant 2 à 6 atomes de carbone, en considérant dans chaque cas comme substituants :
un radical cyano, méthoxy, éthoxy, n-propoxy, isopropoxy, n-butoxy, iso-butoxy, sec.-butoxy, tertio-butoxy, méthoxycarbonyle, éthoxycarbonyle, n-propoxycarbonyle, isopropoxycarbonyle, n-butoxycarbonyle, isobutoxycarbonyle, sec.-butoxycarbonyle et tertio-butoxycarbonyle,
en outre
un groupe phényle ou benzyle non substitué ou portant 1 à 3 substituants identiques ou différents, en considérant comme substituants : le fluor, le chlore, le brome, un radical méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, halogénalkyle ayant 1 ou 2 atomes de carbone et 1 à 5 atomes de fluor ou de chlore identiques ou différents, méthylidènedioxy et éthylidènedioxy et

R et Q représentent, indépendamment l'un de l'autre, de l'hydrogène, un groupe méthyle, éthyle, n-propyle, isopropyle, méthoxy, éthoxy ou halogénalkyle ayant 1 ou 2 atomes de carbone et 1 à 5 atomes de fluor ou de chlore identiques ou différents.

**4.** Esters d'acides acryliques de formule (I) suivant la revendication 1, dans lesquels

$R^1$ est un groupe méthyle, éthyle ou benzyle, en considérant comme substituants du groupe benzyle les substituants mentionnés pour $R^8$,

$R^2$ est un groupe diméthylamino, diéthylamino ou un reste $-Z-R^3$, dans lequel

$R^3$ est un groupe méthyle, éthyle, n-propyle, isopropyle ou benzyle,

Z est de l'oxygène ou du soufre,

A est de l'oxygène, du soufre ou l'un des groupements suivants :
$-(CH_2)-_n$, $>CH-CH_3$, $>CH-C_2H_5$,
$>CH-CH(CH_3)_2$, $>CH-C(CH_3)_3$, $>C(CH_3)_2$,

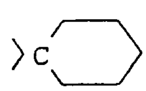 ,

$>N-COOCH_3$, $>N-COOC_2H_5$,

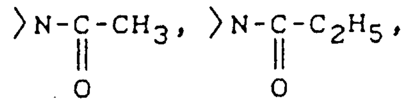

dans lesquels

n représente les nombres 0, 1, 2 ou 3,

B représente le groupe $CH-R^8$ ou $NO-R^9$,

dans lequel

$R^8$ est un groupe phényle, naphtyle, pyridinyle, pyrimidinyle, pyrazinyle, pyridazinyle, thiényle, furyle, pyrrolyle, thiazolyle, isothiazolyle, oxazolyle, isoxazolyle non substitué ou portant chacun 1 à 3 substituants identiques ou différents, en considérant dans chaque cas comme substituants : le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, méthylènedioxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, phénoxy, benzyle, benzyloxy, phénylthio, ou benzylthio portant chacun le cas échéant 1 ou 2 substituants fluoro, chloro, bromo, méthyle et/ou éthyle identiques ou différents ;

$R^9$ est un groupe alkyle non substitué ou substitué ayant 1 à 6 atomes de carbone, un groupe alcényle non substitué ou substitué ayant 2 à 4 atomes de carbone ou un groupe alcynyle non substitué ou substitué ayant 2 à 4 atomes de carbone, en considérant comme substituants un radical cyano, méthoxycarbonyle, éthoxycarbonyle, méthoxy ou alkoxy, en outre, un groupe phényle ou benzyle non substitué ou portant 1 ou 2 substituants identiques ou différents,
en considérant comme substituants :
le brome, le fluor, le chlore, un radical méthoxy, éthoxy, méthylènedioxy, méthyle, éthyle, trifluorométhyle et

R et Q représentent indépendamment l'un de l'autre de l'hydrogène ou un groupe méthyle.

**5.** Esters d'acides acryliques de formule (I) suivant la revendication 1, dans lesquels

$R^1$ est un groupe méthyle ou éthyle,

$R^2$ est un groupe méthoxy, éthoxy, méthylthio ou diméthylamino,

A est de l'oxygène, du soufre ou l'un des groupements suivants
$-(CH_2)-_n$,

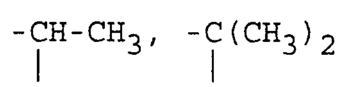

dans lesquels

n représente les nombres 0, 1 ou 2,

B est le groupe $CH-R^8$ ou $NO-R^9$,

dans lequel

$R^8$ est un groupe phényle, naphtyle, pyridinyle, pyrimidinyle, thiényle, furyle, thiazolyle, oxazolyle, isoxazolyle, non substitués ou portant chacun 1 à 3 substituants identiques ou différents, en considérant comme substituants : le fluor, le chlore, le brome, un radical cyano, nitro, méthyle, éthyle, méthoxy, éthoxy, méthylènedioxy, méthylthio, trifluorométhyte, méthoxycarbonyle, éthoxycarbonyle, cyclopentyle, cyclohexyle, phénoxy, phénylthio, benzyloxy, benzylthio,

$R^9$ est un groupe méthyle, éthyle, n-propyle, isopropyle, n-butyle, iso-butyle, tertio-butyle, n-amyle, iso-amyle, allyle, méthallyle, propargyle, benzyle, o-, m- et p-chlorobenzyle, o-, m- et p-méthoxybenzyle, o-, m- et p-méthylbenzyle ou o-, m- et p-fluorobenzyle et

R et Q représentent de l'hydrogène.

**6.** Procédé de production d'esters d'acides acryliques de formule générale (I)

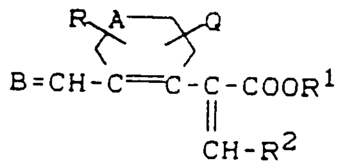

(I)

dans laquelle

- $R^1$ est un groupe alkyle ou un groupe aralkyle substitué ou non substitué,
- $R^2$ est un groupe dialkylamino ou un reste $-Z-R^3$,
- $R^3$ est un groupe alkyle ou un groupe aralkyle non substitué ou substitué,
- Z est de l'oxygène ou du soufre
- A est de l'oxygène, du soufre ou l'un des groupements suivants : $-(CH_2)-_n$,

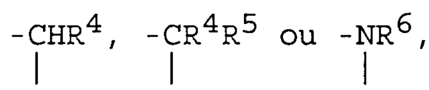

dans lesquels

- n est un nombre de 0 à 6,
- $R^4$ et $R^5$ représentent chacun, indépendamment l'un de l'autre, un groupe alkyle ou forment conjointement une chaîne alkylique de 2 à 7 atomes de carbone et
- $R^6$ est un groupe alkyle ou un reste

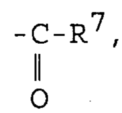

dans lequel

- $R^7$ est un groupe alkyle, alkoxy ou dialkylamino,
- B est le groupe $CH-R^8$ ou $NO-R^9$, où
- $R^8$ est un groupe aryle ou hétaryle non substitué ou substitué et
- $R^9$ est un groupe alkyle, alcényle, alcynyle, aralkyle, aryle ou hétaryle non substitué ou substitué et
- R et Q représentent, indépendamment l'un de l'autre, de l'hydrogène, un groupe alkyle, halogénalkyle ou alkoxy.

caractérisé en ce que

a) on obtient des esters d'acides acryliques substitués de formule (Ia)

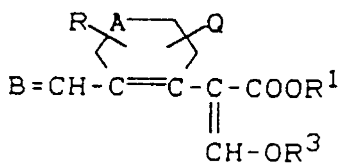

(Ia)

dans laquelle

$R^1$, $R^3$, A, B, R et Q ont la définition indiquée ci-dessus,

lorsqu'on fait réagir des esters d'acides hydroxyacryliques ou leurs sels de métaux alcalins de formule (II),

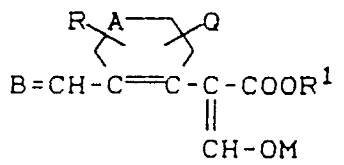

(II)

dans laquelle

| | |
|---|---|
| M | représente de l'hydrogène ou un cation de métal alcalin et |
| $R^1$, A, B, R et Q | ont la définition indiquée ci-dessus |

avec des agents alkylants de formule (III)

$R^3$-$E^1$ (III)

dans laquelle

| | |
|---|---|
| $E^1$ | est un groupe partant attirant les électrons et |
| $R^3$ | a la définition indiquée ci-dessus, |

le cas échéant en présence d'un diluant et en la présence éventuelle d'une substance auxiliaire de réaction ;

b) on obtient des esters d'acides acryliques substitués de formule (Ib)

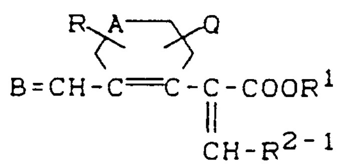

(Ib)

dans laquelle

| | |
|---|---|
| $R^{2-1}$ | représente un groupe dialkylamino, et |
| $R^1$, A, B, R et Q | ont la définition indiquée ci-dessus, |

lorsqu'on fait réagir des esters d'acides acétiques substitués de formule (IV),

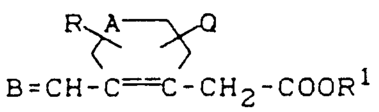

(IV)

dans laquelle

$R^1$, A, B, R et Q ont la définition indiquée ci-dessus,

avec des formamides de formule (Va)

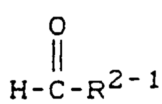

(Va)

dans laquelle

$R^{2-1}$ a la définition indiquée ci-dessus,

ou avec des dérivés de formamide de formule (Vb)

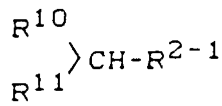

(Vb)

dans laquelle

$R^{10}$ et $R^{11}$ représentent indépendamment l'un de l'autre un groupe alkoxy ou dialkylamino et

$R^{2-1}$ a la définition indiquée ci-dessus,

le cas échéant en présence d'un diluant ;

c) on obtient des esters d'acides acryliques substitués de formule (Ic),

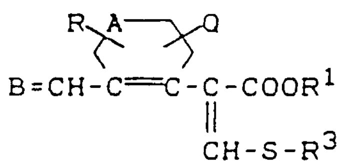

(Ic)

dans laquelle

$R^1$, $R^3$, A, B, R et Q ont la définition indiquée ci-dessus,

lorsqu'on fait réagir des dérivés d'acides cétocarboxyliques de formule (VI)

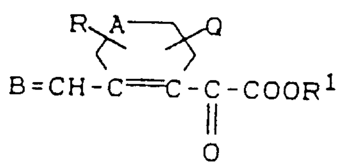

(VI)

dans laquelle

$R^1$, A, B, R et Q ont la définition indiquée ci-dessus,

avec des composés organométalliques de formule (VII)

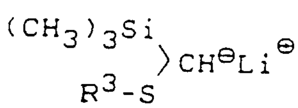

(VII)

dans laquelle

$R^3$ a la définition indiquée ci-dessus,

le cas échéant en présence d'un diluant ;

d) on obtient des esters d'acides acryliques substitués de formule (Ic) lorsqu'on fait réagir des esters d'acides acryliques substitués de formule (VIII),

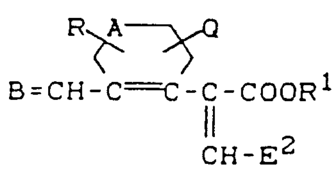

(VIII)

dans laquelle

$R^1$, A, B, R et Q ont la définition indiquée ci-dessus et

$E^2$ est un groupe partant attirant les électrons,

avec des thiols de formule (IX),

$R^3$-SH (IX)

dans laquelle
$R^3$ a la définition indiquée ci-dessus,
le cas échéant en présence d'un diluant et en présence éventuelle d'une substance auxiliaire de réaction.

**7.** Compositions pesticides, caractérisées par une teneur en au moins un ester d'acide acrylique de formule (I) suivant les revendications 1 à 6.

**8.** Utilisation d'esters d'acides acryliques de formule (I) suivant les revendications 1 à 6 pour combattre des parasites.

**9.** Procédé pour combattre des parasites, caractérisé en ce qu'on fait agir des esters d'acides acryliques de formule (I) suivant les revendications 1 à 6 sur les parasites et/ou sur leur milieu.

**10.** Procédé de préparation de compositions pesticides, caractérisé en ce qu'on mélange des esters d'acides acryliques de formule (I) suivant les revendications 1 à 6 avec des diluants et/ou avec des agents tensio-actifs.

**11.** Esters d'acides hydroxyacryliques de formule générale (II)

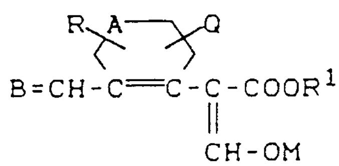 (II)

dans laquelle
$R^1$ est un groupe alkyle ou un groupe aralkyle non substitué ou substitué,
A est de l'oxygène, du soufre ou l'un des groupements suivants :
$-(CH_2)-_n$,

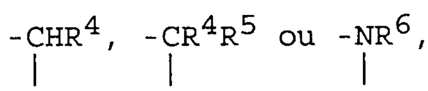

dans lesquels
n est un nombre de 0 à 6,
$R^4$ et $R^5$ représentent chacun, indépendamment l'un de l'autre, un groupe alkyle ou forment ensemble une chaîne alkylique de 2 à 7 atomes de carbone et
$R^6$ est un groupe alkyle ou un reste

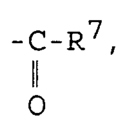

dans laquelle
$R^7$ est un radical alkyle, alkoxy ou dialkylamino,
B représente le groupe CH-$R^8$ ou NO-$R^9$,
dans lequel
$R^8$ est un groupe aryle ou hétaryle non substitué ou substitué et

| | |
|---|---|
| $R^9$ | est un groupe alkyle, alcényle, alcynyle, aralkyle, aryle ou hétaryle non substitué ou substitué et |
| R et Q | représentent indépendamment l'un de l'autre de l'hydrogène, un groupe alkyle, halogénalkyle ou alkoxy et |
| M | est de l'hydrogène ou un cation de métal alcalin. |

**12.** Procédé de production d'esters d'acides hydroxyacryliques de formule (II) suivant la revendication 11, caractérisé en ce qu'on fait réagir des esters d'acides acétiques substitués de formule (IV)

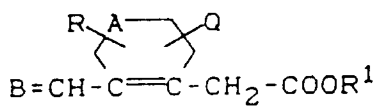

(IV)

dans laquelle

$R^1$, A, B, R et Q ont la définition indiquée ci-dessus,

avec des esters d'acide formique de formule (X),

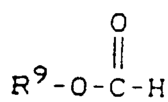

(X)

dans laquelle

$R^9$ est un groupe alkyle, notamment méthyle ou éthyle,

le cas échéant en présence d'un diluant et en la présence éventuelle d'une substance auxiliaire basique de réaction, à des températures de -20 °C à +50 °C.

**13.** Esters d'acides acétiques de formule générale (IV)

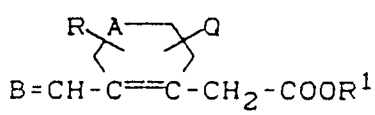

(IV)

dans laquelle

| | |
|---|---|
| $R^1$ | est un groupe alkyle ou un groupe aralkyle non substitué ou substitué, |
| A | est de l'oxygène, du soufre ou l'un des groupements suivants $-(CH_2)-_n$, |

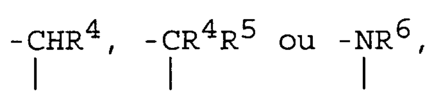

dans laquelle

| | |
|---|---|
| n | est un nombre de 0 à 6, |
| $R^4$ et $R^5$ | représentent chacun, indépendamment l'un de l'autre, un groupe alkyle ou forment ensemble une chaîne alkylique de 2 à 7 atomes de carbone |
| $R^6$ | est un groupe alkyle ou un reste |

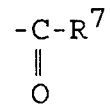

dans lequel

| | |
|---|---|
| $R^7$ | est un radical alkyle, alkoxy ou dialkylamino, |
| B | est le groupe $CH-R^8$ ou $NO-R^9$, dans lequel |
| $R^8$ | est un radical aryle ou hétaryle non substitué ou substitué, |
| $R^9$ | est un radical alkyle, alcényle, alcynyle, aralkyle, aryle ou hétaryle non substitué ou substitué et |
| R et Q | représentent, indépendamment l'un de l'autre, de l'hydrogène, un groupe alkyle, halogénalkyle ou alkoxy. |

**14.** Procédé de production d'esters d'acides acétiques de formule (IV) suivant la revendication 13, caractérisé en ce que

a) au cas où $R^1$, A, R et Q ont la définition indiquée ci-dessus, et

$B^1$ représente le groupe $N-O-R^9$, dans lequel

$R^9$ a la définition indiquée ci-dessus,

on fait réagir des aldéhydes de formule générale (XI)

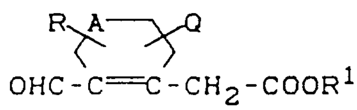
(XI)

dans laquelle

$R^1$, A, R et Q ont la définition indiquée ci-dessus,

avec des composés de formule générale (XII)

$R^9-O-NH_2$ (XII)

dans laquelle

$R^9$ a la définition indiquée ci-dessus,

le cas échéant en présence d'un diluant, à des températures de -20 °C à +50 °C,

ou bien

b) au cas où $R^1$, A, R et Q ont la définition indiquée ci-dessus et

$B^2$ représente le groupe $CH-R^8$, dans lequel

$R^8$ a la définition indiquée ci-dessus,

on fait réagir des aldéhydes de formule générale (XI)

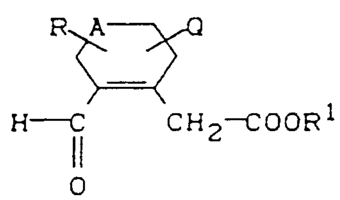
(XI)

dans laquelle

$R^1$, A, R et Q ont la définition indiquée ci-dessus,

α) avec des composés de phosphonium de formule générale (XIII)

$R^8-CH_2-P(R^{12})_3{}^{\oplus}X^{\ominus}$ (XIII)

dans laquelle

$R^8$ a la définition indiquée ci-dessus,

$R^{12}$ est un groupe aryle, de préférence phényle, éventuellement substitué par un radical méthyle, éthyle, méthoxy ou éthoxy et

X représente un halogène, de préférence le brome ou le chlore,

ou bien

$\beta$) avec des réactifs de formule générale (XIV)

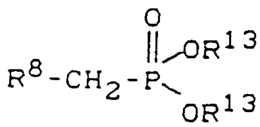

(XIV)

dans laquelle

$R^8$ a la définition indiquée ci-dessus et

$R^{13}$ est un groupe alkyle en $C_1$ à $C_4$, notamment méthyle, éthyle ou n-butyle

en utilisant un solvant inerte dans les conditions de la réaction, et le cas échéant une substance auxiliaire basique, à des températures de -80°C à +60°C, dans le sens d'une réaction de WITTIG ou d'une réaction de HORNER-WITTIG.

**15.** Esters d'acides acryliques de formule générale (VIII)

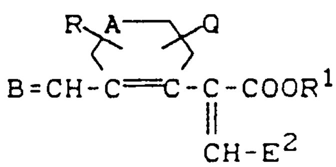

(VIII)

dans laquelle

$R^1$ est un groupe alkyle ou un groupe aralkyle non substitué ou substitué,

A est de l'oxygène, du soufre ou l'un des groupements suivants

$-(CH_2)-_n$, $-CHR^4$, $-CR^4R^5$ ou $-NR^6$,

où

n est un nombre de 0 à 6,

$R^4$ et $R^5$ représentent chacun, indépendamment l'un de l'autre, un groupe alkyle ou forment ensemble une chaîne alkylique de 2 à 7 atomes de carbone et

$R^6$ est un groupe alkyle ou un reste de

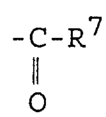

formule dans laquelle

$R^7$ est un groupe alkyle, alkoxy ou dialkylamino,

B est le groupe $CH-R^8$ ou $NO-R^9$,

dans lequel

| | |
|---|---|
| $R^8$ | est un groupe aryle ou hétaryle non substitué ou substitué, |
| $R^9$ | est un groupe alkyle, alcényle, alcynyle, aralkyle, aryle ou hétaryle non substitué ou substitué, |
| R et Q | représentent indépendamment l'un de l'autre de l'hydrogène, un groupe alkyle, halogénalkyle ou alkoxy et |
| $E^2$ | est un groupe partant attirant les électrons. |

**16.** Procédé de production d'esters d'acides acryliques de formule (VIII) suivant la revendication 15, caractérisé en ce qu'on fait réagir des esters d'acides hydroxyacryliques de formule (II)

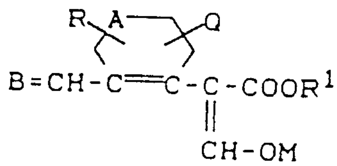

(II)

dans laquelle

| | |
|---|---|
| $R^1$, A, B, R et Q | ont la définition indiquée ci-dessus et M est de l'hydrogène ou un cation de métal alcalin, |

avec des chlorures d'acides de formule (XIV)

$R^{14}$-Cl (XIV)

dans laquelle

| | |
|---|---|
| $R^{14}$ | est un reste acyle ou sulfonyle, notamment un reste acétyle, un reste méthanesulfonyle ou un reste p-toluènesulfonyle, |

le cas échéant en présence d'un diluant et en la présence éventuelle d'un accepteur d'acide, à des températures de -20 °C à +120 °C.

**Revendications pour l'Etat contractant suivant : ES**

**1.** Procédé de production d'esters d'acides acryliques de formule générale (I)

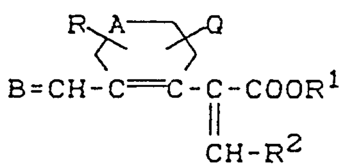

(I)

dans laquelle

| | |
|---|---|
| $R^1$ | est un groupe alkyle ou un groupe aralkyle substitué ou non substitué, |
| $R^2$ | est un groupe dialkylamino ou un reste $-Z-R^3$, |
| $R^3$ | est un groupe alkyle ou un groupe aralkyle non substitué ou substitué, |
| Z | est de l'oxygène ou du soufre |
| A | est de l'oxygène, du soufre ou l'un des groupements suivants : $-(CH_2)-_n$, |

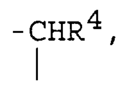 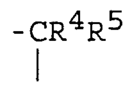 ou 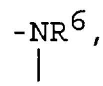

| | |
|---|---|
| | dans lesquels |
| n | est un nombre de 0 à 6, |
| $R^4$ et $R^5$ | représentent chacun, indépendamment l'un de l'autre, un groupe alkyle ou forment |

conjointement une chaîne alkylique de 2 à 7 atomes de carbone et

$R^6$ est un groupe alkyle ou un reste

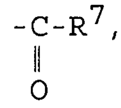

dans lequel

$R^7$ est un groupe alkyle, alkoxy ou dialkylamino,

B est le groupe $CH-R^8$ ou $NO-R^9$,

où

$R^8$ est un groupe aryle ou hétaryle non substitué ou substitué et

$R^9$ est un groupe alkyle, alcényle, alcynyle, aralkyle, aryle ou hétaryle non substitué ou substitué et

R et Q représentent, indépendamment l'un de l'autre, de l'hydrogène, un groupe alkyle, halogénalkyle ou alkoxy.

caractérisé en ce que

a) on obtient des esters d'acides acryliques substitués de formule (Ia)

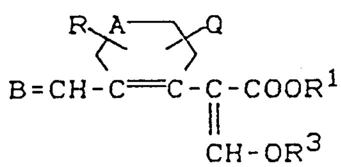

(Ia)

dans laquelle

$R^1$, $R^3$, A, B, R et Q ont la définition indiquée ci-dessus,

lorsqu'on fait réagir des esters d'acides hydroxyacryliques ou leurs sels de métaux alcalins de formule (II),

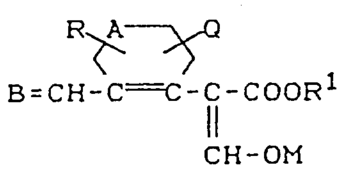

(II)

dans laquelle

M représente de l'hydrogène ou un cation de métal alcalin et

$R^1$, A, B, R et Q ont la définition indiquée ci-dessus

avec des agents alkylants de formule (III)

$R^3-E^1$ (III)

dans laquelle

$E^1$ est un groupe partant attirant les électrons et

$R^3$ a la définition indiquée ci-dessus,

le cas échéant en présence d'un diluant et en la présence éventuelle d'une substance auxiliaire de réaction ;

b) on obtient des esters d'acides acryliques substitués de formule (Ib)

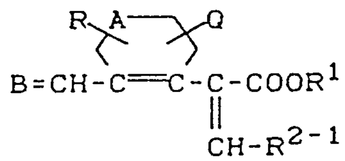

(Ib)

dans laquelle

$R^{2-1}$ représente un groupe dialkylamino, et

$R^1$, A, B, R et Q ont la définition indiquée ci-dessus,

lorsqu'on fait réagir des esters d'acides acétiques substitués de formule (IV),

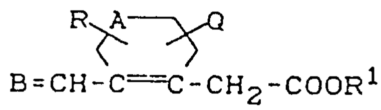

(IV)

dans laquelle

$R^1$, A, B, R et Q ont la définition indiquée ci-dessus,

avec des formamides de formule (Va)

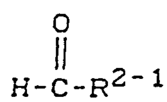

(Va)

dans laquelle

$R^{2-1}$ a la définition indiquée ci-dessus,

ou avec des dérivés de formamide de formule (Vb)

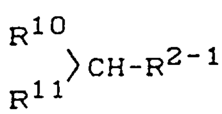

(Vb)

dans laquelle

$R^{10}$ et $R^{11}$ représentent indépendamment l'un de l'autre un groupe alkoxy ou dialkylamino et $R^{2-1}$ a la définition indiquée ci-dessus,

le cas échéant en présence d'un diluant ;

c) on obtient des esters d'acides acryliques substitués de formule (Ic),

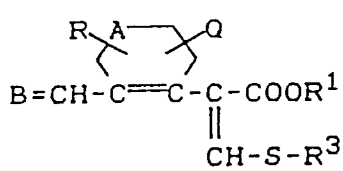

(Ic)

dans laquelle

$R^1$, $R^3$, A, B, R et Q ont la définition indiquée ci-dessus,

lorsqu'on fait réagir des dérivés d'acides cétocarboxyliques de formule (VI)

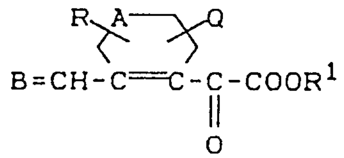 (VI)

dans laquelle

$R^1$, A, B, R et Q ont la définition indiquée ci-dessus,

avec des composés organométalliques de formule (VII)

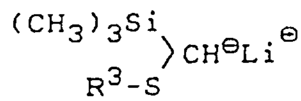 (VII)

dans laquelle

$R^3$ a la définition indiquée ci-dessus,

le cas échéant en présence d'un diluant ;

d) on obtient des esters d'acides acryliques substitués de formule (Ic) lorsqu'on fait réagir des esters d'acides acryliques substitués de formule (VIII),

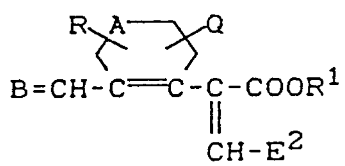 (VIII)

dans laquelle

$R^1$, A, B, R et Q ont la définition indiquée ci-dessus et

$E^2$ est un groupe partant attirant les électrons,

avec des thiols de formule (IX),

$R^3$-SH (IX)

dans laquelle

$R^3$ a la définition indiquée ci-dessus,

le cas échéant en présence d'un diluant et en présence éventuelle d'une substance auxiliaire de réaction.

**2.** Procédé suivant la revendication 1, dans lequel

$R^1$ est un groupe alkyle linéaire ou ramifié ayant 1 à 6 atomes de carbone ou un groupe aralkyle non substitué ou portant dans la partie aryle un ou plusieurs substituants identiques ou différents et ayant 1 à 4 atomes de carbone dans la partie alkyle linéaire ou ramifiée et 6 à 10 atomes de carbone dans la partie aryle, en considérant comme substituants du groupe aryle les substituants mentionnés pour $R^8$,

$R^2$ est un groupe dialkylamino ayant 1 à 6 atomes de carbone dans chacune des parties alkyle individuelles linéaires ou ramifiées ou un reste -Z-$R^3$, dans lequel

$R^3$ est un groupe alkyle linéaire ou ramifié ayant 1 à 6 atomes de carbone ou un groupe aralkyle non substitué ou portant dans la partie aryle un ou plusieurs substituants identiques ou différents, ayant 1 à 4 atomes de carbone dans la partie alkyle linéaire ou

ramifiée et 6 à 10 atomes de carbone dans la partie aryle, en considérant comme substituants du groupe aryle les substituants mentionnés pour $R^8$,

Z est de l'oxygène ou du soufre,

A est de l'oxygène, du soufre ou l'un des groupements suivants :
$-(CH_2)_n-$,

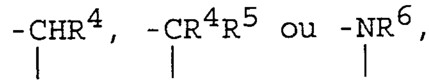

dans lesquels

n est un nombre de 0 à 3,

$R^4$ et $R^5$ représentent chacun, indépendamment l'un de l'autre, un groupe alkyle linéaire ou ramifié ayant 1 à 6 atomes de carbone ou forment conjointement une chaîne alkylique de 2 à 7 atomes de carbone et

$R^6$ est un groupe alkyle linéaire ou ramifié ayant 1 à 6 atomes de carbone ou un reste

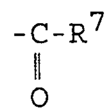

dans lequel

$R^7$ est un groupe alkyle linéaire ou ramifié ayant 1 à 6 atomes de carbone, un groupe alkoxy linéaire ou ramifié ayant 1 à 6 atomes de carbone ou un groupe dialkylamino linéaire ou ramifié ayant 1 à 6 atomes de carbone dans chacune des parties alkyle individuelles,

B est le groupe $CH-R^8$ ou $NO-R^9$,
dans lequel

$R^8$ est un groupe aryle non substitué ou substitué ayant 6 à 10 atomes de carbone dans la partie aryle ou un groupe hétéroaryle ayant 2 à 9 atomes de carbone et 1 à 4 hétéroatomes identiques ou différents - notamment azote, oxygène et/ou soufre - dans la partie hétéroaryle,
en considérant dans chaque cas comme substituants de la partie aryle et de la partie hétéroaryle :
un halogène, un radical cyano, nitro, un radical alkyle, alkoxy ou alkylthio linéaire ou ramifié ayant chacun 1 à 4 atomes de carbone, un radical alkylidènedioxy ayant 1 à 6 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un radical alkoxycarbonyle ou alkoximinoalkyle linéaire ou ramifié ayant chacun 1 à 4 atomes de carbone dans les parties alkyle individuelles, un radical cycloalkyle de 3 à 7 atomes de carbone, un radical alcanediyle divalent ayant 3 à 5 atomes de carbone, un radical aryle, aralkyle, aryloxy, arylthio, aralkyloxy ou aralkylthio ayant chacun 6 à 10 atomes de carbone dans la partie aryle et le cas échéant 1 à 4 atomes de carbone dans la partie alkyle linéaire ou ramifiée, chacun portant le cas échéant dans la partie aryle un ou plusieurs substituants halogéno, alkyle, alkoxy, alkylthio, halogénalkyle, halogénalkoxy ou halogénalkylthio identiques ou différents ayant chacun 1 à 4 atomes de carbone et le cas échéant 1 à 9 atomes d'halogènes identiques ou différents, ou un radical hétéroarylalkyle, hétéroaryloxy, hétéroarylthio ou hétéroaryle ayant chacun 2 à 9 atomes de carbone et 1 à 4 hétéroatomes - notamment azote, oxygène et/ou soufre - identiques ou différents dans la partie hétéroaryle et le cas échéant 1 à 4 atomes de carbone dans la partie alkyle linéaire ou ramifiée, portant chacun le cas échéant dans la partie hétéroaryle un ou plusieurs substituants halogéno, alkyle, alkoxy, alkylthio, halogénalkyle, halogénalkoxy ou halogénalkylthio identiques ou différents ayant chacun 1 à 4 atomes de carbone et le cas échéant 1 à 9 atomes d'halogènes identiques ou différents,

$R^9$ est un groupe alkyle non substitué ou substitué ayant 1 à 8 atomes de carbone, un groupe alcényle non substitué ou substitué ayant 2 à 8 atomes de carbone ou un groupe alcynyle non substitué ou substitué ayant 2 à 8 atomes de carbone, en considérant dans chaque cas comme substituants : un radical cyano, alkoxy ayant 1 à 4 atomes de carbone ou alkoxycarbonyle ayant 1 à 4 atomes de carbone dans la partie alkoxy,
en outre
un groupe aryle de 6 à 10 atomes de carbone non substitué ou portant un ou plusieurs substituants identiques ou différents, un groupe aralkyle de 6 à 10 atomes de carbone dans la partie aryle et 1 ou 2 atomes de carbone dans la partie alkyle non substitué ou portant un ou plusieurs substituants identiques ou différents, en considérant comme substituants : un radical halogéno, alkyle ayant 1 à 4 atomes de carbone, alkoxy ayant 1 à 4 atomes de carbone, halogénalkyle ayant 1 à 4 atomes de carbone et 1 à 9 atomes d'halogènes identiques ou différents ou alkylidènedioxy ayant 1 ou 2 atomes de carbone,
en outre
un groupe hétéroaryle ayant 2 à 9 atomes de carbone et 1 à 4 hétéroatomes identiques ou différents - en particulier azote, oxygène et/ou soufre - dans la partie hétéroaryle et
R et Q représentent, indépendamment l'un de l'autre, de l'hydrogène, un groupe alkyle ayant 1 à 4 atomes de carbone, alkoxy ayant 1 à 4 atomes de carbone ou halogénalkyle ayant 1 à 4 atomes de carbone et 1 à 9 atomes d'halogènes identiques ou différents.

**3.** Procédé suivant la revendication 1, dans lequel
$R^1$ est un groupe méthyle, éthyle, n-propyle, isopropyle, n-butyle, isobutyle, sec.-butyle, tertio-butyle ou un groupe benzyle portant le cas échéant 1 à 3 substituants identiques ou différents, en considérant comme substituants ceux qui sont mentionnés pour $R^8$,
$R^2$ est un groupe dialkylamino ayant 1 à 4 atomes de carbone dans les parties alkyle individuelles linéaires ou ramifiées ou un reste -Z-$R^3$, dans lequel
$R^3$ est un groupe méthyle, éthyle, n-propyle, isopropyle, n-butyle, iso-butyle, sec.-butyle ou tertio-butyle ou un groupe benzyle portant le cas échéant 1 à 3 substituants identiques ou différents, en considérant comme substituants ceux qui ont été mentionnés pour $R^8$, et
Z est de l'oxygène ou du soufre ;
A est de l'oxygène, du soufre ou l'un des groupements suivants
$-(CH_2)_n-$,

$$-\underset{|}{C}HR^4,\quad -\underset{|}{C}R^4R^5 \text{ ou } -\underset{|}{N}R^6,$$

dans lesquels
n représente les nombres 0, 1, 2 et 3,
$R^4$ et $R^5$ représentent chacun indépendamment l'un de l'autre un groupe alkyle linéaire ou ramifié ayant 1 à 4 atomes de carbone ou forment conjointement une chaîne alkylique de 2 à 5 atomes de carbone et
$R^6$ est un groupe alkyle linéaire ou ramifié ayant 1 à 4 atomes de carbone ou un reste

$$-\underset{\underset{O}{\|}}{C}-R^7$$

dans lequel
$R^7$ est un groupe alkyle linéaire ou ramifié ayant 1 à 4 atomes de carbone, un groupe alkoxy linéaire ou ramifié ayant 1 à 4 atomes de carbone ou un groupe dialkylamino linéaire ou ramifié ayant 1 à 4 atomes de carbone dans chacun des restes alkyle individuels,

B  représente le groupe CH-$R^8$ ou NO-$R^9$
dans lequel

$R^8$  est un groupe phényle, naphtyle, pyridinyle, pyrimidinyle, pyrazinyle, pyridazinyle, thiényle, furyle, pyrrolyle, thiazolyle, isothiazolyle, oxazolyle, isoxazolyle non substitué ou portant 1 à 3 substituants identiques ou différents, en considérant dans chaque cas comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylènedioxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, benzyle, phénoxy, benzyloxy, phénylthio ou benzylthio portant chacun le cas échéant dans la partie phényle 1 à 3 substituants fluoro, chloro, bromo, méthyle, éthyle, méthoxy, éthoxy, méthylthio, trifluorométhyle, difluorométhoxy, trifluorométhoxy et/ou trifluorométhylthio identiques ou différents ;

$R^9$  est un groupe alkyle non substitué ou substitué ayant 1 à 6 atomes de carbone, un groupe alcényle non substitué ou substitué ayant 2 à 6 atomes de carbone, un groupe alcynyle non substitué ou substitué ayant 2 à 6 atomes de carbone,
en considérant dans chaque cas comme substituants :
un radical cyano, méthoxy, éthoxy, n-propoxy, isopropoxy, n-butoxy, iso-butoxy, sec.-butoxy, tertio-butoxy, méthoxycarbonyle, éthoxycarbonyle, n-propoxycarbonyle, isopropoxycarbonyle, n-butoxycarbonyle, isobutoxycarbonyle, sec.-butoxycarbonyle et tertio-butoxycarbonyle,
en outre
un groupe phényle ou benzyle non substitué ou portant 1 à 3 substituants identiques ou différents, en considérant comme substituants : le fluor, le chlore, le brome, un radical méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, halogénalkyle ayant 1 ou 2 atomes de carbone et 1 à 5 atomes de fluor ou de chlore identiques ou différents, méthylidènedioxy et éthylidènedioxy et

R et Q  représentent indépendamment l'un de l'autre de l'hydrogène, un groupe méthyle, éthyle, n-propyle, isopropyle, méthoxy, éthoxy ou halogénalkyle ayant 1 ou 2 atomes de carbone et 1 à 5 atomes de fluor ou de chlore identiques ou différents.

**4.** Procédé suivant la revendication 1, dans lequel

$R^1$  est un groupe méthyle, éthyle ou benzyle, en considérant comme substituants du groupe benzyle les substituants mentionnés pour $R^8$,

$R^2$  est un groupe diméthylamino, diéthylamino ou un reste -Z-$R^3$,
dans lequel

$R^3$  est un groupe méthyle, éthyle, n-propyle, isopropyle ou benzyle,

Z  est de l'oxygène ou du soufre,

A  est de l'oxygène, du soufre ou l'un des groupements suivants :
$-(CH_2)-_n$, $>CH-CH_3$, $>CH-C_2H_5$,
$>CH-CH(CH_3)_2$, $>CH-C(CH_3)_3$, $>C(CH_3)_2$,

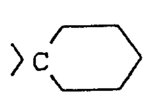 ,

$>N-COOCH_3$, $>N-COOC_2H_5$,

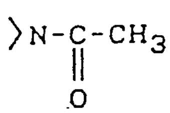 , 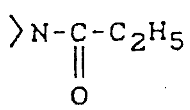 ,

dans lesquels

n représente les nombres 0, 1, 2 ou 3,

B représente le groupe CH-$R^8$ ou NO-$R^9$,

dans lequel

$R^8$ est un groupe phényle, naphtyle, pyridinyle, pyrimidinyle, pyrazinyle, pyridazinyle, thiényle, furyle, pyrrolyle, thiazolyle, isothiazolyle, oxazolyle, isoxazolyle non substitué ou portant chacun 1 à 3 substituants identiques ou différents, en considérant dans chaque cas comme substituants :

le fluor, le chlore, le brome, un radical cyano, nitro, méthyle, éthyle, n-propyle, isopropyle, n-butyle, iso-butyle, sec.-butyle, tertio-butyle, méthoxy, éthoxy, n-propoxy, isopropoxy, méthylènedioxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, phénoxy, benzyle, benzyloxy, phénylthio ou benzylthio portant chacun le cas échéant 1 ou 2 substituants fluoro, chloro, bromo, méthyle et/ou éthyle identiques ou différents ;

$R^9$ est un groupe alkyle non substitué ou substitué ayant 1 à 6 atomes de carbone, un groupe alcényle non substitué ou substitué ayant 2 à 4 atomes de carbone ou un groupe alcynyle non substitué ou substitué ayant 2 à 4 atomes de carbone, en considérant comme substituants un radical cyano, méthoxycarbonyle, éthoxycarbonyle, méthoxy ou alkoxy,

en outre, un groupe phényle ou benzyle non substitué ou portant 1 ou 2 substituants identiques ou différents,

en considérant comme substituants :

le brome, le fluor, le chlore, un radical méthoxy, éthoxy, méthylènedioxy, méthyle, éthyle, trifluorométhyle et

R et Q représentent indépendamment l'un de l'autre de l'hydrogène ou un groupe méthyle.

**5.** Procédé suivant la revendication 1, dans lequel

$R^1$ est un groupe méthyle ou éthyle,

$R^2$ est un groupe méthoxy, éthoxy, méthylthio ou diméthylamino,

A est de l'oxygène, du soufre ou l'un des groupements suivants

$-(CH_2)-_n$,

$$-\underset{|}{C}H-CH_3, \quad -\underset{|}{C}(CH_3)_2$$

dans lesquels

n représente les nombres 0, 1 ou 2,

B est le groupe CH-$R^8$ ou NO-$R^9$,

dans lequel

$R^8$ est un groupe phényle, naphtyle, pyridinyle, pyrimidinyle, thiényle, furyle, thiazolyle, oxazolyle, isoxazolyle, non substitués ou portant chacun 1 à 3 substituants identiques ou différents, en considérant comme substituants : le fluor, le chlore, le brome, un radical cyano, nitro, méthyle, éthyle, méthoxy, éthoxy, méthylènedioxy, méthylthio, trifluorométhyle, méthoxycarbonyle, éthoxycarbonyle, cyclopentyle, cyclohexyle, phénoxy, phénylthio, benzyloxy, benzylthio,

$R^9$ est un groupe méthyle, éthyle, n-propyle, isopropyle, n-butyle, iso-butyle, tertio-butyle, n-amyle, iso-amyle, allyle, méthallyle, propargyle, benzyle, o-, m- et p-chlorobenzyle, o-, m- et p-méthoxybenzyle, o-, m- et p-méthylbenzyle ou o-, m- et p-fluorobenzyle et

R et Q représentent de l'hydrogène.

**6.** Compositions pesticides, caractérisées par une teneur en au moins un ester d'acide acrylique de formule (I) suivant les revendications 1 à 5.

**7.** Utilisation d'esters d'acides acryliques de formule (I) suivant les revendications 1 à 5 pour combattre des parasites.

**8.** Procédé pour combattre des parasites, caractérisé en ce qu'on fait agir des esters d'acides acryliques de formule (I) suivant les revendications 1 à 5 sur les parasites et/ou sur leur milieu.

**9.** Procédé de préparation de compositions pesticides, caractérisé en ce qu'on mélange des esters d'acides acryliques de formule (I) suivant les revendications 1 à 5 avec des diluants et/ou des agents tensio-actifs.

**10.** Procédé de production d'esters d'acides hydroxyacryliques de formule générale (II)

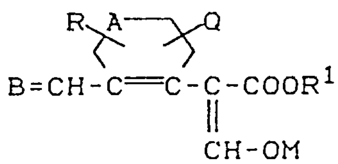

(II)

dans laquelle

| | |
|---|---|
| $R^1$ | est un groupe alkyle ou un groupe aralkyle non substitué ou substitué, |
| A | est de l'oxygène, du soufre ou l'un des groupements suivants : $-(CH_2)-_n$, |

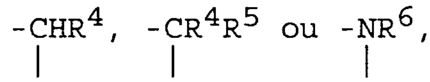

dans lesquels

| | |
|---|---|
| n | est un nombre de 0 à 6, |
| $R^4$ et $R^5$ | représentent chacun, indépendamment l'un de l'autre, un groupe alkyle ou forment ensemble une chaîne alkylique de 2 à 7 atomes de carbone et |
| $R^6$ | est un groupe alkyle ou un reste |

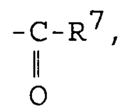

dans laquelle

| | |
|---|---|
| $R^7$ | est un radical alkyle, alkoxy ou dialkylamino, |
| B | représente le groupe $CH-R^8$ ou $NO-R^9$, dans lequel |
| $R^8$ | est un groupe aryle ou hétaryle non substitué ou substitué et |
| $R^9$ | est un groupe alkyle, alcényle, alcynyle, aralkyle, aryle ou hétaryle non substitué ou substitué et |
| R et Q | représentent indépendamment l'un de l'autre de l'hydrogène, un groupe alkyle, halogénalkyle ou alkoxy et |
| M | est de l'hydrogène ou un cation de métal alcalin, |

caractérisé en ce qu'on fait réagir des esters d'acides acétiques substitués de formule (IV)

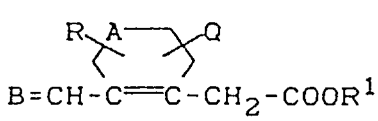

(IV)

dans laquelle

$R^1$, A, B, R et Q ont la définition indiquée ci-dessus,

avec des esters d'acide formique de formule (X),

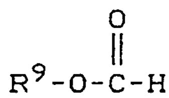

(X)

dans laquelle

R⁹ est un groupe alkyle, notamment méthyle ou éthyle,

le cas échéant en présence d'un diluant et en la présence éventuelle d'une substance auxiliaire basique de réaction, à des températures de -20 °C à +50 °C.

**11.** Procédé de production d'esters d'acides acétiques de formule générale (IV)

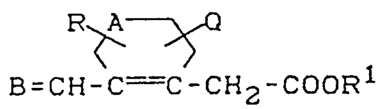

(IV)

dans laquelle

- $R^1$ est un groupe alkyle ou un groupe aralkyle non substitué ou substitué,
- A est de l'oxygène, du soufre ou l'un des groupements suivants $-(CH_2)-_n$,

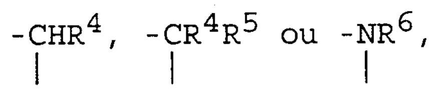

dans laquelle

- n est un nombre de 0 à 6,
- $R^4$ et $R^5$ représentent chacun, indépendamment l'un de l'autre, un groupe alkyle ou forment ensemble une chaîne alkylique de 2 à 7 atomes de carbone
- $R^6$ est un groupe alkyle ou un reste

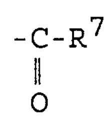

dans lequel

- $R^7$ est un radical alkyle, alkoxy ou dialkylamino,
- B est le groupe $CH-R^8$ ou $NO-R^9$,

dans lequel

- $R^8$ est un radical aryle ou hétaryle non substitué ou substitué,
- $R^9$ est un radical alkyle, alcényle, alcynyle, aralkyle, aryle ou hétaryle non substitué ou substitué et
- R et Q représentent, indépendamment l'un de l'autre, de l'hydrogène, un groupe alkyle, halogénalkyle ou alkoxy,

caractérisé en ce que :

a) au cas où $R^1$, A, R et Q ont la définition indiquée ci-dessus, et

- $B^1$ représente le groupe $N-O-R^9$,

dans lequel

- $R^9$ a la définition indiquée ci-dessus, on fait réagir des aldéhydes de formule générale (XI)

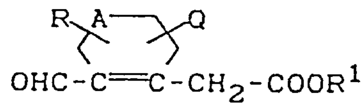

(XI)

dans laquelle

$R^1$, A, R et Q ont la définition indiquée ci-dessus,

avec des composés de formule générale (XII)

$R^9$-O-$NH_2$ (XII)

dans laquelle

$R^9$ a la définition indiquée ci-dessus,

le cas échéant en présence d'un diluant, à des températures de -20 °C à +50 °C,

ou bien

b) au cas où $R^1$, A, R et Q ont la définition indiquée ci-dessus et

$B^2$ représente le groupe CH-$R^8$,
dans lequel

$R^8$ a la définition indiquée ci-dessus,

on fait réagir des aldéhydes de formule générale (XI)

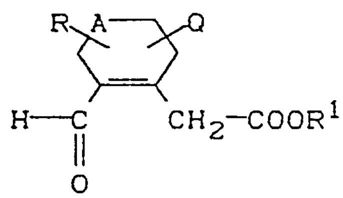

(XI)

dans laquelle

$R^1$, A, R et Q ont la définition indiquée ci-dessus,

α) avec des composés de phosphonium de formule générale (XIII)

$R^8$-$CH_2$-$P(R^{12})_3^{\oplus}X^{\ominus}$ (XIII)

dans laquelle

$R^8$ a la définition indiquée ci-dessus,

$R^{12}$ est un groupe aryle, de préférence phényle, éventuellement substitué par un radical méthyle, éthyle, méthoxy ou éthoxy et

X représente un halogène, de préférence le brome ou le chlore,

ou bien

β) avec des réactifs de formule générale (XIV)

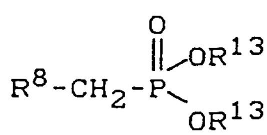

(XIV)

dans laquelle

$R^8$ a la définition indiquée ci-dessus et

$R^{13}$ est un groupe alkyle en $C_1$ à $C_4$, notamment méthyle, éthyle ou n-butyle

en utilisant un solvant inerte dans les conditions de la réaction, et le cas échéant une substance auxiliaire basique, à des températures de -80 °C à +60 °C, dans le sens d'une réaction de WITTIG ou d'une réaction de HORNER-WITTIG.

**12.** Procédé de production d'esters d'acides acryliques de formule générale (VIII)

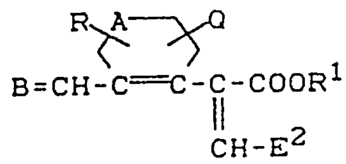

(VIII)

dans laquelle

- $R^1$ est un groupe alkyle ou un groupe aralkyle non substitué ou substitué,
- A est de l'oxygène, du soufre ou l'un des groupements suivants $-(CH_2)-_n$,

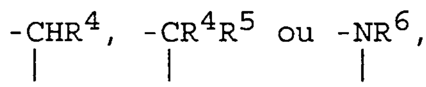

où

- n est un nombre de 0 à 6,
- $R^4$ et $R^5$ représentent chacun, indépendamment l'un de l'autre, un groupe alkyle ou forment ensemble une chaîne alkylique de 2 à 7 atomes de carbone et
- $R^6$ est un groupe alkyle ou un reste de formule

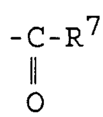

dans laquelle

- $R^7$ est un groupe alkyle, alkoxy ou dialkylamino,
- B est le groupe $CH-R^8$ ou $NO-R^9$, dans lequel
- $R^8$ est un groupe aryle ou hétaryle non substitué ou substitué,
- $R^9$ est un groupe alkyle, alcényle, alcynyle, aralkyle, aryle ou hétaryle non substitué ou substitué,
- R et Q représentent indépendamment l'un de l'autre de l'hydrogène, un groupe alkyle, halogénalkyle ou alkoxy et
- $E^2$ est un groupe partant attirant les électrons,

caractérisé en ce qu'on fait réagir des esters d'acides hydroxyacryliques de formule (II)

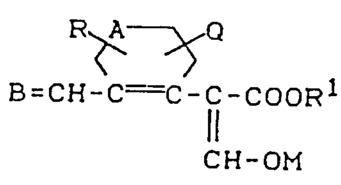

(II)

dans laquelle

- $R^1$, A, B, R et Q ont la définition indiquée ci-dessus et M est de l'hydrogène ou un cation de métal alcalin,

avec des chlorures d'acides de formule (XIV)

$R^{14}$-Cl (XIV)

dans laquelle

$R^{14}$ est un reste acyle ou sulfonyle, notamment un reste acétyle, un reste méthanesulfonyle ou un reste p-toluènesulfonyle,

le cas échéant en présence d'un diluant et en la présence éventuelle d'un accepteur d'acide, à des températures de -20 °C à +120 °C.